# EUROPEAN PATENT SPECIFICATION

(11) **EP 3 183 263 B1**
(45) Date of publication and mention of the grant of the patent: **27.05.2020**
(21) Application number: 15750745.0
(22) Date of filing: 14.08.2015
(51) Int. Cl.: C07K 14/435, C12N 9/16, C12P 19/00, C12N 9/18, C12P 7/62, C12P 19/02, C12P 7/40, A23L 29/00

(54) **ENZYMES WITH CHLOROGENIC ACID ESTERASE ACTIVITY AND FERULOYL ESTERASE ACTIVITY**
ENZYME MIT CHLOROGENER SÄUREESTERASEAKTIVITÄT UND FERULOYLESTERASEAKTIVITÄT
ENZYMES AYANT UNE ACTIVITÉ ACIDE CHLOROGÉNIQUE ESTÉRASE ET UNE ACTIVITÉ FÉRULOYLE ESTÉRASE

(30) Priority: 22.08.2014 EP 14182006
(43) Date of publication of application: 28.06.2017
(60) Divisional application: 19169195.5
(73) Proprietor: SternEnzym GmbH & Co. KG, 22926 Ahrensburg (DE)
(72) Inventor: NIETER, Annabel, 30167 Hannover (DE); LINKE, Diana, 31547 Bad Rehburg (DE); BERGER, Ralf Günter, 30455 Hannover (DE); THIESING, David, 20535 Hamburg (DE); POPPER, Lutz, Hamburg 22527 (DE)
(74) Representative: Moré, Solveig Helga
(86) International application number: PCT/EP2015/068800
(87) International publication number: WO 2016/026793

(56) References cited:
- WO-A2-2004/009804
- DATABASE UniProt [Online] 11 June 2014 (2014-06-11), "RecName: Full=Carboxylic ester hydrolase {ECO:0000256|RuleBase:RU361238}; EC=3.1.1.- {ECO:0000256|RuleBase:RU361238};", XP002784557, retrieved from EBI accession no. UNIPROT:X8IZA2 Database accession no. X8IZA2
- DATABASE UniProt [Online] 11 June 2014 (2014-06-11), "RecName: Full=Carboxylic ester hydrolase {ECO:0000256|RuleBase:RU361238}; EC=3.1.1.- {ECO:0000256|RuleBase:RU361238};", XP002784558, retrieved from EBI accession no. UNIPROT:X8J3Z2 Database accession no. X8J3Z2
- DATABASE UniProt [Online] 5 October 2010 (2010-10-05), "RecName: Full=Carboxylic ester hydrolase {ECO:0000256|RuleBase:RU361238}; EC=3.1.1.- {ECO:0000256|RuleBase:RU361238};", XP002784559, retrieved from EBI accession no. UNIPROT:D8QHC3 Database accession no. D8QHC3 & Robin A Ohm ET AL: "Genome sequence of the model mushroom Schizophyllum commune", Nature Biotechnology, vol. 28, no. 9, 11 July 2010 (2010-07-11), pages 957-963, XP055078455, ISSN: 1087-0156, DOI: 10.1038/nbt.1643
- DATABASE UniProt [Online] 5 October 2010 (2010-10-05), "RecName: Full=Carboxylic ester hydrolase {ECO:0000256|RuleBase:RU361238}; EC=3.1.1.- {ECO:0000256|RuleBase:RU361238};", XP002784560, retrieved from EBI accession no. UNIPROT:D8QHD6 Database accession no. D8QHD6
- DATABASE Geneseq [Online] 23 February 2017 (2017-02-23), "Schizophyllum commune PC-2 protein, SEQ ID 80.", XP002784561, retrieved from EBI accession no. GSP:BDL66254 Database accession no. BDL66254 & WO 2016/207351 A2 (GENENCOR INT BV [NL]) 29 December 2016 (2016-12-29)
- DATABASE Geneseq [Online] 23 February 2017 (2017-02-23), "Schizophyllum commune PC-2 protein, SEQ ID 81.", XP002784572, retrieved from EBI accession no. GSP:BDL66255 Database accession no. BDL66255
- BENOIT I ET AL: "Feruloyl esterases as a tool for the release of phenolic compounds from agro-industrial by-products", CARBOHYDRATE RESEARCH, PERGAMON, GB, vol. 341, no. 11, 14 August 2006 (2006-08-14), pages 1820-1827, XP025010505, ISSN: 0008-6215, DOI: 10.1016/J.CARRES.2006.04.020 [retrieved on 2006-08-14]
- I. BENOIT ET AL: "Gene Overexpression and Biochemical Characterization of the Biotechnologically Relevant Chlorogenic Acid Hydrolase from Aspergillus niger", APPLIED AND ENVIRONMENTAL MICROBIOLOGY, vol. 73, no. 17, 1 September 2007 (2007-09-01), pages 5624-5632, XP055221299, US ISSN: 0099-2240, DOI: 10.1128/AEM.00374-07
- ANNABEL NIETER ET AL: "A halotolerant type A feruloyl esterase from Pleurotus eryngii", FUNGAL BIOLOGY, vol. 118, no. 3, 1 March 2014 (2014-03-01) , pages 348-357, XP055164553, ISSN: 1878-6146, DOI: 10.1016/j.funbio.2014.01.010
- DATABASE Genebank [Online] NLM; Birren, B. et al.: "The genome sequence of Ustilago maydes", XP002735056, retrieved from http://www.ncbi.nlm.nih.gov/protein/XP_756 329.1 Database accession no. XP_756329.1
- DIANA LINKE ET AL: "An esterase from the basidiomycete Pleurotus sapidus hydrolyzes feruloylated saccharides", APPLIED MICROBIOLOGY AND BIOTECHNOLOGY, vol. 97, no. 16, 1 August 2013 (2013-08-01), pages 7241-7251, XP055164550, ISSN: 0175-7598, DOI: 10.1007/s00253-012-4598-7
- NIETER A ET AL: "A chlorogenic acid esterase from Ustilago maydis with a unique substrate specificity", APPLIED AND ENVIRONMENTAL MICROBIOLOGY, 29 December 2014 (2014-12-29), pages 1-38, XP008174445, ISSN: 0099-2240, DOI: 10.1128/AEM.02911-14 [retrieved on 2014-12-29]
- DATABASE UniProt [Online] 11 June 2014 (2014-06-11), "RecName: Full=Carboxylic ester hydrolase {ECO:0000256|RuleBase:RU361238}; EC=3.1.1.- {ECO:0000256|RuleBase:RU361238};", XP002784557, retrieved from EBI accession no. UNIPROT:X8IZA2 Database accession no. X8IZA2
- DATABASE UniProt [Online] 11 June 2014 (2014-06-11), "RecName: Full=Carboxylic ester hydrolase {ECO:0000256|RuleBase:RU361238}; EC=3.1.1.- {ECO:0000256|RuleBase:RU361238};", XP002784558, retrieved from EBI accession no. UNIPROT:X8J3Z2 Database accession no. X8J3Z2
- DATABASE UniProt [Online] 5 October 2010 (2010-10-05), "RecName: Full=Carboxylic ester hydrolase {ECO:0000256|RuleBase:RU361238}; EC=3.1.1.- {ECO:0000256|RuleBase:RU361238};", XP002784559, retrieved from EBI accession no. UNIPROT:D8QHC3 Database accession no. D8QHC3 & Robin A Ohm ET AL: "Genome sequence of the model mushroom Schizophyllum commune", Nature Biotechnology, vol. 28, no. 9, 11 July 2010 (2010-07-11), pages 957-963, XP055078455, ISSN: 1087-0156, DOI: 10.1038/nbt.1643
- DATABASE UniProt [Online] 5 October 2010 (2010-10-05), "RecName: Full=Carboxylic ester hydrolase {ECO:0000256|RuleBase:RU361238}; EC=3.1.1.- {ECO:0000256|RuleBase:RU361238};", XP002784560, retrieved from EBI accession no. UNIPROT:D8QHD6 Database accession no. D8QHD6
- DATABASE Geneseq [Online] 23 February 2017 (2017-02-23), "Schizophyllum commune PC-2 protein, SEQ ID 80.", XP002784561, retrieved from EBI accession no. GSP:BDL66254 Database accession no. BDL66254 & WO 2016/207351 A2 (GENENCOR INT BV [NL]) 29 December 2016 (2016-12-29)
- DATABASE Geneseq [Online] 23 February 2017 (2017-02-23), "Schizophyllum commune PC-2 protein, SEQ ID 81.", XP002784572, retrieved from EBI accession no. GSP:BDL66255 Database accession no. BDL66255
- NIETER A ET AL: "A chlorogenic acid esterase from Ustilago maydis with a unique substrate specificity", APPLIED AND ENVIRONMENTAL MICROBIOLOGY, 29 December 2014 (2014-12-29), pages 1-38, XP008174445, ISSN: 0099-2240, DOI: 10.1128/AEM.02911-14 [retrieved on 2014-12-29]

## Description

Disclosed are enzymes having chlorogenic acid esterase activity and feruloyl esterase activity, i.e. having hydrolysis and synthesis activity of chlorogenic acid and hydroxycinnamic acid esters derived from the basidiomycetes *Ustilago maydis* (UmChIE), *Rhizoctonia solani* (RsChIE), *Schizophyllum commune* (ScFae1 and ScFae3) and derivatives thereof, respectively. Disclosed are nucleic acids, expression vectors, and host cells comprising the nucleic acid sequences as well as methods of preparing and using the enzyme, e.g., on synthetic as well as complex natural substrates, such as sugar beet pectin, destarched wheat bran and coffee pulp. Disclosed is the use of enyzmes having chlorogenic acid esterase activity and/or feruloyl esterase activity, in particular, enzymes with such activities derived from basidiomycetes, for dough softening, as well as food products comprising said enzymes.

Plant cell walls consist of a complex set of polysaccharides including cellulose, hemicellulose and pectin. This polysaccharide network forms three-dimensional structures containing ester-linked hydroxycinnamates (1). These phenolic acids (caffeic, ferulic and p-coumaric acid) influence not only the rigidity and mechanical properties of the cell wall, but play also a role in plant defense (2). They are linked differently to sugar residues in plants: (A) in grasses such as wheat, barley or maize, ferulates and p-coumarates are mainly found esterified to the O-5 position of arabinose residues, whereas (B) in pectin of dicotyledons, such as spinach or sugar beet, ferulic acid is esterified to the O-2 position of arabinose and 0-6 position of galactose residues (3). Furthermore, hydroxycinnamates also exist as soluble ester conjugates of quinic acid, with the best known example chlorogenic acid (4). Particularly high concentrations of chlorogenic acid are present in coffee, apple, pear and potato tuber (4). Especially health, cosmetic and pharmaceutical industries are interested in hydroxycinnamates due to their anticarcinogenic, anti-inflammatory and antioxidant properties (5). Consequently, enzymes able to release these phenolic compounds are of special interest for their potential industrial and medical application.

One group of these enzymes are feruloyl esterases (FAE; EC 3.1.1.73), a subclass of the carboxylic ester hydrolases (EC 3.1.1). They hydrolyze the ester linkage between ferulic acid or related cinnamic acids and complex plant cell wall polysaccharides (6). Over the years, a large number of FAEs were isolated and characterized mainly from bacteria, yeasts and fungi (7-10). Crepin et al. (10) developed a classification system for FAEs dividing them into four types (A - D) based on their substrate specificity supported by primary sequence identity. Type A FAEs are active on methyl ferulate (MFA), methyl *p*-coumarate (M*p*CA), methyl sinapate (MSA), but not on methyl caffeate (MCA). In contrast Type B FAEs hydrolyze MFA, M*p*CA and MCA, while Type C and D act on all four substrates. Only Type A and D are able to release diferulates from complex substrates.

However, there were only few studies focusing on chlorogenic acid hydrolase (EC 3.1.1.42). To date, a small number of chlorogenic acid esterases are published, all from *Aspergillus* sp.. Schöbel and Pollmann (11, 12) isolated and partially characterized a specific chlorogenic acid esterase from a pectinolytic enzyme preparation of *Aspergillus niger.* More recently, Asther et al. (13) demonstrated the efficiency of another *A. niger* enzyme to release caffeic acid from industrial by-products such as coffee pulp and apple marc. The corresponding gene was homologously overexpressed by Benoit et al. (14), and the properties of the recombinant and native enzyme were compared. Adachi et al. induced a chlorogenic acid esterase in mycelia of *Aspergillus sojae* with either instant coffee powder or coffee pulp (15). A hydroxycinnamic acid ester hydrolase from *Aspergillus japonicus* (16) and a cinnamate esterase from *A. niger* (17) also hydrolyzed chlorogenic acid. Additionally, activity towards chlorogenic acid was shown for the feruloyl esterase FAEB from *A. niger* (18), AoFaeB and AoFaeC from *Aspergillus oryzae* (19), TsFaeC from *Talaromyces stipitatus* (20) and Fae1A from *Anaeromyces mucronatus* (21). Couteau et al. (22) isolated and characterized human colonic bacteria acting on chlorogenic acid.

In light of this, the inventors solved the problem of providing a new enzyme having chlorogenic acid esterase activity and/or feruloyl esterase activity with alternative characteristics and substrate specificity which can be used, e.g., to generate the respective products. For example, hydroxycinnamate can be released from industrial by-products. Furthermore, they surprisingly found new applications of enzymes having chlorogenic acid esterase activity and/or feruloyl esterase activity, in particular, chlorogenic acid esterase activity, in baking.

This problem is solved, e.g., by the subject matter of the claims.

The present disclosure provides specific uses of an enzyme having phenylpropanoid esterase activity, as well as specific phenylpropanoid esterase enyzmes. In particular, the disclosure provides specific uses of an enzyme with chlorogenic acid esterase activity and/or feruloyl esterase activity comprising a sequence having at least 85%, at least 90%, at least 95%, at least 96%, at least 97%, at least 98%, or at least 99% amino acid identity to SEQ ID NO: 1.

The disclosure provides an enzyme with chlorogenic acid esterase activity and/or feruloyl esterase activity comprising
a. a sequence having at least 85% amino acid identity to SEQ ID NO: 1, and not comprising SEQ ID NO: 3, or
b. a sequence having at least 85% amino acid identity to SEQ ID NO: 1 and not comprising SEQ ID NO: 19, or
c. a sequence having at least 85% amino acid identity to SEQ ID NO: 22, or
d. a sequence having at least 85% amino acid identity to SEQ ID NO: 26, or
e. a sequence having at least 85% amino acid identity to SEQ ID NO: 30.

The enzyme preferably comprises a sequence having SEQ ID NO: 1, and is designated UmChIE. It may also consist of SEQ ID NO: 1. In some embodiments, the enzyme comprising SEQ ID NO: 1 does not comprise the endogenous signaling sequence of SEQ ID NO: 3. In other embodiments, it is the full length sequence of UmChIE provided by the inventors (SEQ ID NO: 17), including the signal sequence. In one embodiment, the present disclosure does not cover an enzyme comprising the sequence of SEQ ID NO: 19 (Um00182.1, GenBank accession no. **XP_756329.1**). An enzyme with chlorogenic acid esterase activity and/or feruloyl esterase activity comprising a sequence having at least 85%, at least 90%, at least 95%, at least 96%, at least 97%, at least 98%, or at least 99% amino acid identity to SEQ ID NO: 1 may also comprise a leucine in position 492 of the sequence (calculated with reference to the positions in SEQ ID NO: 19).

Preferably, the enzyme has chlorogenic acid esterase activity, more preferably, the enzyme has chlorogenic acid esterase activity and feruloyl esterase activity, in particular, type B feruloyl esterase activity (Crepin et al., 2004). Preferably, the enzyme activity is comparable (e.g., at least 70% activity, at least 80% activity or at least 90% activity) to the activity on different at least one substrate as shown for UmChIE, preferably, on all substrates investigated in Example 1.

Surprisingly, the inventors detected chlorogenic acid esterase activity in a basidiomycete, *Ustilago maydis,* and were able to isolate the responsible enzyme, UmChIE, despite problems with melanin production when standard nutrition medium was used. Only specific culture conditions, which induced higher esterase activities and avoided melanin production, allowed for purification of the enzyme. The enzyme was found to have an exceptionally broad substance specificity, as it has feruloyl esterase activity in addition to chlorogenic acid esterase activity. The enzyme was fully sequenced and characterized.

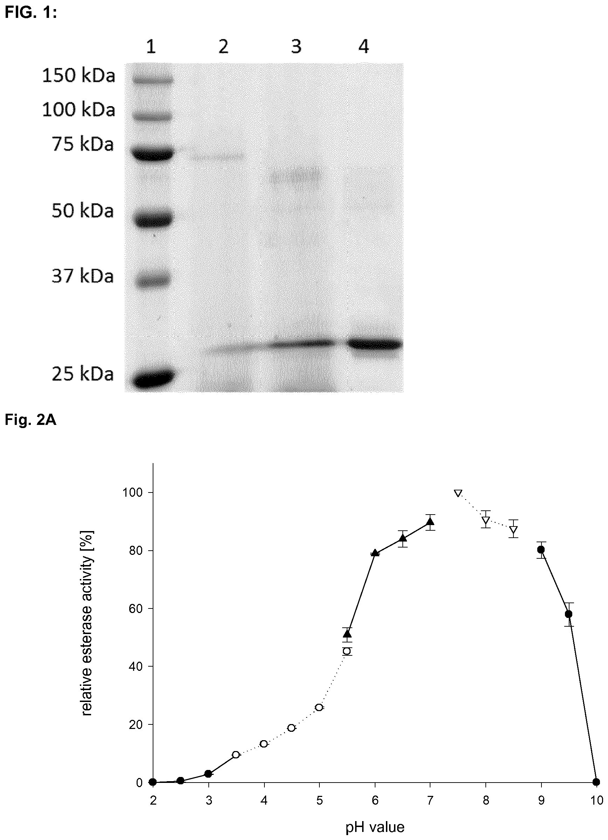

The molecular mass of the novel enzyme, as determined by denaturing SDS-PAGE, preferably is 71 kDA, as shown for the chlorogenic acid esterase isolated from *U. maydis* (Fig.1, lane 2). The apparent mass of the native enzyme estimated by size exclusion chromatography shows that UmChIE is a monomer. UmChIE thus differs from the other chlorogenic acid esterase enzymes described in literature. While Asther et al. (13) and Adachi et al. (15) identified the native chlorogenic acid hydrolases as homodimers of about 2 x 80 kDa, Benoit et al. (14) suggested the recombinant *A. niger* enzyme to exist as tetrameric form. Schöbel and Pollmann (12) characterized the second known chlorogenic acid esterase from *A. niger* also as a tetramer (4 x 60 kDa), whereas Okamura and Watanabe (16) described the hydroxycinnamic acid ester hydrolase of *A. japonicus* as a monomer of 145 kDa.

The sequence largely corresponds to the hypothetical protein Um00182.1 (SEQ ID NO: 19) from *U. maydis* 521 (GenBank accession no. **XP_756329.1**), which was annotated via a genome project and roughly classified as a member of the esterase_lipase superfamily containing a region specific for carboxylesterases. No further information on that the sequence had been available before the present invention, e.g., there are no indications that the protein was ever prepared and isolated, and there was no information on its glycosylation or on its actual enzymatic activity.

The complete coding sequence of UmChIE as provided by the inventors has a length of 1,758 bp, corresponding to a protein of 585 amino acids (aa). The full aa sequence of UmChIE (SEQ ID NO: 17) purified from *U. maydis* by the inventors possesses three differences on nucleotide level which lead to one change on the amino acid level at position 492 (proline to leucine) compared to the sequence of Um00182. The UmChIE sequence showed highest similarity (89 %) and identity (81 %) to a carboxylesterase from *Pseudozyma hubeiensis* SY62 (GenBank accession no. **GAC96757**). In contrast, only 29 % identity was obtained with an aa sequence of the chlorogenic acid hydrolase CHIE from *A. niger* (GenBank accession no. **DQ993161**). A signal peptide of 21 aa was identified at the N-terminus of the UmChIE sequence based upon prediction using the web-based program SignalP 4.01. The predicted molecular mass of the mature protein was calculated to be 63.7 kDa using the Compute p//Mw tool of ExPASy. The discrepancy to the molecular mass deduced from denaturing SDS-PAGE (71 kDa) indicated a glycosylated protein. Nine potential *N-*glycosylation sites were identified at positions 5, 109, 151, 298, 308, 370, 387, 487 and 558 (http://www.cbs.dtu.dk/services/NetNGlyc/). Treatment with endoglycosidase H results in a deglycosylated protein of approximately 63 kDa (Fig. 1, lane 3) suggesting that the UmChIE contains Asn-linked high-mannose oligosaccarides. Similar results were described for the chlorogenic acid hydrolase CHIE from *A. niger* (14). For CHIE a mass difference of 25 kDa was caused by glycosylation. Consequently, UmChIE has a lower degree of *N*-glycosylation than ChIE. This also explains the thermolability of UmChIE shown below, as it has been reported that *N-*glycosylation is important not only for protein folding, but also for thermostability (55).

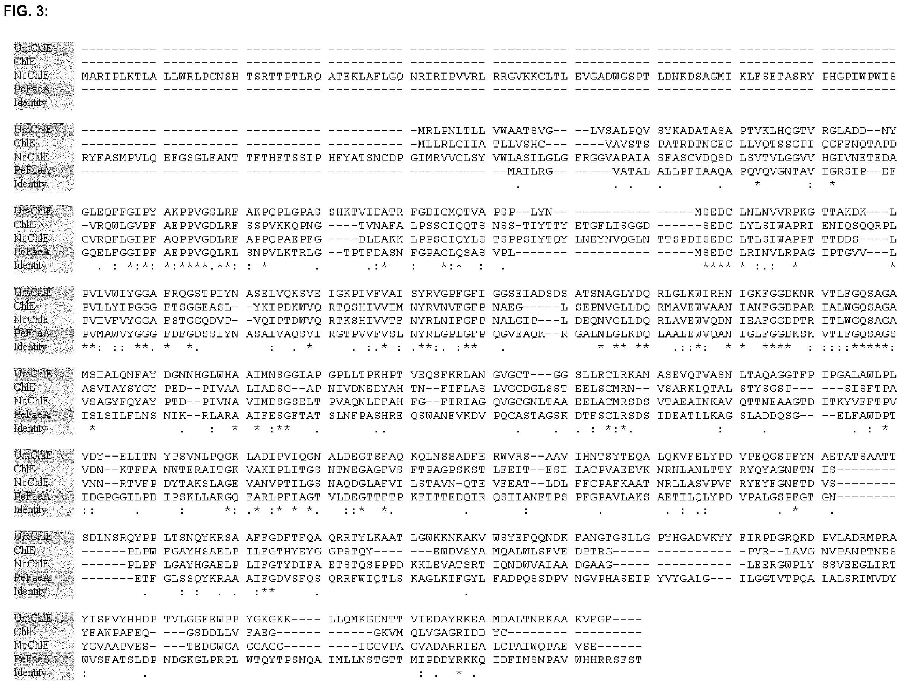

The sequence of the UmChIE contains a G-X-S-X-G motif (Fig. 3) characteristic of the serine esterase family (56, 57). Here, the motif G-Q-S-A-G is the same as in the CHIE sequence. This motif was also found in feruloyl esterases of *P*. *eryngii* PeFaeA (29) and *P. sapidus* Est1 (25), while other feruloyl esterase often possess G-H-S-L-G and G-C-S-T-G as conserved motifs (19, 58). The catalytic triad of UmChIE is predicted to consist of Ser228, Glu360 and His497 (http://pfam.sanger.ac.uk/). Likewise, ChIE contains a catalytic triad of Ser-Glu-His, while the annotated chlorogenic acid esterase from *N. crassa* (GenBank accession no. **EAA32507.3)** showed the glutamic acid replaced by aspartic acid (Fig. 3). Udatha et al. (48) reported a catalytic triad of Ser-Asp-His for 324 analyzed feruloyl esterase sequences. Nonetheless, two feruloyl esterases were also known containing a catalytic triad of Ser-Glu-His (25, 29). It could accordingly not have been predicted based on the sequence that UmChIE is a chlorogenic acid esterase.

The enzyme may have one or more of the following characteristics, preferably, all characteristics listed under a) to j), optionally, all of them:
a) non-gylcosylated or glycosylated at 1, 2, 3, 4, 5, 6, 7, 8, or 9 N-glycosylation sites;
b) monomeric;
c) chlorogenic acid esterase activity at about pH 3-10;
d) pH optimum of chlorogenic acid esterase activity at about pH 5.5-9.5, most preferably at about pH 7.5;
e) stability at pH 4-10;
f) feruloyl esterase activity on the substrates methyl p-coumarate, methyl ferulate, and methyl caffeate, but not methyl sinapate;
g) pi of about 3.0;
h) thermal stability up to about 40°C;
i) optimal activity at about 37°C;
j) optimal activity at about 0.1-3 M NaCl, preferably, 1-3 M NaCl or about 3 M NaCl; and/or
k) comprising a tag suitable for purification, such as a His tag, preferably, at the C-terminus.

In the context of the invention, the term "about" is intended to be understood as "+/- 10%). If "about" relates to a range, it refers to both lower and upper limit of the range. "A" is intended to mean "one or more", if not explicitly mentioned otherwise.

In another embodiment, option c above, the enzyme preferably comprises a sequence having at least 85%, at least 90%, at least 95%, at least 96%, at least 97%, at least 98%, or at least 99% amino acid identity to SEQ ID NO: 22, or, preferably, having SEQ ID NO: 22. The enzyme having SEQ ID NO: 22 is designated RsChIE. It may also consist of SEQ ID NO: 22. In some embodiments, the enzyme comprising SEQ ID NO: 22 does not comprise the endogenous signaling sequence which is additionally comprised in SEQ ID NO: 23. In other embodiments, it comprises the full length sequence of RsChIE provided by the inventors (SEQ ID NO: 23), including the signal sequence.

Preferably, the enzyme has chlorogenic acid esterase activity, more preferably, the enzyme has chlorogenic acid esterase activity and feruloyl esterase activity, in particular, type B feruloyl esterase activity. Preferably, the enzyme activity is comparable (e.g., at least 70% activity, at least 80% activity or at least 90% activity) to the activity on at least one substrate as shown for RsChIE, preferably, all substrates as investigated in Example 2.

After having found chlorogenic acid esterase activity in the basidiomycete, *Ustilago maydis,* and were able to isolate the responsible enzyme, UmChIE, they investigated a further basidiomycete, *Rhizoctonia solani,* for the presence of a chlorogenic acid esterase, and successfully amplified its sequence. The enzyme, RsChIE, was found to have an exceptionally broad substance specificity, comparable to UmChIE, as it has feruloyl esterase activity in addition to chlorogenic acid esterase activity. The enzyme was fully sequenced and characterized.

The molecular mass of the novel enzyme derived from RsChIE, as determined by denaturing SDS-PAGE, preferably is about 70 kDA.

The complete coding sequence of RsChIE as provided by the inventors has a length of 1692 bp, corresponding to a protein of 563 amino acids (aa). A signal peptide of 38 aa was identified at the N-terminus of the RsChIE sequence. The predicted molecular mass of the mature protein was calculated to be 56.9 kDa using the Compute p//Mw tool of ExPASy. The discrepancy to the molecular mass deduced from denaturing SDS-PAGE (70 kDa) indicated a glycosylated protein.

The enzyme comprising a sequence having at least 85% amino acid identity to SEQ ID NO: 22 may have one or more of the following characteristics, preferably, all characteristics listed under a) to g):
a) glycosylation
b) chlorogenic acid esterase activity at about pH 3-7,5;
c) pH optimum of chlorogenic acid esterase activity at about pH 5.5-6.5, most preferably at about pH 6;
d) feruloyl esterase activity on the substrates methyl p-coumarate, methyl ferulate, and methyl caffeate, but not methyl sinapate;
e) thermal stability up to about 40°C;
f) optimal activity at about 30°C; and/or
g) comprising a tag suitable for purification, such as a His tag, preferably, at the C-terminus.

The inventors identified and prepared further enzymes from *S. commune.* Surprisingly, they found that these enzymes, ScFae1 and ScFae3 also had both feruloyl esterase activity and chlorogenic acid esterase activity. Thus, in another embodiment, options d and e above, the enzyme preferably comprises a sequence having at least 85%, at least 90%, at least 95%, at least 96%, at least 97%, at least 98%, or at least 99% amino acid identity to SEQ ID NO: 26 or 30. Preferably, it has SEQ ID NO: 26 or 30. The enzyme having SEQ ID NO: 26 is designated ScFae1. It may also consist of SEQ ID NO: 26. The enzyme having SEQ ID NO: 30 is designated ScFae3. It may also consist of SEQ ID NO: 30. In some embodiments, the enzyme comprising SEQ ID NO: 26 or 30 does not comprise the endogenous signaling sequence which is additionally comprised in SEQ ID NO: 27 or 31. In other embodiments, it comprises the full length sequence of ScFae1 and ScFae3 provided by the inventors (SEQ ID NO: 27 or 31), including the signal sequence.

Preferably, these enzymes have feruloyl esterase activity, in particular, type C and D feruloyl esterase activity. More preferably, the enzyme has chlorogenic acid esterase activity and feruloyl esterase activity. Preferably, the enzyme activity is comparable (e.g., at least 70% activity, at least 80% activity or at least 90% activity) to the activity on at least one substrate as shown for ScFae1 and ScFae3.

The complete coding sequence of ScFae1 as provided by the inventors has a length of 1572 bp, corresponding to a protein of 523 amino acids (aa). A signal peptide of 24 aa was identified at the N-terminus of the ScFae1sequence.

The complete coding sequence of ScFae3 as provided by the inventors has a length of 1569 bp, corresponding to a protein of 522 amino acids (aa). A signal peptide of 23 aa was identified at the N-terminus of the ScFae3 sequence.

The enzyme comprising a sequence having at least 85% amino acid identity to SEQ ID NO: 26 or 30 may have one or more of the following characteristics, preferably, all characteristics listed under a) to f):
a) glycosylation
b) feruloyl acid esterase activity at about pH 3-9,5;
c) pH optimum of feruloyl acid esterase activity at about pH 4.5-9.5, most preferably at about pH 5-8.5;
d) feruloyl esterase activity on the substrates methyl *p*-coumarate, methyl ferulate, methyl caffeate, and methyl sinapate;
e) thermal stability up to about 50°C;
f) optimal activity at about 30-50°C, preferably about 39-43°C; and/or
comprising a tag suitable for purification, such as a His tag, preferably, at the C-terminus.

The enzyme comprising a sequence having at least 85% amino acid identity to SEQ ID NO: 26 or 30 have highest avtivity in reacting methyl *p*-coumarate (100 %). Activity on chlorogenic acid esterase is about 10% of that on coumaric acid methyl ester. The enzymes preferably are type C oder D FAEs, as they can also react methyl sinapate. Preferred enzymatic activities of enyzmes having at least 85% amino acid identity to SEQ ID NO: 26 or 30 are shown in the examples below.

In some embodiments the enzyme is obtainable from a basidiomycete, and, if it has at least 85% sequence identity with UmChIE preferably from the *Ustilago* genus, most preferably from *Ustilago maydis,* or, if it has at least 85% sequence identity with RsChIE, it is preferably obtainable from a basidiomycete from the *Rhitoctonia* genus, most preferably, *R. solani.* Alternatively, it may be obtainable form a basidiomycete preferably from the *Schizophyllum* genus, most preferably from *Schizophyllum commune,* and have at least 85% sequence identity with ScFae1 or ScFae3. Homologues from other basidiomycetes are routinely obtainable for the skilled person, e.g., comprising using a probe from the nucleotide sequence encoding the enzyme of the invention for screening a cDNA bank of the basidiomycete under low stringency, medium stringency or high stringency conditions, thus isolating a homologous nucleic acid, and expressing the same in a suitable host cell. Alternatively, purification methods such as a combination of chromatographic steps, e.g., anion exchange chromatography on Q Sepharose FF, preparative IEF, and a combination of hydrophobic interaction chromatography and affinity chromatography, such as polyamide affinity chromatography can be employed to purify homologous enzymes from other basidiomycetes. Suitable methods are described in the examples in detail.

In other embodiments, the enzyme is obtainable by recombinant expression in a suitable host cell, e.g., in yeast, preferably in *Saccharomyces, Pichia, Kluyveromyces, Hansenula and Yarrowia* genera, more preferably in *Kluyveromyces lactis, Saccharomyces cerevisiae, or Schizosaccharomyces pombe,* and most preferably in *Pichia pastoris, e.g., P*. *pastoris* GS115. The successful heterologous production of the active enzyme of the invention in *P. pastoris,* as described in detail below, opens up opportunities for applications and enzyme engineering. Identification of the catalytic centers and comparison with known enzymes further simplifies enzyme engineering, e.g., generation of mutants having only chlorogenic acid esterase activity or feruloyl esterase activity, having modified substrate specificity, or still better tolerance towards, e.g., low or high pH, salt, metal ions or high temperatures. In general, for engineered mutants which maintain the enzymatic activity of the UmChE, RsChIE, ScFae1 or ScFae3, respectively, the catalytic centers and, preferably, residues which also contact the substrates, e.g., residues adjacent to the catalytic residues, or such residues which form the catalytic pocket, should be conserved.

The present disclosure also relates to a nucleic acid encoding the enzyme of the invention, i.e., an enzyme with chlorogenic acid esterase activity and/or feruloyl esterase activity having at least 85%, preferably, 90%, 95%, 96%, 97%, 98%, 99% or 100% amino acid identity to SEQ ID NO: 1. In particular, the disclosure relates to a nucleic acid encoding an enzyme with chlorogenic acid esterase activity and/or feruloyl esterase activity having at least 85%, preferably, 90%, 95%, 96%, 97%, 98%, 99% or 100% amino acid identity to SEQ ID NO: 1 not comprising SEQ ID NO: 4, or not encoding an enzyme comprising SEQ ID NO: 19.

The inventors surprisingly found that it was advantageous to use a heterologous signal sequence for expression. Accordingly, the nucleic acid of the invention preferably does not comprise SEQ ID NO: 4, the nucleic acid sequence encoding the native signal sequence. The nucleic acid of the invention may comprise the *U. maydis* sequence of SEQ ID NO: 2 encoding the enzyme of SEQ ID NO: 1 without the signal peptide, or consist thereof, or it may comprise the *U. maydis* full length nucleotide sequence SEQ ID NO: 18. Preferably, it does not comprise a sequence encoding the hypothetical gene bank protein Um00182.1 having SEQ ID NO: 19.

The nucleic acid of the invention may also encode a protein of SEQ ID NO: 1 with a sequence different from SEQ ID NO: 2 by virtue of the degeneracy of the genetic code.

The present disclosure also relates to a nucleic acid encoding the further enzyme of the invention, i.e., an enzyme with chlorogenic acid esterase activity and/or feruloyl esterase activity having at least 85%, preferably, 90%, 95%, 96%, 97%, 98%, 99% or 100% amino acid identity to SEQ ID NO: 22.

The inventors surprisingly found that it was advantageous to use a heterologous signal sequence for expression. Accordingly, the nucleic acid of the invention in one option does not comprise the nucleic acid sequence encoding the native signal sequence. The nucleic acid of the invention may comprise the *R*. *solani* sequence of SEQ ID NO: 20 encoding the enzyme of SEQ ID NO: 22 without the signal peptide, or consist thereof, or it may comprise the *U. maydis* full length nucleotide sequence SEQ ID NO: 21. The nucleic acid of the invention may also encode a protein of SEQ ID NO: 22 with a sequence different from SEQ ID NO: 20 or SEQ ID NO:21 by virtue of the degeneracy of the genetic code.

The present disclosure also relates to a nucleic acid encoding the further enzyme of the invention, i.e., an enzyme with chlorogenic acid esterase activity and/or feruloyl esterase activity having at least 85%, preferably, 90%, 95%, 96%, 97%, 98%, 99% or 100% amino acid identity to SEQ ID NO: 26 or SEQ ID NO:30.

The inventors surprisingly found that it was advantageous to use a heterologous signal sequence for expression. Accordingly, the nucleic acid of the invention in one option does not comprise the nucleic acid sequence encoding the native signal sequence. The nucleic acid of the invention may comprise the S. *commune* sequence of SEQ ID NO: 24 or SEQ ID NO:28 encoding the enzyme of SEQ ID NO: 26 or SEQ ID NO:30, respectively, without the signal peptide, or consist thereof, or it may comprise the *U. maydis* full length nucleotide sequence SEQ ID NO: 25 or 29. The nucleic acid of the invention may also encode a protein of SEQ ID NO: 26 or 30 with a sequence different from SEQ ID NO: 24 or 28 or SEQ ID NO: 25 or 29 by virtue of the degeneracy of the genetic code.

The nucleic acid of the disclosure may be an expression vector comprising the nucleic acid encoding the enzyme of the invention, which is functionally linked to a heterologous promoter, e.g., an alcohol oxidase (AOX1) promoter, glyceraldehydes-3-phosphate dehydrogenase (GAP) promoter, alcohol dehydrogenase (ADH) promoter, or galactose (GAL) promoter, preferably, an alcohol oxidase promoter (AOX1). The nucleic acid encoding the enzyme is preferably functionally linked to a nucleic acid encoding the heterologous signal sequence, e.g., an α-factor secretion signal such as *Saccharomyces cerevisiae* α-factor secretion signal, an invertase secretion signal or a lysozyme secretion signal.

Functional linkage requires that the sequences are, insofar, as they encode amino acids, in frame, and in general, that they are positioned in a way that allows them to perform their respective intended functions. Accordingly, a promoter has to be positioned in a way leading to expression of the protein encoded by the functionally linked nucleic acid, and a signal sequence has to lead to secretion of the linked protein in the selected host cell. Optionally, the signal sequence can be cut off after secretion.

It the nucleic acid is not linked to a signal sequence, the expressed protein can be purified from the host cells, e.g., after lysing said cells. Alternatively, if the expressed protein is secreted into the culture supernatant, it can be purified from the supernatant.

A nucleotide sequence encoding for an affinity tag, such as a hexahistidine tag, may be incorporated at the C-terminus or N-terminus. This allows for easy purification of the enzyme of the invention, e.g., from the culture supernatant, with a suitable affinity column, e.g., an Ni-NTA. Alternatively, other purification methods, such as anion exchange chromatography on Q Sepharose FF, preparative IEF, and/or a combination of hydrophobic interaction chromatography and affinity chromatography, such as polyamide affinity chromatography, may be employed, or the cultures or culture supernatant may be employed as such.

The disclosure also relates to a suitable host cell, e.g., as mentioned above, comprising the expression vector of the invention. The host cell preferably is not *U. maydis, S. commune* or *R. solani,* i.e., the host cell is heterologous.

One surprising feature of the enzyme of the disclosure is its broad substrate specificity towards both chlorogenic acid esters and/or ferulic acid esters, which is described in detail in the examples below. Chlorogenic acid esterase activity and/or feruloyl esterase activity can be determined as described in the examples below. In particular, chlorogenic acid activity is determined by the capability of hydrolyzing chlorogenic acid. Feruloyl esterase activity is determined by the capability of hydrolyzing the 4-hydroxy-3-methoxycinnamoyl (feruloyl) group from an esterified sugar, which is usually arabinose. An enzyme having the respective enzymatic activity may have at least 10%, at least 20%, at least 25%, at least 30%, at least 33,3%, at least 40%, at least 50%, at least 60%, at least 70%, at least 80%, at least 90% or at least 100% of the respective activity determined for either of the enzyme described in the examples below. It may also have higher activity.

The disclosure also provides the use of an enzyme with chlorogenic esterase activity and/or feruloyl esterase activity comprising a sequence having at least 85% to SEQ ID NO: 1 for hydrolyzing a substrate and/or generating a product selected from the group comprising:
(a) a chlorogenic acid, i..e, an ester of quinic acid with a phenylpropanoic acid selected from the group consisting of caffeic acid, ferulic acid and p-coumaric acid, preferably, caffeic acid; and/or
(b) an ester of a saccharide or C1- or C2-alcohol with a phenylpropanoic acid optionally comprising exactly one methoxy on the benzoic ring, wherein the phenylpropanoic acid preferably is caffeic acid, ferulic acid or p-coumaric acid, most preferably, caffeic acid.

The disclosure also provides the use of an enzyme with chlorogenic esterase activity and/or feruloyl esterase activity wherein the enzyme is selected from the group comprising an enzyme having at least 85% amino acid identity to SEQ ID NO: 22, an enzyme having at least 85% amino acid identity to SEQ ID NO: 26, and an enzyme having at least 85% amino acid identity to SEQ ID NO: 30 for hydrolyzing a substrate and/or generating a product selected from the group comprising:
(a) a chlorogenic acid, i..e, an ester of quinic acid with a phenylpropanoic acid selected from the group consisting of caffeic acid, ferulic acid and p-coumaric acid, preferably, caffeic acid; and/or
(b) an ester of a saccharide or C1- or C2-alcohol with a phenylpropanoic acid optionally comprising exactly one methoxy on the benzoic ring, wherein the phenylpropanoic acid preferably is caffeic acid, ferulic acid or p-coumaric acid, most preferably, caffeic acid.

Preferably, the enzyme is used for hydrolyzing one or more of said substrates, but the inventors found that under suitable conditions, the enzyme can also be used for the synthesis reaction.

The disclosure further provides a method of hydrolyzing a substrate selected from the group comprising:
(a) an ester of quinic acid with a phenylpropanoic acid selected from the group consisting of caffeic acid, ferulic acid and p-coumaric acid preferably, caffeic acid; and/or
(b) an ester of a saccharide or C1- or C2-alcohol with a phenylpropanoic acid optionally comprising exactly one methoxy on the benzoic ring, wherein the phenylpropanoic acid preferably is caffeic acid, ferulic acid or p-coumaric acid, most preferably, caffeic acid.

Said method comprises a step wherein an enzyme with chlorogenic esterase activity and/or feruloyl esterase activity comprising a sequence having at least 85% amino acid identity to SEQ ID NO: 1 is brought into contact with said substrate under suitable conditions, such as 37 °C, pH 7.5.

The disclosure further provides a method of hydrolyzing a substrate selected from the group comprising:
(a) an ester of quinic acid with a phenylpropanoic acid selected from the group consisting of caffeic acid, ferulic acid and p-coumaric acid preferably, caffeic acid; and/or
(b) an ester of a saccharide or C1- or C2-alcohol with a phenylpropanoic acid optionally comprising exactly one methoxy on the benzoic ring, wherein the phenylpropanoic acid preferably is caffeic acid, ferulic acid or p-coumaric acid, most preferably, caffeic acid.

Said method comprises a step wherein an enzyme with chlorogenic esterase activity and/or feruloyl esterase activity, wherein the enzyme is selected from the group comprising, an enzyme having at least 85% amino acid identity to SEQ ID NO: 22, an enzyme having at least 85% amino acid identity to SEQ ID NO: 26, and an enzyme having at least 85% amino acid identity to SEQ ID NO: 30, is brought into contact with said substrate under suitable conditions, such as 30 °C, pH 6 for an enzyme having at least 85% amino acid identity to SEQ ID NO: 22.

The methods relating to the compounds under (a) are or special interest in the context of the invention.

The substrate may be a component of a natural material. If the substrate is a component of a natural material, the ester under (b) normally is an ester with a saccharide, such as a polysaccharide, e.g., di- or trisaccaride. The ester may, e.g., be a feruloylated mono-, di- and trisaccharides (F-A, F-AX, F-AXG). Further preferred substrates are described in the experimental part below.

The present disclosure further provides a method of preparing a product selected from the group comprising
a) quinic acid; and/or
b) a phenylpropanoic acid optionally comprising exactly one methoxy on the benzoic ring, preferably, caffeic acid, ferulic acid and/or p-coumaric acid, most preferably, caffeic acid; and/or
c) a saccharide such as a mono- or polysaccharide.

The products of group a) and b) and c) are products of the hydrolysis reactions catalyzed by the enzyme of the invention. Suitable conditions can be determined by the skilled person or are described in the examples below, e.g., 37°C, pH 7.5. As the enzyme can, under certain conditions, e.g., described in the examples below or the literature cited therein, also synthesize the esters, the present disclosure further provides a method of preparing a product selected from the group comprising
d) an ester of quinic acid with a phenylpropanoic acid selected from the group consisting of caffeic acid, ferulic acid and p-coumaric acid, preferably, caffeic acid; and/or
e) an ester of a saccharide or C1- or C2-alcohol with phenylpropanoic acid optionally comprising exactly one methoxy on the benzoic ring, e.g., caffeic acid, ferulic acid or p-coumaric acid, preferably, caffeic acid.

Said methods comprise a step wherein an enzyme with phenylpropanoic acid esterase activity, preferably, chlorogenic esterase activity and/or feruloyl esterase activity comprising a sequence having at least 85% amino acid identity to SEQ ID NO: 1 is brought into contact with the educts of the catalyzed reaction generating the respective product under suitable conditions.

Alternatively, said methods comprise a step wherein an enzyme with phenylpropanoic acid esterase activity, preferably, chlorogenic esterase activity and/or feruloyl esterase activity, wherein the enzyme is selected from the group comprising, an enzyme having at least 85% amino acid identity to SEQ ID NO: 22, an enzyme having at least 85% amino acid identity to SEQ ID NO: 26, and an enzyme having at least 85% amino acid identity to SEQ ID NO: 30, is brought into contact with the educts of the catalyzed reaction generating the respective product under suitable conditions.

Said methods optionally comprise a further step wherein the product is isolated. It may further be formulated, e.g., in a composition for cosmetic application or, preferably, in a pharmaceutical composition for medical application, e.g., to a human patient.

An extracellular halotolerant chlorogenic acid esterase from *U. maydis,* UmChIE, was purified to homogeneity and characterized. A further chlorogenic acid esterase, RsChIE, was derived from *R. solani,* and enzymes which are mainly characterized as feruloyl esterases, ScFae1 and ScFae3, were derived from *S. commune.* This allowed for provision of the enzymes of the invention. Compared to previously described chlorogenic acid hydrolases said enzymes appear to display a broader substrate profile. Especially the ability to release not only caffeic acid, but also *p*-coumaric and ferulic acid from agro-industrial waste material, such as coffee pulp, renders these enzymes attractive candidates for industrial applications. The phenolic acids liberated may be used as natural aroma precursors or antioxidants in food and cosmetics.

A recent study in mice did not confirm some of the supposed beneficial effects of dietary chlorogenic acid; quite the contrary of earlier positive indications was found (59). Depending on the outcome of this debate, the enzyme of the invention could provide a general and efficient tool for reducing dietary risks involved in the consumption of higher levels of chlorogenic acid.

An enzyme of the invention may be contacted with a natural material, e.g., food, which may be for human or animal consumption, such as apples, pears and potato tubers, or agro-industrial waste material, such as coffee pulp, destarched wheat bran, and sugar beet pectin. The invention thus provides a method of preparing natural material such as food comprising a reduced amount of an ester of quinic acid with a phenylpropanoic acid selected from the group consisting of caffeic acid, ferulic acid and p-coumaric acid, preferably, caffeic acid. The present invention also provides a method for reducing dietary risks involving the consumption of chlorogenic acid, in particular, high levels thereof, comprising contacting a food product with an enzyme with chlorogenic esterase activity and/or feruloyl esterase activity comprising a sequence having at least 85% amino acid identity to SEQ ID NO: 1, SEQ ID NO: 22, SEQ ID NO: 26 or SEQ ID NO: 30.

The disclosure also relates to a food product, e.g., a bakery product, such as a baked food product or a food product intended for baking, comprising an enzyme of the invention, wherein said enzyme may comprise a sequence having at least 85% amino acid identity to SEQ ID NO: 1, SEQ ID NO: 22, SEQ ID NO: 26 or SEQ ID NO: 30.

The inventors surprisingly found that the enzymes of the disclosure as well as other enzymes having chlorogenic acid esterase and/or feruloyl esterase activity, preferably at least chlorogenic acid esterase activity, can be used for dough softening (i.e., decreasing dough strength). The enzyme used for dough softening preferably is an enzyme having at least 85% amino acid identity to SEQ ID NO: 1 or an enzyme having at least 85% amino acid identity to SEQ ID NO: 22. Most preferably, it is an enzyme of the invention as described above, e.g., UmChIE or RsChIE.

The inventors also found that other enzymes having chlorogenic acid esterase and/or feruloyl esterase activity, which enzymes are preferably derived from a basidiomycete, such as *Schizophyllum commune.* ScFae1 and ScFae, can also be used for dough softening. An enzyme having at least 85% sequence identity to ScFae1 or ScFae3, and preferably having comparable enzymatic activity as chlorogenic acid esterase and/or feruloyl esterase may also be used for dough softening.

Accordingly, the disclosure also provides a method of preparing a bakery product, comprising contacting dough of the bakery product with an enzyme having chlorogenic acid esterase activity and/or feruloyl esterase activity, preferably at least chlorogenic acid esterase activity, wherein the enzyme is selected from the group comprising an enzyme having at least 85% amino acid identity to SEQ ID NO: 1, SEQ ID NO: 22, SEQ ID NO:26 and SEQ ID NO:30. Most preferably, it is an enzyme of the disclosure as described above, e.g., UmChIE or RsChIE.

The enzymes used for dough softening in the context of the disclosure are thus characterized by their chlorogenic acid esterase and/or feruloyl esterase activity, and they are selected from the group comprising UmChIE, RsChIE, ScFae1 und ScFae3 and enzymes at least 85% sequence identity to any of said enzymes, and having a comparable enzymatic activity.

This dough softening property can be used advantageously in several baking applications, e.e., for reduction of dough shrinkage. Preferably, the enzymes are used for preparation of hard biscuits and crackers, where they may contribute to reduction of dough shrinkage and baked good deformation, for preparation of pizza crusts, where they may improve moulding properties and reduce dough shrinkage. In preparation of sheeted (laminated) dough, e.g. for croissants or Danish pastry, the enzymes may Improve extension and sheeting properties.

Without willing to be bound by the theory, the technological effect may be based on the reduced capability of the dough to retract into the form before moulding, due to the reduced strength of the gluten-pentosan network.

The enzymes may be used in dough preparation together with other enzymes, e.g., xylanase and/or amylase, or without other enzymes.

Furthermore, the significant pH stability of UmChIE under alkaline conditions makes the enzyme particularly suitable for the treatment of pulp in the paper industry (paper pulp). It may be used alone or in combination with one or more different enzyme(s), e.g., a hemicellulase, a xylanase, a β-xylosidasen, an α-arabinofuranosidase or a pectinase. The inventors found that the simultaneous incubation of UmChIE with *T. viride* xylanase (0.025 U) increased the amount of released ferulic acid from DSWB more than twelvefold. This result can be explained by the action of endoxylanases producing shorter-chain xylo-oligosaccharides which are more accessible substrates for esterases.

In the methods and uses of the disclosure all specific enzymes disclosed above may be used, including an enzyme having SEQ ID NO: 19.

### BRIEF DESCRIPTION OF THE DRAWINGS

**FIG. 1** shows an SDS-PAGE analysis of the purified chlorogenic acid esterase (UmChIE) from *U*. *maydis* and endoglycosidase H treated enzyme. Precision plus ProteinTM Standard (1), purified UmChIE after polyamide column (2), deglycosylated UmChIE after treatment with endogylcosidase H for 1 h at 37 °C (3), endoglycosidase H (4).

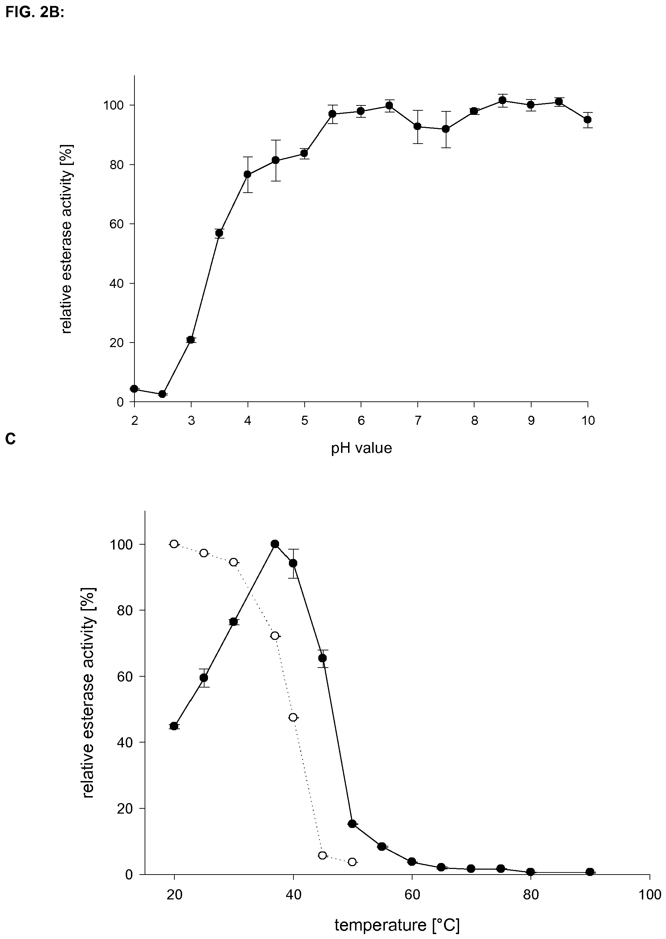

**FIG. 2** illustrates the effect of pH and temperature on the UmChIE activity. (A) pH optimum is determined using glycine buffer (filled circle), acetate buffer (open circle), Bis-Tris buffer (filled triangle) and Tris buffer (open inverted triangle); (B) pH stability; (C) temperature optimum (filled circle) and thermostability profile (open circle). For pH and thermostability measurements residual activities are determined after preincubating the enzyme for 1 h in the buffer systems above (pH 2-10) and at temperatures in the range of 20-60 °C, respectively. Remaining enzyme activity is determined after further incubation (1 h, 37 °C) with 1 mM methyl ferulate. Relative enzyme activity [%] is defined as the percentage of activity detected with respect to the maximum observed esterase activity in each experiment. Values are the average of triplicate experiments, with standard deviation shown as error bars.
**FIG. 3** illustrates the amino acid sequence alignment of *Ustilago maydis* UmChIE (GenBank accession no. HG970190) with chlorogenic acid esterase from *Aspergillus niger* CHIE (GenBank accession no. DQ993161), chlorogenic acid esterase from *Neurospora crassa* (NcChIE, GenBank accession no. EAA32507.3) and feruloyl esterase from *Pleurotus eryngii* PeFaeA (GenBank accession no. CDI44666.1). Peptide fragments identified by ESI-MS/MS- analysis are underlined. The catalytic triad of serine, glutamic/ aspartic acid and histidine is highlighted by boxes.

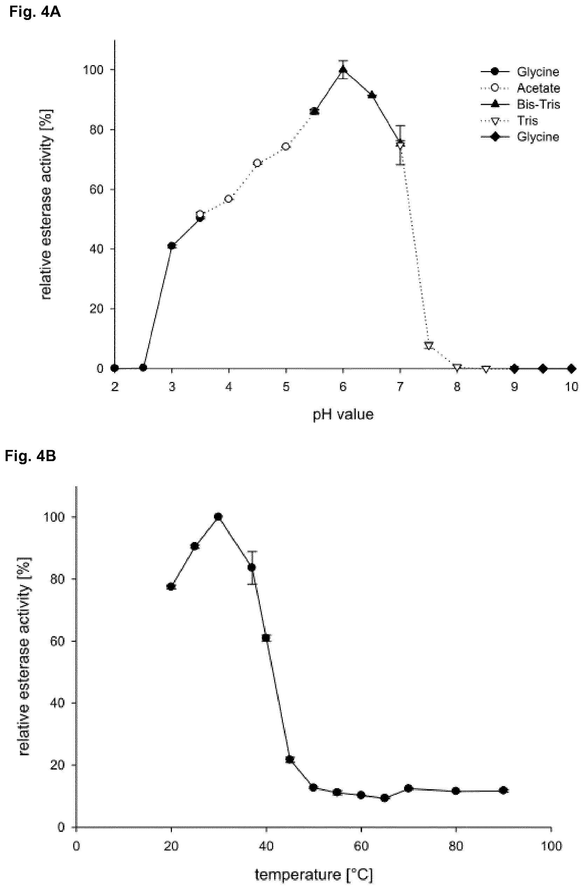

**FIG. 4** illustrates the effect of pH and temperature on the RsChIE activity. (A) pH optimum is determined using glycine buffer (filled circle), acetate buffer (open circle), Bis-Tris buffer (filled triangle) and Tris buffer (open inverted triangle); (B) temperature optimum (filled circle). Values are the average of triplicate experiments, with standard deviation shown as error bars.

### EXAMPLES

### Materials and methods

### Chemicals and substrates

All chemicals used had highest purity grade and were purchased from Carl Roth (Karlsruhe, Germany), Merck (Darmstadt, Germany), Fluka (Buchs, Switzerland) or Sigma-Aldrich (Taufkirchen, Germany), unless otherwise noted. Methyl and ethyl ferulate were obtained from Alfa Aesar (Karlsruhe, Germany). Methyl caffeate, p-coumarate, and sinapate were synthesized according to Borneman et al. (23). Feruloylated saccharides (5-O-*trans*-feruloyl-L-arabinofuranose (F-A); *β*-D-xylopyranosyl-(1→2)-5-O-*trans*-feruloyl-L-arabinofuranose (F-AX); *α*-L-galactopyranosyl-(1→2)-*β*-D-xylopyranosyl-(1→2)-5-O-*trans*-feruloyl-L-arabinofuranose(F-AXG)) prepared from destarched corn bran were kindly supplied by M. Bunzel (KIT, Karlsruhe, Germany). These esterase substrates were purified and identified as described (24, 25). Sugar beet pectin was from Herbstreith & Fox (Neuenbürg, Germany), coffee was from Tchibo (Hamburg, Germany) and wheat bran was from Alnatura (Lorsch, Germany). PCR primers were synthesized by Eurofins MWG Operon (Ebersberg, Germany).

### Cultivation of Ustilago maydis (Uma)

The basidiomycete *U. maydis* was purchased from the Deutsche Sammlung für Mikroorganismen und Zellkulturen (DSMZ, Braunschweig, Germany; No. 17144). The strain was grown on standard nutrition liquid (SNL)-agar and maintained at 4 °C. SNL medium was 30.0 g L⁻¹ D-(+)-glucose-monohydrate, 4.5 g L⁻¹ L-asparagine monohydrate, 3.0 g L⁻¹ yeast extract, 1.5 g L⁻¹ KH₂PO₄, 0.5 g L⁻¹ MgSO₄; 1.0 mL L⁻¹ trace element solution (0.08 g L⁻¹ FeCl₃ x 6 H₂O, 0.09 g L⁻¹ ZnSO₄ x 7 H₂O, 0.03 g L⁻¹ MnSO₄ x H₂O, 0.005 g L⁻¹ CuSO₄ x 5 H₂O, 0.4 g L⁻¹ EDTA); the pH was adjusted to 6.0 with 1 M NaOH prior to autoclaving. For SNL-agar plates, 15 g L⁻¹ agar was added to SNL.

Starter cultures were grown on 10 % (v/v) SNL with 5 % (w/v) wheat bran. Main cultures of *U. maydis* were cultivated in 10 % (v/v) SNL with 1 % (w/v) wheat bran as inductor for esterase production (26). After thirteen days of cultivation at 24 °C and 150 rpm on a rotary shaker (Infors, Bottmingen, Switzerland), cultures were harvested and stored until further processing at -20 °C.

### Enzyme purification

Culture supernatant of *U. maydis* was separated from the frozen cells by centrifugation (9.600 x g, 30 min, 4 °C) followed by filtration under reduced pressure using a 0.45 µm polyethersulfone (PES) membrane (Merck Millipore, Billerica, USA). 0.75 L filtered supernatant was diluted one time with buffer A (50 mM Bis-Tris pH 6.5) and applied to a 25 mL self-casted Q Sepharose FF column (GE Healthcare, Buckinghamshire, England) pre-equilibrated with buffer A. Fractions of 6.0 mL were collected at a flow rate of 4 mL min⁻¹ and monitored for esterase activity. Active fractions eluted with 50 % buffer B (buffer A with 1 M NaCl) were pooled, concentrated and desalted using an ultrafiltration module (30 kDa cut off, PES, Sartorius, Göttingen, Germany). Afterwards, a preparative isoelectric focusing (IEF) was performed with a Rotofor preparative IEF cell (Biorad, Hercules, USA). The concentrated, desalted sample was mixed with 2 % ampholyte (pH 2 to 4, Serva, Heidelberg, Germany). Two percent Chaps and proline were added to the sample to reduce protein precipitation during the run. The solution was focused at 12 W constant power for four hours at 4 °C. Fractions were collected, and the pH and esterase activity were determined. Active fractions from the preparative IEF were pooled and washed with an ultrafiltration module (30 kDa cut off, PES, Sartorius, Göttingen, Germany), to remove additives. Final purification of the enzyme was achieved with a combination of affinity and hydrophobic interaction chromatography using a 15 mL self-casted polyamide column (MP Biomedicals, Eschwege, Germany). The active sample was diluted one time with buffer (50 mM acetate pH 6.5 with 4 M (NH₄)₂SO₄) and loaded on the column. UmChIE was eluted with 65 % of 50 mM acetate buffer pH 6.5.

### Enzyme assay

Chlorogenic acid esterase activity was determined at 37 °C in 50 mM Tris buffer at pH 7.5 using chlorogenic acid and methyl ferulate as standard substrates, unless otherwise stated. The release of the corresponding free acids was analyzed with HPLC-UV. If appropriate, the hydrolysis of other benzoic and cinnamic acid ester derivatives as well as feruloylated saccharides was measured with the same method. After incubation for one hour the reaction was terminated by doubling the reacting volume with acetonitrile. Samples were centrifuged (15 min, 15.000 x g) before injection to the HPLC (Chromolith Performance RP-18e reverse-phase column, 100 x 4.6 mm, Merck, Darmstadt, Germany). Substrates and products were separated using a step-wise gradient at a flow velocity of 1.5 mL min⁻¹: 10 µL sample loaded in 90 % buffer A (0.1 % formic acid), 10 % buffer B (acetonitrile); 10 - 36 % buffer B in 8 min; 36 - 50 % buffer B in 1.5 min; 55 - 96 % buffer B in 0.5 min; 96 - 99 % buffer B in 2.5 min; 99 - 15 % buffer B in 0.5 min; 15 - 10 % buffer B in 1 min; re-equilibrate with 10 % buffer B for 1 min; total run time 14 min. Substance elution was detected at 232 nm (benzoic and 3-hydroxybenzoic acid), 254 nm (4-hydroxybenzoic and vanillic acid), 275 nm (cinnamic and gallic acid), 306 nm (*p*-coumaric acid) and 323 nm (ferulic, caffeic, chlorogenic and sinapic acid) using a Shimadzu UV-Vis detector (SPD-10A VP, Shimadzu Deutschland GmbH, Duisburg, Germany). One unit of enzyme activity was defined as the amount of enzyme releasing one µmol of free acid per minute under the specified conditions. All measurements were performed at least twice.

### Biochemical characterization of UmChIE

### i. Determination of temperature and pH optimum

The optimal pH was determined by measuring the esterase activity at 37 °C with pH in the range of 2 - 10 using the following buffers: 50 mM solutions of glycine (pH 2 - 3.5; pH 9 - 10), acetate (pH 3.5 - 5.5), Bis-Tris (pH 5.5 - 7), and Tris (pH 7 - 9). For the determination of the temperature optimum the activity assay was performed for 60 min at various temperatures (20 °C - 90 °C).

### ii. Temperature and pH stability

For determination of temperature stability the enzyme solution was incubated for 60 min at different temperatures (20 °C - 60 °C). Subsequently, the substrate and 50 mM Tris buffer pH 7.5 was added and the activity assay was performed for 60 min at 37 °C. The pH stability was determined using the above described buffer sytems (30 mM, pH 2 - 10) with incubation for 60 min at 37 °C. Residual enzyme activity was analyzed after further incubation (60 min, 37°C) in presence of substrate and 125 mM Tris buffer pH 7.5.

### iii. Effect of solvents, metal ions and inhibitors on enzyme activity

Enzyme tolerance to different solvents was investigated in the presence of 50 % (v/v) acetone, acetonitrile, diethyl ether, DMF, DMSO, ethanol, isoamyl alcohol, isobutyl alcohol, isopropanol, methanol, *n*-hexane, *n*-heptane, *tert*-butyl methyl ether or toluene, respectively, in 50 mM Tris buffer pH 7.5 containing 0.7 mM substrate. In an additional experiment, 0.84 mM substrate was used. After incubation (60 min, 37 °C, 900 rpm) the mixture was applied for HPLC analysis as described above. The effect of inhibitors on enzyme activity was tested with following: cetyltrimethylammonium bromide (CTAB), EDTA, iodacetamide, phenylmethylsulfonylfluoride (PMSF), SDS and various metal ions, such as Cs⁺, K⁺, Li⁺, Sr⁺, Ca²⁺, Co²⁺, Cu²⁺, Fe²⁺, Hg²⁺, Mg²⁺, Mn²⁺, Ni²⁺, Zn²⁺, Al³⁺, Fe³⁺ and Cr³⁺. Apart from PMSF (1 mM), all other potential inhibitors were added at concentrations of 5 mM. The effect of high salt concentrations (0.1 - 3 M NaCl) on enzyme activity was investigated, too.

### iv. Substrate specificity and kinetic parameters

Substrate specificity of the UmChIE was determined towards different cinnamate and benzoate esters as well as natural substrates, such as F-A, F-AX and F-AXG, with activity assays as described above. Three mM stock solutions of all substrates were prepared in water.

Kinetic parameters were determined from the initial hydrolysis rate of 0.005 - 1 mM chlorogenic acid, methyl ferulate and methyl *p*-coumarate (60 min, 37 °C, pH 7.5). *Kₘ* and vₘₐₓ values were calculated with non-linear regression using SigmaPlot 10.0 (Systat Software Inc., Chicago, USA). Blanks were carried out for all assays using water instead of enzyme. For calculation of *k*_{cat} the molecular mass was determined using denaturing SDS-PAGE.

### v. Enzymatic hydrolysis of natural substrates

Destarched wheat bran (DSWB) was prepared as described by Johnson et al. (27). Total content of ferulic, p-coumaric and caffeic acid in DSWB, sugar beet pectin (SBP) and coffee pulp was determined after alkaline hydrolysis with 2 M NaOH (4 h, 50 °C, 220 rpm). Phenolic acid content of each sample was analyzed with HPLC after acidification with acetic acid. The level of chlorogenic acid in coffee was determined after incubation with 80 % methanol. Samples (20 mg) of DSWB and coffee pulp as well as 0.5 % SBP were incubated (37°C, 20 h, 650 rpm) with purified UmChIE (0.02 U) in a final volume of 400 µL containing 125 mM Tris buffer pH 7.5. To investigate synergism with other carbohydrases, the release of ferulic acid was also analyzed after parallel incubation of UmChIE with either *Trichoderma viride* xylanase (DSWB, 0.025 U, Fluka, Buchs, Switzerland), tomato pectin esterase (SBP, Sigma, Taufkirchen, Germany) or pectinase preparations (SBP, SternEnzym, Ahrensburg, Germany). The amount of released phenolic acids from the natural substrates was quantified using HPLC.

### Denaturing SDS-PAGE

The enzyme purity and the molecular mass was determined using SDS-PAGE analysis (12 % resolving gel, 4 % stacking gel) according to Laemmli (28). Samples were prepared by mixing 1:1 with sample buffer (0.1 M Tris-HCI pH 6.8, 0.2 M DTT, 4 % SDS, 20 % glycerol, 0.2 % bromophenol blue) and boiling for 10 min. After electrophoresis at 15 mA per gel, gels were stained with InstantBlue (Expedeon, Cambridgeshire, Great Britain).

### Peptide mass fingerprint

Amino acid sequences of tryptic peptides of the UmChIE were deduced from ESI-MS/MS using a maXis QTOF mass spectrometer (Bruker, Bremen, Germany). The procedure is described in detail elsewhere (25). NCBI BLAST (program blastp) searches were performed with the obtained amino acid sequences.

### Amplification of chlorogenic acid esterase sequence

Isolation of total RNA from mycelium of *U. maydis,* grown for thirteen days in 10 % (v/v) SNL supplemented with 1 % (w/v) wheat bran, as well as cDNA synthesis were performed as described by Linke et al. (25). Amplification of the complete *CHLE* sequence was achieved by using the untranslated region (UTR) primers Uma_fw_UTR (TCCGTTCTTAGAAGCCAAACA (SEQ ID NO: 5)) and Uma_rev_UTR (GAAAGCCAAGCAACAAGGTT (SEQ ID NO: 6)), which were deduced based upon available Um00182.1 gene sequence (GenBank accession no. XP_756329.1). PCRs were performed by mixing 10 µL 5x Phusion High-Fidelity DNA Polymerase buffer, 0.4 µL dNTP mix (10 mM each), 25 pmol forward primer, 25 pmol reverse primer, 0.02 U Phusion High-Fidelity DNA Polymerase (Fermentas, St. Leon-Roth, Germany), 100 ng ss cDNA, and ddH₂O to 50 µL. PCR was performed in a MasterCycler gradient (Eppendorf, Hamburg, Germany) by incubating the reaction mixture at 98 °C for 2 min, then for 35 cycles at 98 °C for 30 s, 60 °C for 30 s and 72 °C for 60 s. Final elongation was performed at 72 °C for 10 min. All further steps including analysis of PCR products, ligation, transformation in *E. coli* and colony PCR were performed as described previously (29).

### Heterologous expression in Pichia pastoris

The *CHLE* gene with and without the native 21 amino acid signal peptide sequence was amplified using the primers UmChIE_Sfw (TTT TTC TCG AGA AAA GAG AGG CTA GGC TGC CTA ATC TG (SEQ ID NO: 7)), UmChIE_Ofw (TTT TTC TCG AGA AAA GAG AGG CTG AAG CTC TTC CAC AAG TCT C (SEQ ID NO: 8)) and UmChIE_rv (TTT TTG CGG CCG CTT AAT GAT GAT GAT GAT GAT GGA AGC CAA ACA C (SEQ ID NO: 9)) (underlined bases are the *Xh*oI and *Not*I restriction sites, respectively) and inserted into the *Xho*I*-Not*I site of the *P. pastoris* expression vector pPIC9 (Invitrogen, Karlsruhe, Germany). *P. pastoris* GS115 was transformed with the *Pme*I linearized expression construct pPIC9-*HIS-CHLE* to achieve *CHLE* gene insertion at the *AOX1* locus by electroporation (MicroPulser Electroporator, Bio-Rad, München, Germany) (30). After selection of *P*. *pastoris* transformants with the ability to grow on histidine-deficient agar plates, 48 transformants of each expression construct were screened for chlorogenic acid esterase activity. The transformants were cultivated in 96-well-plates as described by Sygmund et al. (31). Culture supernatants were analyzed for enzyme activity after 24, 48, 72, 96 and 120 h with the assay described above. To maintain stable expression conditions 0.5 % (v/v) methanol was added daily.

### Results and discussion

### Purification and general properties of UmChIE

When *U. maydis* was cultivated in SNL medium, esterase activity was detected, but the cultures turned deep black impeding chromatographic purification. The composition of SNL medium seemed to promote the synthesis of melanins. Melanin production has been described for *U*. *maydis* (32, 33). Production was avoided by reducing SNL to 10 % (v/v) and adding 1 % (w/v) wheat bran. These conditions induced esterase production with even higher activities than in the black SNL cultures. After optimization of the culture medium, the purification of the chlorogenic acid esterase UmChIE from culture supernatant of *U. maydis* was accomplished by three complementary separation steps including anion exchange chromatography on Q Sepharose FF, preparative IEF and finally a combination of affinity and hydrophobic interaction chromatography on polyamide.

The molecular mass of the chlorogenic acid esterase as determined by denaturing SDS-PAGE was 71 kDa (Fig.1, lane 2). The apparent mass of the native enzyme estimated by size exclusion chromatography suggested that the UmChIE was a monomer. These results demonstrated that UmChIE differed from the other enzymes described in literature. While Asther et al. (13) and Adachi et al. (15) identified the native chlorogenic acid hydrolases as homodimers of about 2 x 80 kDa, Benoit et al. (14) suggested the recombinant *A. niger* enzyme to exist as tetrameric form. Schöbel and Pollmann (12) characterized the second known chlorogenic acid esterase from A. *niger* also as a tetramer (4 x 60 kDa), whereas Okamura and Watanabe (16) described the hydroxycinnamic acid ester hydrolase of *A. japonicus* as a monomer of 145 kDa. The preparative IEF, used as second purification step, yielded a pi of about 3.0 for the UmChIE.

### Characterization of the purified chlorgenic acid esterase

To date, only few chlorogenic acid hydrolases (EC 3.1.1.42) are well characterized (11-15), while numerous feruloyl esterases (FAE; EC 3.1.1.73), a subclass of the carboxylic ester hydrolases, were purified and characterized from a wide range of bacteria and fungi (7-10). Some feruloyl esterases showed the ability to hydrolyze chlorogenic acid, such as *A. niger* FAEB (34) and a type B feruloyl esterase from *Neurospora crassa* (35). As UmChIE accepted chlorgenic acid as the most preferred substrate, but also hydrolyzed feruloyl esterase substrates efficiently, the enzyme was compared both with chlorogenic acid hydrolases and feruloyl esterases in terms of their properties.

As shown in Fig. 2, the influence of pH on esterase activity and stability was investigated in the pH range of 2 - 10. Maximum activity for hydrolysis of methyl ferulate was observed at pH 7.5, and more than 50 % residual activity was detected in the pH range of 5.5 - 9.5 (Fig. 2A). After one hour incubation at 37 °C the enzyme was more stable in neutral to alkaline than in acidic conditions (Fig. 2B). UmChIE retained more than 80 % of its maximum activity between pH 4.5 - 10. The determined pH optima of the two published chlorogenic acid hydrolases from *A. niger* (11, 13) and of type B FAEs listed by Koseki et al. (36) were between pH 6 and 7. Especially the pH stability of UmChIE was different from the characterized chlorogenic acid hydrolases from *A. niger* which were more stable in acidic than in alkaline conditions. The chlorogenic acid esterase isolated by Schöbel and Pollmann (12) was stable in a pH range of 3 - 8.5 after 10 minutes of incubation at an undefined temperature. Thus, UmChIE has a significant pH stability of the in alkaline conditions, which makes the enzyme an attractive candidate for biotechnological applications, for instance, the treatment of pulp in the paper industry (37).

Optimal reaction temperature of the UmChIE was recorded at 37 °C (Fig. 2C). Enzyme activity started to decrease rapidly at 45 °C to be nearly inactive at 60 °C. After pre-incubation at pH 7.5 and 37 °C for one hour, the enzyme lost 28 % of its activity. The thermostability of UmChIE decreased rapidly above 40 °C. The determined temperature optimum was lower than those reported for chlorogenic acid esterases and most of the feruloyl esterases (36). Furthermore the purified enzyme was less thermostable than those previously described (12-14).

The applicability of the UmChIE for ester synthesis was examined in 1:1 (v/v) buffer emulsions with different organic solvents (see Table 1). Relative enzyme activities were calculated based on a sample incubated in buffer without organic solvent (37 °C; one hour, pH 7.5, 900 rpm). Some of the tested solvents are water-immiscible, but a large interfacial area between organic and polar phase was ensured by vigorous shaking of the reaction mixture. UmChIE showed good stability in *n-*hexane and *n*-heptane with relative activities of 87 % and 84 %. Moderate to slight activity was detected in isoamyl alcohol (64 %) and isobutyl alcohol (23 %). In all other systems the residual enzyme activity was less than 7 %. The good stability of the UmChIE in *n*-hexane and *n*-heptane indicated the potential of the enzyme for reverse-synthetic activity. Indeed, Topakas et al. showed the synthesis of phenolic acid esters with a feruloyl esterase of *Fusarium oxysporum* (38) and *Sporotrichum thermophile* (39) using a surfactantless microemulsion formed in a ternary water-organic mixture consisting of *n*-hexane, 1-propanol/1-butanol and water as a reaction system.

**Table 1 Effect of organic solvents on UmChIE activity.**

| **Organic solvent** | **Relative esterase activity [%]** | |
|---|---|---|
| *n*-hexane | 87 | (2.12) |
| *n*-heptane | 84 | (0.89) |
| isoamyl alcohol | 64 | (2.43) |
| isobutyl alcohol | 23 | (6.53) |
| Toluene | 7 | (1.67) |
| Isopropanol | 6 | (0.37) |
| Methanol | 5 | (0.23) |
| Acetone | 3 | (2.59) |
| acetonitrile | 3 | (3.18) |
| DMF | 1 | (0.76) |
| diethyl ether | ND | |
| DMSO | ND | |
| Ethanol | ND | |
| *tert*-butyl methyl ether | ND | |

| | | |
|---|---|---|
| Residual activities of UmChIE for the hydrolysis of 0.7 mM methyl ferulate (1 h, 37 °C, 900 rpm) in a binary system composed of Tris buffer pH 7.5 and organic solvents in the ratio 1:1 (v/v). Esterase activity determined without organic solvent was set to 100 %. Numbers in parentheses are the estimates of the standard error. ND: activity not detectable. | | |

### Inhibition studies

Various metal ions and enzyme inhibitors were examined for their effect on chlorogenic acid esterase activity (Table 2). Residual enzyme activity was measured using the standard activity assay in presence of 5 mM metal ions/inhibitors (except PMSF, 1 mM). Addition of Hg²⁺ resulted in complete loss of enzyme activity. Hg²⁺ reacts with thiol groups and can also reduce disulphide bonds (40). Among the tested metal ions, a significant inhibition of UmChIE activity, to residual activities of 50 - 76 %, was detected in presence of Al³⁺ , Co²⁺, Cr³⁺, Fe³⁺ and Zn²⁺, while Mn²⁺ decreased the enzyme activity slightly to 90 %. Accordingly, the enzyme of the invention can be used in the presence of all tested metal ions except Hg²⁺ with significant residual activity. The inhibition of UmChIE activity by Zn²⁺, Hg²⁺ and Al³⁺ was consistent with the properties of a feruloyl esterase from *Russula virescens* (41). This feruloyl esterase was also inhibited by Fe²⁺, whereas UmChIE was not, but it was only inhibited by Fe³⁺. The negative effect of Fe³⁺ on esterase activity was also shown by Donaghy and McKay (42) and Yao et al. (43). Some esterases were also inhibited by Cu²⁺ (42, 43) and Cd²⁺ (41).

**Table 2 Effect of metal ions, inhibitors and NaCl on UmChIE activity.**

| **Concentration [mM]** | **Substance** | **Relative esterase activity [%]** | |
|---|---|---|---|
| 5 | KCl | 108 | (0.57) |
| 5 | NiSO₄ | 108 | (0.87) |
| 5 | MgCl₂ | 106 | (0.53) |
| 5 | LiCI | 106 | (1.39) |
| 5 | CsCl | 105 | (1.21) |
| 5 | SrNO₃ | 104 | (0,18) |
| 5 | CaCl₂ | 103 | (2.76) |
| 5 | FeSO₄ | 102 | (0.50) |
| 5 | Cu(NO₃)₂ | 96 | (0.53) |
| 5 | MnSO₄ | 90 | (0.09) |
| 5 | ZnSO₄ | 76 | (1.26) |
| 5 | Cr(NO₃)₃ | 69 | (3.54) |
| 5 | CoCl₂ | 67 | (0.11) |
| 5 | FeCl₃ | 66 | (0.65) |
| 5 | Al₂(SO₄)₃ | 53 | (0.12) |
| 5 | HgAc₂ | ND | |
| 5 | EDTA | 105 | (1.14) |
| 5 | CTAB | 104 | (0.70) |
| 5 | Iodacetamide | 102 | (0.81) |
| 5 | SDS | 59 | (0.83) |
| 1 | PMSF | 36 | (2.01) |
| 100 | NaCl | 105 | (0.02) |
| 500 | NaCl | 121 | (0.01) |
| 1000 | NaCl | 125 | (0.02) |
| 2000 | NaCl | 142 | (0.06) |
| 3000 | NaCl | 150 | (0.04) |

| | | | |
|---|---|---|---|
| Remaining enzyme activity was measured after 1 h incubation with 1mM, or, in a further experiment, 1.95 mM methyl ferulate and different metal ions as well as inhibitors in concentrations listed above (at 37 °C, 50 mM Tris buffer pH 7.5). Numbers in parentheses are the estimates of the standard error. A sample without additive was used for the calculation of relative enzyme activities. ND: activity not detectable. | | | |

The enzyme activity was inhibited 41 % by SDS and 64 % by PMSF, while incubation with CTAB, EDTA and iodacetamide showed no effect (Table 2). The almost complete loss of activity in the presence of PMSF, a known esterase inhibitor, suggested that the catalytic triad of UmChIE contained a serine residue (44). In contrast, enzyme activity was not influenced by CTAB, which reacts with tryptophane and SH groups of cysteine, indicating that these amino acid residues were not involved in enzyme catalysis (45).

Furthermore, the tolerance of UmChIE towards various NaCl concentrations (0.1 - 3 M) was investigated. It was shown that the enzyme activity increased with the salt concentration, up to 150 % in presence of 3 M NaCl (Table 2). A stimulatory effect on esterase activity by NaCl was previously reported by Nieter et al. (29) and Kumar et al. (46).

### Hydrolytic specificity and kinetic properties of UmChIE

A comprehensive set of substrates was examined for hydrolysis by UmChIE. The substrates included cinnamic- and benzoic acid esters as well as natural substrates, such as F-A, F-AX, F-AXG and chlorogenic acid (Table 3). Chlorgenic acid was found to be hydrolyzed most effectively. The determined specific activity of 2.8 U mg⁻¹ for chlorogenic acid was set to 100 % and used for the calculation of relative activities for all other substrates. UmChIE hydrolyzed methyl p-coumarate (M*p*CA, 52 %), methyl ferulate (MFA, 46 %) and methyl caffeate (MCA, 18 %), but no methyl sinapate (MSA). These four typical synthetic feruloyl ester substrates were commonly used to classify FAEs. Crepin et al. (10) divided FAEs into four types (A - D) based on their substrate specificity, sequence homology and biochemical analysis. The lack of hydrolysis of MSA would result in type B FAE according to Crepin et al. (10). In the meantime the classification system was extended by phylogenetic analysis of fungal FAEs by Benoit et al. (47), while a descriptor-based computational analysis with pharmacophore modeling (48) offered an alternative attempt for classification.

**Table 3 Substrate specificity of purified UmChIE.**

| **Substance** | **Relative esterase activity [%]** | | **Specific esterase activity [U mg⁻¹]** |
|---|---|---|---|
| chlorogenic acid | 100 | | 2.76 |
| F-A | 75 | (0.16) | 2.06 |
| F-AX | 59 | (0.36) | 1.63 |
| methyl *p*-coumarate | 52 | (0.06) | 1.43 |
| methyl ferulate | 46 | (0.16) | 1.27 |
| F-AXG | 33 | (0.09) | 0.92 |
| ethyl ferulate | 28 | (0.45) | 0.78 |
| methyl caffeate | 18 | (0.03) | 0.49 |
| methyl benzoate | ND | | ND |
| ethyl benzoate | ND | | ND |
| methyl 3-hydroxy benzoate | ND | | ND |
| methyl 4-hydroxy benzoate | ND | | ND |
| ethyl 4-hydroxy benzoate | ND | | ND |
| propyl 4-hydroxy benzoate | ND | | ND |
| butyl 4-hydroxy benzoate | ND | | ND |
| methyl cinnamate | ND | | ND |
| ethyl cinnamate | ND | | ND |
| methyl gallate | ND | | ND |
| methyl sinapate | ND | | ND |
| methyl vanillate | ND | | ND |
| ethyl vanillate | ND | | ND |

| | | | |
|---|---|---|---|
| Purified UmChIE was incubated with 1 mM, or, in a further experiment, 1.95 mM of different substrates for 1 h (at 37 °C, 50 mM Tris buffer pH 7.5). Relative enzyme activities were calculated based on the most preferred substance of the UmChIE: chlorogenic acid which was set to 100 %. Numbers in parentheses are the estimates of the standard error. Abbreviations: F-A: 5-O-*trans*-feruloyl-L-arabinofuranose, F-AX: β-o-xylopyranosyl-(1→2)-5-O-*trans*-feruloyl-L-arabinofuranose, F-AXG: α-L-galactopyranosyl-(1→2)-β-D-xylopyranosyl-(1→2)-5-O-*trans*-feruloyl-L-arabinofuranose and ND: activity not detectable. | | | |

UmChIE was also able to release ferulic acid from ethyl ferulate, but about 50 % less than from MFA. Consequently, elongation of the ester-linked alkyl chain resulted in decreasing enzyme activity (49). While the presence of one methoxy group at the *meta* position of the benzoic ring lowered enzyme activity marginally (M*p*CA 52 % vs. MFA 46 %), a second methoxy group caused total loss of activity (MSA). Moreover UmChIE hydrolyzed no substrate without hydroxyl or methoxy group on the benzoic ring (methyl/ethyl cinnamate). The fact, that UmChIE showed no hydrolytic activity against any benzoate derivative indicates the necessity of a certain distance between the aromatic ring and the ester bond. This requirement for the catalytic activity of feruloyl esterases was also reported by Topakas et al. (50) and Kroon et al. (51).

UmChIE hydrolyzed also small natural substrates such as feruloylated mono-, di- and trisaccharides (F-A, F-AX, F-AXG). F-A was hydrolyzed most efficiently (2.0 U mg⁻¹) followed by FAX (1.6 U mg⁻¹) and F-AXG (0.9 U mg⁻¹). Likewise, *A. niger* FAEA (34) preferred the natural FAE substrates in the same order, whereas PeFaeA of *Pleurotus eryngii* (29) and Est1 of *Pleurotus sapidus* (25) preferred the natural substrate F-A less than F-AX. In contrast, the FAE of *Penicillium expansum* had higher specific activities for methyl ferulate (14.4 U mg⁻¹) than for F-AX (5.3 U mg⁻¹) (42).

Kinetic properties were determined from initial hydrolysis rates for the most preferred substrates chlorogenic acid, MFA and M*p*CA. The purified UmChIE possessed the following kinetic constants *(K*ₘ; *k*_{cat}; *k*_{cat}/*K*ₘ): MFA (101.8 µM; 0.38 s⁻¹; 3.75 mM⁻¹s⁻¹), M*p*CA (64.1 µM; 0.49 s⁻¹; 7.63 mM⁻¹s⁻¹) and chlorogenic acid (19.6 µM; 0.51 s⁻¹; 25.83 mM⁻¹s⁻¹). Determination of *K*ₘ values demonstrated that the enzyme had a three- and fivefold higher affinity for chlorogenic acid than for M*p*CA and MFA. Furthermore the *k*_{cat}/*K*ₘ values revealed that the UmChIE hydrolyzed chlorogenic acid three- and sevenfold more efficiently than M*p*CA and MFA. Consequently, the catalytic efficiency (*k*_{cat}/*K*ₘ), a good indicator for enzyme efficiency and specificity, was better for chlorogenic acid than for the two synthetic substrates.

While the UmChIE could still be classified as a feruloyl esterase based on its substrate profile, the kinetic constants clearly showed the affiliation to the family of chlorogenic acid esterases. Here, the previously reported *K*ₘ values for chlorogenic acid as substrate were 6.5 - 10 µM (13, 14) and 0.7 mM (11) for the second published chlorogenic acid hydrolases from *A. niger.* There is few other literature available dealing with an enzyme activity on chlorogenic acid. In contrast, *K*ₘ values were determined for three type B feruloyl esterases for the substrate chlorogenic acid as following: 245 µM for *A. niger* FAEB (34), 180 µM for type B feruloyl esterase from *N. crassa* (35) and 630 µM for AoFaeB from *A. oryzae* (19). The feruloyl esterases mentioned above exhibited not only a more than tenfold lower affinity for chlorogenic acid than UmChIE but possessed also a threefold lower *K*m for M*p*CA (20 µM) than UmChIE (64 µM). They also showed higher specificity for MCA than for MFA.

Finally, the UmChIE distinguished clearly from the two other published enzymes by its broad substrate profile. Previously identified chlorogenic acid hydrolases were solely active on chlorogenic acid. Asther et al. (13) tested activity of the native chlorogenic acid hydrolase for methyl ester of hydroxycinnamates, while Benoit et al. (14) investigated F-A and F-AX as substrates for the recombinant enzyme, but none of these substrates was hydrolyzed.

### Release of phenolic acids from natural substrates

For the determination of UmChIE specificity, SBP and DSWB were used as substrates due to the different linkage of ferulic acid to arabinose residues (3). The agro-industrial by-product coffee pulp was selected due to its high content of chlorogenic acid. Total amounts of caffeic acid, ferulic and *p*-coumaric acid were quantified after alkaline hydrolysis with HPLC for coffee pulp (1.65 mg g⁻¹, 0.53 mg g⁻¹, 0.07 mg g⁻¹), DSWB (- ; 4.45 mg g⁻¹; 0.18 mg g⁻¹) and SBP (- ; 6.78 mg g⁻¹; - ;). Further, coffee pulp contained 2.66 mg g⁻¹ of chlorogenic acid in total.

After incubation of coffee pulp with UmChIE (0.02 U; 20 h, 37 °C and 650 min⁻¹ on a thermoshaker) 68 % of the initial chlorogenic acid amount was hydrolyzed. In contrast, the native as well as the recombinant chlorogenic acid hydrolase from *A. niger* released after overnight incubation 100 % of the initial chlorogenic acid content of coffee pulp, but the enzymes were used with two to sixfold higher concentrations than UmChIE (13, 14). UmChIE also liberated significant amounts of phenolic acids from coffee pulp. Thus, 18 %, 15 % and 26 % of the alkali-extractable caffeic, ferulic and *p*-coumaric acid level were released, respectively. The higher content of alkali-extractable caffeic acid than the total amount of chlorogenic acid can be explained by the existence of caffeic acid conjugates other than chlorogenic acid, such as dicaffeoyl quinic acid or esters of feruloyl-caffeoyl quinic acid (4).

UmChIE (0.02 U) liberated small amounts of ferulic acid from DSWB. The simultaneous incubation with *T. viride* xylanase (0.025 U) increased the amount of released ferulic acid from DSWB more than twelvefold, whereas the incubation with *T. viride* xylanase liberated no ferulic acid at all. In contrast, the UmChIE was less active on SBP, even in presence of different pectinase preparations 3-fold less of the alkali-extractable ferulic acid was liberated from SBP than from DSWB. The observed synergistic interaction of UmChIE with both xylanase and pectinases can be explained by the action of carbohydrases producing shorter-chain feruloylated oligosaccharides which are more accessible substrates for the enzyme. A synergistic action of xylanases and feruloyl esterases was reported previously (26, 52-54).

### Amplification and genetic characterization of the UmChIE sequence

Seven tryptic peptides of the purified chlorogenic acid esterase were obtained by ESI-MS/MS: ADATASAPTVK (P1, SEQ ID NO: 10), FAKPQPLGPASSHK (P2, SEQ ID NO: 11), HPTVEQSFKR (P3, SEQ ID NO: 12), LANGVGCTGGSLLR (P4, SEQ ID NO: 13), VWSYEFQQNDK (P5, SEQ ID NO: 14), EAMDALTNR (P6, SEQ ID NO: 15) and AKPPVGSLR (P7, SEQ ID NO: 16). Homology search with Blastp (NCBI BLAST) identified them (P1 - P7) as part of the hypothetical protein Um00182.1 from *U. maydis* 521 (GenBank accession no. **XP_756329.1**). After RNA isolation from *U. maydis* cultures grown for thirteen days in 10 % (v/v) SNL media supplemented with 1 % (w/v) wheat bran, cDNA was synthesized, and the sequence of the UmChIE (GenBank accession no. **HG970190)** was successfully amplified using UTR primers. The complete coding sequence of UmChIE has a length of 1,758 bp, corresponding to a protein of 585 amino acids (aa). The full nucleic acid sequence of UmChIE (SEQ ID NO: 18) possessed three differences on nucleotide level which led to one change on the amino acid level (SEQ ID NO: 17) at position 492 (proline to leucine) compared to the sequence of Um00182.1 (SEQ ID NO: 19).

### Heterologous expression of UmChIE in P. pastoris

To confirm that the annotated sequence (GenBank accession no. **HG970190)** encodes the characterized chlorogenic acid esterase UmChIE from *U. maydis,* the putative sequence was cloned in frame with the *Saccharomyces cerevisiae α*-factor secretion signal sequence under the transcriptional control of the alcohol oxidase *(AOX1)* promoter and integrated into *P. pastoris* GS115. Expression constructs were generated with and without the native signal sequence. A hexahistidine tag was incorporated at the C-terminus. Only the transformants carrying the expression construct without native signal sequence showed chlorogenic acid esterase activity. After induction with 0.5 % (v/v) methanol, these *P*. *pastoris* transformants expressed the recombinant gene successfully and secreted the active enzyme into the culture media. After methanol induction for 96 h the chlorogenic acid esterase activity of the culture medium (0.8 U L⁻¹) was eightfold higher than in control cultures of *P*. *pastoris* GS115.

### Example 2: Chlorogenic acid esterase from Rhizoctonia solani (RsChIE)

A sequence encoding a chlorogenic acid esterase from *Rhizoctonia solani,* RsChIE, was amplified from Rhizoctonia solani and expressed in *Pichia pastoris.* The coding sequence was cloned into the expression vector, e.g., pPIC9, without the native signal sequence (SEQ ID NO:20). A tag, e.g., a C-terminal His-tag, was added to the coding sequence as one option for purification. The nucleotide and amino acid sequence of RsChIE are as follows:

The native signal sequence is shown in italics, bold and underlined. The sequences without the signal sequences are designated SEQ ID NO: 22 (amino acid) and SEQ ID NO:20 (nucleic acid).

After purification of the recombinant RsChIE from the culture supernatant of Pichia pastoris with Ni-NTA, the enzyme was biochemically characterized. The experiments were performed like for UmChIE. pH and temperature optimums were analyzed. Maximal esterase activity was achieved at pH 6.0 (Fig. 4a) and at 30°C (Fig. 4b).

**Table 4: Substrate specificity of purified RsChIE.**

| **substance** | **Relative esterase activity [%] (SE)** | | **Specific activity [U/mg]** |
|---|---|---|---|
| methyl coumarate | 100 | (0.11) | 6.19 |
| chlorogenic acid | 83 | (0.12) | 5.15 |
| F-AX | 80 | (0.05) | 4.97 |
| methyl caffeate | 74 | (0.13) | 4.61 |
| F-A | 72 | (0.13) | 4.48 |
| F-AXG | 59 | (0.08) | 3.66 |
| ethyl ferulate | 58 | (0.07) | 3.60 |
| methyl ferulate | 51 | (0.02) | 3.19 |
| methyl cinnamate | 34 | (0.09) | 2.10 |
| ethyl cinnamate | 15 | (0.05) | 0.91 |
| methyl sinapate | - | | |

| | | | |
|---|---|---|---|
| Purified RsChIE was incubated with 1 mM, or in an alternative experiment, 1.95 mM of different substrates for 1 h (at 30 °C, 50 mM Bis-Tris buffer pH 6.0). Relative enzyme activities were calculated based on the most preferred substance of the RsChIE: methyl p-coumarate which was set to 100 %. Numbers in parentheses are the estimates of the standard error. Abbreviations: F-A: 5-O-*trans*-feruloyl-L-arabinofuranose, F-AX: β-D-xylopyranosyl-(1→2)-5-O-*trans*-feruloyl-L-arabinofuranose, F-AXG: α-L-galactopyranosyl-(1→2)-β-D-xylopyranosyl-(1→2)-5-O-*trans*-feruloyl-L-arabinofuranose and ND: activity not detectable. | | | |

The highest activity of RsChIE was detected with regard to methyl coumarate, followed by the natural substrates chlorogenic acid and F-AX. Furthermore, the lack of hydrolysis of methyl sinapate shows that RsChIE, like UmChIE, also has type B feruloyl esterase activity. Furthermore, also like UmChIE, RsChIE does not show activity on benzoic acid derivatives.

In the following, the kinetic constants were determined to decide if the enzyme has mainly chlorogenic acid esterase or feruloyl esterase activity.

**Table 5: kinetic analysis of RsChIE**

| | Km [µM] | vₘₐₓ [U/mg] | k_{cat} [s⁻¹] | kcat/Km [mM * s⁻¹] |
|---|---|---|---|---|
| methyl caffeate | 28,5 | 16 | 20,00 | 70,18 |
| methyl coumarate | 35,8 | 26,5 | 33,13 | 925,28 |
| chlorogenic acid | 18,3 | 23,4 | 29,25 | 1598,36 |
| F-AX | 38,4 | 23,3 | 29,13 | 758,46 |

Based on the *K*ₘ values and the catalytic efficiency, (*k*_{cat}/*K*ₘ) it could be shown that the new esterase from *Rhizoctonia solani* mainly is a chlorogenic acid esterase (for comparison (UmChIE *K*ₘ = 19,6 µM).

### Example 3

ScFae1 and ScFae3 were amplified form *S. commune.* The respective nucleic acid sequences are shown as SEQ ID NO: 24 and 28 (without signal sequence) and SEQ ID NO: 25 and 29 (with endogenous signal sequence).The respective amino acid sequences are shown as SEQ ID NO: 26 and 30 (without signal sequence) and SEQ ID NO: 27 and 31 (with endogenous signal sequence).

The enzymes were characterized with regard to activity at different temperatures: Enzymes were incubated with methyl ferulate for 30 min at 20 - 90 °C, 50 mM Tris buffer pH 7.5 (n=3). The reaction was terminated with 50 % acetonitrile and the release of ferulic acid from the substrate was analyzed using HPLC-UV. A temperature optimum of about 39-45°C was determined.

The enzymes were characterized with regard to activity at different pH: Enzymes were incubated with methyl ferulate for 30 min at 37 °C, pH 2 - 10 (n=3). The following buffer systems were used: 50 mM glycine (2.0 - 3.5; 9.0 - 10.0), acetate (3.5 - 5.5), Bis-Tris (5.5 - 7.0) or Tris buffer (7.0 - 9.0). The reaction was terminated with 50 % acetonitrile and the release of ferulic acid from the substrate was analyzed using HPLC-UV. A high pH stability at pH 3-9.5 and a broad optimum at about pH 5- 8.5 was determined.

On SDS-PAGE, for both ScFae, there was one main protein band in the samples with a molecular mass of about 100 kDA. The protein band which was excised after partial purification of the native enzymes had only a molecular mass of 65 kDA. To detemine if the molecular mass difference resulted from hypergylcosylation of the recombinant enzymes or if the single protein band on the gel a host protein, the purified samples were treated with Endo H to remove N-glycosylation which are typical posttranslational modifications performed by *Pichia.* After treatment with endo H, on the SDS-PAGE gel, both enzymes showed a lower molecular mass. While the deglycosylated ScFae3 60993 had a molecular mass of about 85 kDA, the ScFae1 possessed a molecular mass of about 70 kDA. The marked protein bands were excised to verify production of both recombinant enzymes. Sequencing verified these protein bands as the respective enzymes.

**Table 6**

| | ScFae1 relative FAE activity [%] | ScoFae3 relative FAE activity [%] |
|---|---|---|
| Methyl gallate | 4,36 | 5,13 |
| Methyl cinnamate | 8,76 | 8,71 |
| Ethyl cinnamate | 4,31 | 3,60 |
| Methyl caffeate | 49,56 | 47,25 |
| Chlorogenic acid | 14,15 | 11,50 |
| Methyl ferulate | 72,16 | 64,58 |
| Ethyl ferulate | 69,88 | 62,31 |
| F-A | 86,16 | 83,99 |
| F-AX | 96,87 | 105,70 |
| F-AXG | 92,93 | 83,32 |
| Methyl sinapate | 3,12 | 2,89 |
| Methyl benzoate | 1,98 | 2,32 |
| Ethyl benzoate | 1,26 | 1,32 |
| 3-OH-methyl benzoate | 6,68 | 6,65 |
| 4-OH- methyl benzoate | 26,69 | +31,42 |
| 4-OH- ethyl benzoate | 23,02 | 21,27 |
| 4-OH-propyl benzoate | 20,58 | 23,05 |
| 4-OH- butyl benzoate | 22,15 | 20,52 |
| Methyl salicylate | 0,00 | 0,00 |
| Methyl vanillate | 11,14 | 10,40 |
| Ethyl vanillate | 10,30 | 10,18 |
| Methyl coumarate | 100,00 | 100,00 |

| | | |
|---|---|---|
| Purified enzymes were incubated with 1 mM of different substrates for 30 min at 37 °C in 50 mM Tris buffer pH 7.5. Relative enzyme activities were calculated based on the most preferred substance of the enzyme: methyl p-coumarate which was set to 100 %. Abbreviations: F-A: 5-O-*trans*-feruloyl-L-arabinofuranose, F-AX: β-D-xylopyranosyl-(1→2)-5-O-*trans*-feruloyl-L-arabinofuranose, F-AXG: α-L-galactopyranosyl-(1→2)-β-D-xylopyranosyl-(1→2)-5-O-*trans*-feruloyl-L-arabinofuranose. | | |

A comprehensive set of substrates was examined for hydrolytic activity of the purified recombinant ScFAEs. The substrates included different cinnamic- and benzoic acid esters as well as natural substrates, such as F-A, F-AX, F-AXG and chlorogenic acid. Both enzymes possessed a highly comparable substrate profile, although the sequences of both enzymes have only 90 % homology on amino acid level. Methyl coumarate was found to be hydrolyzed most effectively directly followed by the feruloylated saccharides. The determined activities for methyl coumarate were set to 100 % and used for the calculation of relative activities for all other substrates. This enzyme is the first analysed enzyme with feruloyl esterase and/or chlorogenic acid esterase activity hydrolyzing a synthetic substrate most effectively. The new ScFAEs possess a unique substrate profile. Furthermore, the hydrolysis of methyl *p*-coumarate, methyl ferulate, methyl sinapate and methyl caffeate verified these new enzymes as type C or D FAEs according the classification system of fungal FAEs. The difference between type C and D FAEs is the ability to release diferulic acid from natural substrates, which was not investigated. Additionally, the substrate profile demonstrated that the lengthening of the ester-linked alkyl chain didn't affect the hydrolytic activity significantly. So the determined relative esterase activities for the methyl, ethyl, propyl and butyl ester of the respective substrate differs only slightly. These are the first basidiomycete FAEs analysed which were slightly active on benzoate derivatives.

### Example 4

### Application of chlorogenic acid esterases and/or feruloyl esterases in bakery products

### A Use of UmChIE in baking processes

The purified enzyme UmChIE was exemplarily used for demonstration of the enzymes' impact in bakery products. More precisely, split rolls and pan breads were produced in a bakery trial.

For baking trials, the basic doughs were prepared as follows: 1000 g wheat flour (T 550), 1.5 % yeast, 1.8 % NaCl, 2 % sugar, 2 % Biskin (palm fat), 40 ppm ascorbic acid and 600 mL water. The effect of UmChIE (1 U) on bun volume was studied in combination with a xylanase (25 ppm) and amylase (15 ppm). Dough treated with amylase alone or xylanase and amylase served as reference. The dough was kneaded with a Diosna SP 12 with frequence converter at 25 Hz (2 min) and 50 Hz (6 min). After 10 min of first proofing, the dough was divided into 50 g pieces. After further proofing for 30 min at room temperature the dough pieces were placed in a fermentation chamber at 32 °C for 40/60 min. Finally the dough pieces were baked at 230 °C for 18 min.

The volume of the cool baking goods was monitored according to the repression principle. Results for split rolls (50 g dough) were compared and shown in following table:

**Table 7: Baking volume of split rolls (50 g), calculated in specific volume (ml/100g):**

| | amylase | amylase, xylanase | amylase, xylanase, 1U UmChIE |
|---|---|---|---|
| normalfermented | 664.7 | 732.7 | 718 |
| overfermented | 685.8 | 725.1 | 710.2 |

The presence of the enzyme UmChIE surprisingly results in a decrease of the specific volume during a baking trial. This phenomenon can be explained by dough softening. These results show that the chlorogenic acid esterase/feruloyl esterase UmChIE may be used for dough softening.

### B Rheological properties of dough treated with chlorogenic acid esterase and/or feruloyl esterase

With this experiment, the recombinantly produced chlorogenic acid esterases and/or feruloyl esterases from *Ustilago mayidis* UmChIE, *Rhizoctonia solani* RsChIE and *Schizophyllum commune* ScFae1 and ScFae3 were investigated for their impact on the dough softening. Dough softening was demonstrated via the Kieffer Dough Extensibility Rig test (Kieffer et al., 1998) using a SMS/Kieffer rig and an TA TX2 Textur analyzer (Stable Micro Systems Ltd., UK). The enzyme treated doughs were prepared according to the following protocol:
1. 42 g flour is adjusted to 60 % water absorption capacity
2. Add 25 ml tap water containing 3.4 % sodium chlorid
3. Add chlorogenic acid esterase preparation (1 U/ml)
4. Knead for 1 min. using a Y1-ETK laboratory fork kneader
5. Weight 50 g dough and round for 30 sec as well as sheet for 30 sec using a rounding- and sheeting station
6. Place dough in desiccator at 90 % humidity and rest for 3 min
7. Prepare Kieffer rig according to system manual. Discard strand 1-4 and 14-17
8. Place rig for three minutes in desiccators (90 % humidity) and start measurements
9. Monitor at least nine measurements in order to minimize measuring errors

The enzyme dosages were empirically chosen with 0.1, 1 and 10 Units. Data evaluation was performed using the averaged measures compared to reference measures. An untreated dough was used as a reference.

**Table 8: Kieffer-Rig test for UmChIE**

| | | | **UmChIE** | | |
|---|---|---|---|---|---|
| dosage | [U] | 0 | 0.1 | 1 | 10 |
| max. force | [g] | 51.0 | 51.5 | 44.6 | 41.4 |
| path | [mm] | -23.4 | -22.7 | -21.8 | -25 |
| rel. max force | [%] | | **0.8** | **-12.6** | **-19.0** |
| rel. path | [%] | | -2.8 | -6.8 | 6.9 |

**Table 9: Kieffer-Rig test for ScFae from selected P. pastoris transformants**

| | | | **ScFae1** | | | **ScFae3** | | |
|---|---|---|---|---|---|---|---|---|
| dosage | [U] | 0 | 0.1 | 1 | 10 | 0.1 | 1 | 10 |
| max. force | [g] | 51.0 | 52.1 | 52.5 | 45.4 | 59.2 | 48.1 | 46.5 |
| path | [mm] | -23.4 | -23.9 | -22.1 | -24.2 | -24.2 | -22.4 | -23.2 |
| rel. max force | [%] | | **2.1** | **2.9** | **-11.1** | **15.9** | **-5.8** | **-8.9** |
| rel. path | [%] | | -2.1 | 2.1 | -5.5 | -3.2 | -4.5 | -0.7 |

**Table 10: Kieffer-Rig for RsChIE from different samples**

| | | | **RsChIE (sample 1)** | | | **RsChIE (sample 2)** | | |
|---|---|---|---|---|---|---|---|---|
| dosage | [U] | 0 | 0.1 | 1 | 4 | 0.1 | 1 | 4 |
| max. force | [g] | 51.0 | - | 45.4 | 29.2 | - | 41.9 | 26.3 |
| path | [mm] | -23.4 | - | -23.3 | -25.7 | - | -24.6 | -23.5 |
| rel. max force | [%] | | - | **-11.0** | **-42.7** | **-** | **-17.8** | **-48.5** |
| rel. path | [%] | | - | -0.3 | 9.8 | - | -5.3 | -0.4 |

The relative maximal force is a measure of dough softening. It is described by the differences between the reference value and the measured value. The results show that all chlorogenic acid esterases and/or feruloyl esterases led to dough softening. The effect was dependent on the concentration of enzyme used. The use of enzymes having chlorogenic acid esterase activity and/or feruloyl esterase activity for dough softening is shown for the first time in the present invention.

### REFERENCES

1. Ishii T. 1997. Structure and function of feruloylated polysaccharides. Plant Sci. 127:111-127.
2. Cosgrove DJ. 2001. Wall structure and wall loosening. A look backwards and forwards. Plant physiology 125:131-134.
3. Ralet MC, Faulds CB, Williamson G, Thibault JF. 1994. Degradation of feruloylated oligosaccharides from sugar-beet pulp and wheat bran by ferulic acid esterases from Aspergillus niger. Carbohydrate research 263:257-269.
4. Clifford MN. 1999. Chlorogenic acids and other cinnamates - nature, occurrence, dietary burden, absorption and metabolism. J Sci Food Agric 79:362-372.
5. Kroon PA, Garcia-Conesa MT, Fillingham IJ, Hazlewood GP, Williamson G. 1999. Release of ferulic acid dehydrodimers from plant cell walls by feruloyl esterases. J Sci Food Agric 79:428-434.
6. Williamson G, Faulds CB, Kroon PA. 1998. Specificity of ferulic acid (feruloyl) esterases. Biochemical Society transactions 26:205-209.
7. Wong DW. 2006. Feruloyl esterase: a key enzyme in biomass degradation. Applied biochemistry and biotechnology 133:87-112.
8. Fazary AE, Ju YH. 2007. Feruloyl esterases as biotechnological tools: current and future perspectives. Acta biochimica et biophysica Sinica 39:811-828.
9. Topakas E, Vafiadi C, Christakopoulos P. 2007. Microbial production, characterization and applications of feruloyl esterases. Process Biochem. 42:497-509.
10. Crepin VF, Faulds CB, Connerton IF. 2004. Functional classification of the microbial feruloyl esterases. Applied Microbiology and Biotechnology 63:647-652.
11. Schöbel B, Pollmann W. 1980. Further characterization of a chlorogenic acid hydrolase from Aspergillus niger. Zeitschrift fur Naturforschung. Section C: Biosciences 35:699-701.
12. Schöbel B, Pollmann W. 1980. Isolation and characterization of a chlorogenic acid esterase from Aspergillus niger. Zeitschrift fur Naturforschung. Section C: Biosciences 35:209-212.
13. Asther M, Estrada Alvarado MI, Haon M, Navarro D, Asther M, Lesage-Meessen L, Record E. 2005. Purification and characterization of a chlorogenic acid hydrolase from Aspergillus niger catalysing the hydrolysis of chlorogenic acid. Journal of biotechnology 115:47-56.
14. Benoit I, Asther M, Bourne Y, Navarro D, Canaan S, Lesage-Meessen L, Herweijer M, Coutinho PM, Asther M, Record E. 2007. Gene overexpression and biochemical characterization of the biotechnologically relevant chlorogenic acid hydrolase from Aspergillus niger. Applied and environmental microbiology 73:5624-5632.
15. Adachi O, Ano Y, Akakabe Y, Shinagawa E, Matsushita K. 2008. Coffee pulp koji of Aspergillus sojae as stable immobilized catalyst of chlorogenate hydrolase. Applied Microbiology and Biotechnology 81:143-151.
16. Okamura S, Watanabe M. 1982. Purification and properties of hydroxycinnamic acid ester hydrolase from Aspergillus japonicus. Agric. Biol. Chem. 46:1839-1848.
17. Barbe C, Dubourdieu D. 1998. Characterization and purification of a cinnamate esterase from Aspergillus niger industrial pectinase preparation. J Sci Food Agric 78:471-478.
18. Levasseur A, Benoit I, Asther M, Asther M, Record E. 2004. Homologous expression of the feruloyl esterase B gene from Aspergillus niger and characterization of the recombinant enzyme. Protein expression and purification 37:126-133.
19. Koseki T, Hori A, Seki S, Murayama T, Shiono Y. 2009. Characterization of two distinct feruloyl esterases, AoFaeB and AoFaeC, from Aspergillus oryzae. Applied Microbiology and Biotechnology 83:689-696.
20. Vafiadi C, Topakas E, Christakopoulos P, Faulds CB. 2006. The feruloyl esterase system of Talaromyces stipitatus: determining the hydrolytic and synthetic specificity of TsFaeC. Journal of biotechnology 125:210-221.
21. Qi M, Wang P, Selinger LB, Yanke LJ, Forster RJ, McAllister TA. 2011. Isolation and characterization of a ferulic acid esterase (Fae1A) from the rumen fungus Anaeromyces mucronatus. Journal of applied microbiology 110:1341-1350.
22. Couteau D, McCartney AL, Gibson GR, Williamson G, Faulds CB. 2001. Isolation and characterization of human colonic bacteria able to hydrolyse chlorogenic acid. Journal of applied microbiology 90:873-881.
23. Borneman WS, Hartley RD, Morrison WH, Akin DE, Ljungdahl LG. 1990. Feruloyl and p-coumaroyl esterase from anaerobic fungi in relation to plant cell wall degradation. Applied Microbiology and Biotechnology 33:345-351.
24. Allerdings E, Ralph J, Steinhart H, Bunzel M. 2006. Isolation and structural identification of complex feruloylated heteroxylan side-chains from maize bran. Phytochemistry (Elsevier) 67:1276-1286.
25. Linke D, Matthes R, Nimtz M, Zorn H, Bunzel M, Berger RG. 2013. An esterase from the basidiomycete Pleurotus sapidus hydrolyzes feruloylated saccharides. Applied Microbiology and Biotechnology 97:7241-7251.
26. Garcia-Conesa MT, Crepin VF, Goldson AJ, Williamson G, Cummings NJ, Connerton IF, Faulds CB, Kroon PA. 2004. The feruloyl esterase system of Talaromyces stipitatus: production of three discrete feruloyl esterases, including a novel enzyme, TsFaeC, with a broad substrate specificity. Journal of biotechnology 108:227-241.
27. Johnson KG, Harrison BA, Schneider H, MacKenzie CR, Fontana JD. 1988. Xylan-hydrolysing enzymes from Streptomyces spp. Enzyme Microb. Technol. 10**.**
28. Laemmli UK. 1970. Cleavage of structural proteins during the assembly of the head of bacteriophage T4. Nature 227:680-685.
29. Nieter A, Haase-Aschoff P, Linke D, Nimtz M, Berger RG. 2014. A halotolerant type A feruloyl esterase from Pleurotus eryngii. Fungal biology 118:348-357.
30. Lin-Cereghino J, Wong WW, Xiong S, Giang W, Luong LT, Vu J, Johnson SD, Lin-Cereghino GP. 2005. Condensed protocol for competent cell preparation and transformation of the methylotrophic yeast Pichia pastoris. BioTechniques 38:44, 46, 48.
31. Sygmund C, Gutmann A, Krondorfer I, Kujawa M, Glieder A, Pscheidt B, Haltrich D, Peterbauer C, Kittl R. 2012. Simple and efficient expression of Agaricus meleagris pyranose dehydrogenase in Pichia pastoris. Applied Microbiology and Biotechnology 94:695-704.
32. Nicolaus RA, Piattelli M. 1965. Progress in the chemistry of natural black pigments. Academia delle Scienze Fisiche e Matematiche in Napoli. Rendiconto (Series 4) 32:83 - 97.
33. Wheeler MH. 1983. Comparison of fungal melanin biosynthesis in ascomycetous, imperfect and basidiomycetous fungi. Trans. Br. mycol. Soc. 81:29-36.
34. Benoit I, Navarro D, Marnet N, Rakotomanomana N, Lesage-Meessen L, Sigoillot JC, Asther M, Asther M. 2006. Feruloyl esterases as a tool for the release of phenolic compounds from agro-industrial by-products. Carbohydrate research 341:1820-1827.
35. Crepin VF, Faulds CB, Connerton IF. 2003. A non-modular type B feruloyl esterase from Neurospora crassa exhibits concentration-dependent substrate inhibition. The Biochemical journal 370:417-427.
36. Koseki T, Fushinobu S, Ardiansyah, Shirakawa H, Komai M. 2009. Occurrence, properties, and applications of feruloyl esterases. Applied Microbiology and Biotechnology 84:803-810.
37. Sigoillot C, Camarero S, Vidal T, Record E, Asther M, Perez-Boada M, Martinez MJ, Sigoillot JC, Colom JF, Martinez AT. 2005. Comparison of different fungal enzymes for bleaching high-quality paper pulps. Journal of biotechnology 115:333-343.
38. Topakas E, Stamatis H, Biely P, Kekos D, Macris BJ, Christakopoulos P. 2003. Purification and characterization of a feruloyl esterase from Fusarium oxysporum catalyzing esterification of phenolic acids in ternary water-organic solvent mixtures. Journal of biotechnology 102:33-44.
39. Topakas E, Stamatis H, Biely P, Christakopoulos P. 2004. Purification and characterization of a type B feruloyl esterase (StFAE-A) from the thermophilic fungus Sporotrichum thermophile. Applied Microbiology and Biotechnology 63:686-690.
40. Vallee BL, Ulmer DD. 1972. Biochemical effects of mercury, cadmium and lead. Annu Rev Biochem. 41:91-128.
41. Wang L, Zhang R, Ma Z, Wang H, Ng T. 2014. A feruloyl esterase (FAE) characterized by relatively high thermostability from the edible mushroom Russula virescens. Applied biochemistry and biotechnology 172:993-1003.
42. Donaghy J, McKay AM. 1997. Purification and characterization of a feruloyl esterase from the fungus Penicillium expansum. Journal of applied microbiology 83:718-726.
43. Yao J, Chen QL, Shen AX, Cao W, Liu YH. 2013. A novel feruloyl esterase from a soil metagenomic library with tannase activity. J. Mol. Catal. B: Enzym. 95:55-61.
44. Gold AM, Fahrney D. 1964. Sulfonyl fluorides as inhibitors of esterases. II. Formation and reactions of phenylmethanesulfonyl alpha chymotrypsin. Biochemistry 3:783-791.
45. Anson ML. 1940. The reactions of iodine and iodoacetamide with native egg albumin. The Journal of general physiology 23:321-331.
46. Kumar L, Singh B, Adhikari DK, Mukherjee J, Ghosh D. 2012. A thermoalkaliphilic halotolerant esterase from Rhodococcus sp. LKE-028 (MTCC 5562): Enzyme purification and characterization. Process Biochem. 47:983-991.
47. Benoit I, Danchin EGJ, Bleichrodt R-J, de Vries RP. 2008. Biotechnological applications and potential of fungal feruloyl esterases based on prevalence, classification and biochemical diversity. Biotechnology Letters 30:387-396.
48. Udatha DB, Kouskoumvekaki I, Olsson L, Panagiotou G. 2011. The interplay of descriptor-based computational analysis with pharmacophore modeling builds the basis for a novel classification scheme for feruloyl esterases. Biotechnology advances 29:94-110.
49. de Vries RP, vanKuyk PA, Kester HC, Visser J. 2002. The Aspergillus niger faeB gene encodes a second feruloyl esterase involved in pectin and xylan degradation and is specifically induced in the presence of aromatic compounds. The Biochemical journal 363:377-386.
50. Topakas E, Christakopoulos P, Faulds CB. 2005. Comparison of mesophilic and thermophilic feruloyl esterases: characterization of their substrate specificity for methyl phenylalkanoates. Journal of biotechnology 115:355-366.
51. Kroon PA, Faulds CB, Brezillon C, Williamson G. 1997. Methyl phenylalkanoates as substrates to probe the active sites of esterases. European journal of biochemistry / FEBS 248:245-251.
52. Faulds CB, Sancho Al, Bartolome B. 2002. Mono- and dimeric ferulic acid release from brewer's spent grain by fungal feruloyl esterases. Applied Microbiology and Biotechnology 60:489-494.
53. Moukouli M, Topakas E, Christakopoulos P. 2008. Cloning, characterization and functional expression of an alkalitolerant type C feruloyl esterase from Fusarium oxysporum. Applied Microbiology and Biotechnology 79:245-254.
54. Faulds CB, Williamson G. 1995. Release of ferulic acid from wheat bran by a ferulic acid esterase (FAE-III) from Aspergillus niger. Applied Microbiology and Biotechnology 43:1082-1087.
55. Benoit I, Asther M, Sulzenbacher G, Record E, Marmuse L, Parsiegla G, Gimbert I, Asther M, Bignon C. 2006. Respective importance of protein folding and glycosylation in the thermal stability of recombinant feruloyl esterase A. FEBS letters 580:5815-5821.
56. Brenner S. 1988. The molecular evolution of genes and proteins: a tale of two serines. Nature 334:528-530.
57. Dodson G, Wlodawer A. 1998. Catalytic triads and their relatives. Trends in biochemical sciences 23:347-352.
58. Shin HD, Chen RR. 2007. A type B feruloyl esterase from Aspergillus nidulans with broad pH applicability. Applied Microbiology and Biotechnology 73:1323-1330.
59. Mubarak A, Hodgson JM, Considine MJ, Croft KD, Matthews VB. 2013. Supplementation of a high-fat diet with chlorogenic acid is associated with insulin resistance and hepatic lipid accumulation in mice. Journal of agricultural and food chemistry 61:4371-4378.
60. GenBank accession no. **XP_756329.1**
61. Kieffer, R, Wieser, H, Henderson, MH, & Graveland, A. 1998. Correlations of the Breadmaking Performance of Wheat Flour with Rheological Measurements on a Micro-scale. Journal of Cereal Science, 27(1), 53-60.
62. MacKenzie and Bilous, 1988, Appl Environ Microbiol 54:1170-1173.

## Claims

1. An enzyme with chlorogenic acid esterase activity and feruloyl esterase activity comprising a sequence having at least 85% amino acid identity to SEQ ID NO: 1, and not comprising SEQ ID NO: 3.

2. The enzyme of claim 1, wherein the enzyme has a molecular mass of about 71 kDa.

3. The enzyme of any of the preceding claims comprising a sequence having SEQ ID NO: 1.

4. The enzyme of any of the preceding claims, wherein the enzyme is obtainable from a basidiomycete.

5. The enzyme of any of the preceding claims, wherein the enzyme is obtainable by expression in yeast, preferably, in *Pichia pastoris.*

6. A nucleic acid encoding the enzyme of any of the preceding claims, not comprising SEQ ID NO: 4.

7. The nucleic acid of claim 6, wherein the nucleic acid is an expression vector comprising the nucleic acid of claim 6 functionally linked to a heterologous promoter, wherein the nucleic acid encoding the enzyme is preferably functionally linked to a heterologous signal sequence.

8. A method of preparing an enzyme of any of claims 1-5 comprising transforming a suitable host cell with the nucleic acid of any of claims 6 or 7, and, optionally, isolating the enzyme.

9. A heterologous host cell comprising the nucleic acid of any of claims 6 or 7.

10. Use of an enzyme of any of claims 1-5 for dough softening.

11. A method of preparing a bakery product, comprising contacting dough of the bakery product with an enzyme of any of claims 1-5.

12. Use of an enzyme of any of claims 1-5, for hydrolyzing a substrate and/or generating a product selected from the group comprising
a. a chlorogenic acid, i.e, an ester of quinic acid with a phenylpropanoic acid, preferably, caffeic acid; and/or
b. an ester of a saccharide or C1- or C2-alcohol with phenylpropanoic acid optionally comprising exactly one methoxy on the benzoic ring, preferably, caffeic acid.

13. A method of hydrolyzing a substrate selected from the group comprising
a. an ester of quinic acid with a phenylpropanoic acid, preferably, caffeic acid; and/or
b. an ester of a saccharide or C1- or C2-alcohol with phenylpropanoic acid optionally comprising exactly one methoxy on the benzoic ring, preferably, caffeic acid,
the method comprising a step wherein an enzyme of any of claims 1-5 is brought into contact with said substrate under suitable conditions.

14. A method of preparing a product selected from the group comprising
a. quinic acid;
b. phenylpropanoic acid optionally comprising exactly one methoxy on the benzoic ring, preferably, caffeic acid;
c. a saccharide;
d. an ester of quinic acid with a phenylpropanoic acid, preferably, caffeic acid;
e. an ester of a saccharide or C1- or C2-alcohol with a phenylpropanoic acid optionally comprising exactly one methoxy on the benzoic ring, preferably, caffeic acid; and/or
f. a natural material such as food comprising a reduced amount of an ester of quinic acid with a phenylpropanoic acid, preferably, caffeic acid;
the method comprising a step wherein an enzyme of any of claims 1-5 is brought into contact with the educts of the catalyzed reaction generating the respective product under suitable conditions, wherein the enzyme is preferably contacted with a natural material such as agro-industrial waste material, such as coffee pulp, destarched wheat bran, and sugar beet pectin or paper pulp;
wherein the product is optionally isolated.

15. A method for reducing dietary risks involving in the consumption of chlorogenic acid, comprising contacting a food product with an enzyme of any of claims 1-5.

16. A food product comprising an enzyme of any of claims 1-5, wherein the food product optionally is a bakery product selected from the group comprising dough, par-baked product and baked product.

## Patentansprüche

1. Ein Enzym mit Chlorogensäure-Esterase-Aktivität und Feruloyl-Esterase-Aktivität, umfassend eine Sequenz mit mindestens 85% Aminosäure-Identität zu SEQ ID NO: 1, welche nicht SEQ ID NO: 3 umfasst.

2. Das Enzym nach Anspruch 1, wobei das Enzym eine Molekülmasse von ungefähr 71 kDa aufweist.

3. Das Enzym nach einem der vorhergehenden Ansprüche, umfassend eine Sequenz mit SEQ ID NO: 1.

4. Das Enzym nach einem der vorhergehenden Ansprüche, wobei das Enzym aus einem Basidiomyceten erhältlich ist.

5. Das Enzym nach einem der vorhergehenden Ansprüche, wobei das Enzym durch Expression in Hefe, bevorzugt in *Pichia pastoris,* erhältlich ist.

6. Eine Nukleinsäure, kodierend für das Enzym nach einem der vorhergehenden Ansprüche, nicht umfassend SEQ ID NO: 4.

7. Die Nukleinsäure nach Anspruch 6, wobei die Nukleinsäure ein Expressionsvektor ist, umfassend die Nukleinsäure nach Anspruch 6, die funktionell mit einem heterologen Promoter verbunden ist, wobei die für das Enzym kodierende Nukleinsäure bevorzugt funktionell mit einer heterologen Signalsequenz verbunden ist.

8. Ein Verfahren zur Herstellung eines Enzyms nach einem der Ansprüche 1-5, umfassend das Transformieren einer geeigneten Wirtszelle mit der Nukleinsäure nach einem der Ansprüche 6 oder 7 und, optional, das Isolieren des Enzyms.

9. Eine heterologe Wirtszelle, umfassend die Nukleinsäure nach einem der Ansprüche 6 oder 7.

10. Verwendung eines Enzyms nach einem der Ansprüche 1-5 zum Erweichen von Teig.

11. Ein Verfahren zur Herstellung eines Backwarenprodukts, umfassend das In-Kontakt-Bringen von Teig des Backwarenprodukts mit einem Enzym nach einem der Ansprüche 1-5.

12. Verwendung eines Enzyms nach einem der Ansprüche 1-5 zur Hydrolyse eines Substrats und/oder Generierung eines Produkts, ausgewählt aus der Gruppe umfassend
a. eine Chlorogensäure, d. h., einen Ester von Chinasäure mit einer Phenylpropansäure, bevorzugt Kaffeesäure; und/oder
b. einen Ester eines Saccharids oder C1- oder C2-Alkohols mit Phenylpropansäure, die optional exakt eine Methoxygruppe auf dem Benzolring aufweist, bevorzugt Kaffeesäure.

13. Ein Verfahren zur Hydrolyse eine Substrats, ausgewählt aus der Gruppe umfassend
a. einen Ester von Chinasäure mit einer Phenylpropansäure, bevorzugt Kaffeesäure; und/oder
b. einen Ester eines Saccharids oder C1- oder C2-Alkohols mit Phenylpropansäure, die optional exakt eine Methoxygruppe auf dem Benzolring aufweist, bevorzugt Kaffeesäure,
wobei das Verfahren einen Schritt umfasst, bei dem ein Enzym nach einem der Ansprüche 1-5 unter geeigneten Bedingungen mit dem Substrat in Kontakt gebracht wird.

14. Ein Verfahren zur Herstellung eines Produkts, ausgewählt aus der Gruppe umfassend
a. Chinasäure;
b. Phenylpropansäure, die optional exakt eine Methoxygruppe auf dem Benzolring aufweist, bevorzugt Kaffeesäure;
c. ein Saccharid;
d. einen Ester von Chinasäure mit einer Phenylpropansäure, bevorzugt Kaffeesäure;
e. einen Ester eines Saccharids oder C1-oder C2-Alkohols mit einer Phenylpropansäure, die optional exakt eine Methoxygruppe auf dem Benzolring aufweist, bevorzugt Kaffeesäure; und/oder
f. ein natürliches Material wie Lebensmittel, umfassend eine reduzierte Menge eines Esters von Chinasäure mit einer Phenylpropansäure, bevorzugt Kaffeesäure;
wobei das Verfahren einen Schritt umfasst, bei dem ein Enzym nach einem der Ansprüche 1-5 unter geeigneten Bedingungen mit den Edukten der katalysierten Reaktion, die das entsprechende Produkt generiert, in Kontakt gebracht wird, wobei das Enzym bevorzugt mit einem natürlichen Material wie agro-industriellem Abfall, wie Kaffeepulpe, entstärkter Weizenkleie und Zuckerrübenpektin oder Papierpulpe in Kontakt gebracht wird;
wobei das Produkt optional isoliert wird.

15. Ein Verfahren zur Verringerung von ernährungsbedingten Risiken bei der Konsumierung von Chlorogensäure, umfassend das In-Kontakt-Bringen eines Lebensmittelprodukts mit einem Enzym nach einem der Ansprüche 1-5.

16. Ein Lebensmittelprodukt, das ein Enzym nach einem der Ansprüche 1-5 umfasst, wobei das Lebensmittelprodukt optional ein Backwarenprodukt ist, ausgewählt aus der Gruppe, die Teig, ein halbgebackenes Produkt und ein gebackenes Produkt umfasst.

## Revendications

1. Enzyme à activité acide chlorogénique estérase et activité féruloyle estérase, comprenant une séquence ayant un degré d'identité d'au moins 85 % avec la séquence SEQ ID NO: 1 et ne comprenant pas la séquence SEQ ID NO: 3.

2. Enzyme selon la revendication 1, où l'enzyme a une masse moléculaire d'environ 71 kDa.

3. Enzyme selon l'une quelconque des revendications précédentes, comprenant une séquence ayant la séquence SEQ ID NO: 1.

4. Enzyme selon l'une quelconque des revendications précédentes, où l'enzyme peut être obtenue à partir d'un basidiomycète.

5. Enzyme selon l'une quelconque des revendications précédentes, où l'enzyme peut être obtenue par expression dans une levure, de préférence dans *Pichia pastoris.*

6. Acide nucléique codant l'enzyme selon l'une quelconque des revendications précédentes, ne comprenant pas la séquence SEQ ID NO: 4.

7. Acide nucléique selon la revendication 6, où l'acide nucléique est un vecteur d'expression comprenant l'acide nucléique de la revendication 6 fonctionnellement lié à un promoteur hétérologue, dans lequel l'acide nucléique codant l'enzyme est de préférence fonctionnellement lié à une séquence signal hétérologue.

8. Procédé de préparation d'une enzyme selon l'une quelconque des revendications 1 à 5, comprenant le fait de transformer une cellule hôte convenable avec l'acide nucléique de l'une quelconque des revendications 6 et 7, et, en option, le fait d'isoler l'enzyme.

9. Cellule hôte hétérologue comprenant l'acide nucléique de l'une quelconque des revendications 6 et 7.

10. Utilisation d'une enzyme de l'une quelconque des revendications 1 à 5 pour l'assouplissement de pâte.

11. Procédé de préparation d'un produit de boulangerie, comprenant le fait de mettre la pâte du produit de boulangerie en contact avec une enzyme selon l'une quelconque des revendications 1 à 5.

12. Utilisation d'une enzyme selon l'une quelconque des revendications 1 à 5, pour hydrolyser un substrat et/ou engendrer un produit choisi dans l'ensemble comprenant
a. un acide chlorogénique, à savoir un ester d'acide quinique avec un acide phénylpropanoïque, de préférence l'acide caféique ; et/ou
b. un ester d'un saccharide ou alcool en C₁ ou C₂ avec un acide phénylpropanoïque en option comprenant exactement un groupe méthoxy sur le cycle benzoïque, de préférence, l'acide caféique.

13. Procédé d'hydrolyse d'un substrat choisi dans l'ensemble comprenant
a. un ester d'acide quinique avec un acide phénylpropanoïque, de préférence l'acide caféique ; et/ou
b. un ester d'un saccharide ou alcool en C₁ ou C₂ avec un acide phénylpropanoïque en option comprenant exactement un groupe méthoxy sur le cycle benzoïque, de préférence, l'acide caféique,
le procédé comprenant une étape dans laquelle une enzyme selon l'une quelconque des revendications 1 à 5 est mise en contact avec ledit substrat dans des conditions convenables.

14. Procédé de préparation d'un produit choisi dans l'ensemble comprenant
a. l'acide quinique ;
b. un acide phénylpropanoïque, en option comprenant exactement un groupe méthoxy sur le cycle benzoïque, de préférence, l'acide caféique ;
c. un saccharide ;
d. un ester d'acide quinique avec un acide phénylpropanoïque, de préférence l'acide caféique ;
e. un ester d'un saccharide ou alcool en C₁ ou C₂ avec un acide phénylpropanoïque en option comprenant exactement un groupe méthoxy sur le cycle benzoïque, de préférence, l'acide caféique ; et/ou
f. une matière naturelle telle qu'un produit alimentaire comprenant une quantité réduite d'un ester d'acide quinique avec un acide phénylpropanoïque, de préférence l'acide caféique ;
le procédé comprenant une étape dans laquelle une enzyme selon l'une quelconque des revendications 1 à 5 est mise en contact avec les produits de départ de la réaction catalysée, pour engendrer le produit respectif dans des conditions convenables, dans lequel l'enzyme est de préférence mise en contact avec une matière naturelle, telle qu'une matière de déchet agro-industriel, telle que de la pulpe de café, le son de blé désamidonné, et la pectine de betterave sucrière ou la pâte à papier ;
dans lequel le produit est en option isolé.

15. Procédé pour réduire les risques alimentaires impliqués dans la consommation d'acide chlorogénique, comprenant le fait de mettre un produit alimentaire en contact avec une enzyme selon l'une quelconque des revendications 1 à 5.

16. Produit alimentaire comprenant une enzyme selon l'une quelconque des revendications 1 à 5, où le produit alimentaire en option est un produit de boulangerie choisi dans l'ensemble comprenant de la pâte, un produit précuit et un produit cuit.


### SEQUENCE LISTING

<110> SternEnzym GmbH&Co. KG
<120> An enzyme with chlorogenic acid esterase activity and feruloyl esterase activity
<130> STR15531PCT
<160> 31
<170> BiSSAP 1.3
<210> 1
   <211> 564
   <212> PRT
   <213> Ustilago maydis
<400> 1
   Leu Pro Gln Val Ser Tyr Lys Ala Asp Ala Thr Ala Ser Ala Pro Thr
   1               5                   10                  15
   Val Lys Leu His Gln Gly Thr Val Arg Gly Leu Ala Asp Asp Asn Tyr
               20                  25                  30
   Gly Leu Glu Gln Phe Phe Gly Ile Pro Tyr Ala Lys Pro Pro Val Gly
           35                  40                  45
   Ser Leu Arg Phe Ala Lys Pro Gln Pro Leu Gly Pro Ala Ser Ser His
       50                  55                  60
   Lys Thr Val Ile Asp Ala Thr Arg Phe Gly Asp Ile Cys Met Gln Thr
   65                  70                  75                  80
   Val Ala Pro Ser Pro Leu Tyr Asn Met Ser Glu Asp Cys Leu Asn Leu
                   85                  90                  95
   Asn Val Val Arg Pro Lys Gly Thr Thr Ala Lys Asp Lys Leu Pro Val
               100                 105                 110
   Leu Val Trp Ile Tyr Gly Gly Ala Phe Arg Gln Gly Ser Thr Pro Ile
           115                 120                 125
   Tyr Asn Ala Ser Glu Leu Val Gln Lys Ser Val Glu Ile Gly Lys Pro
       130                 135                 140
   Ile Val Phe Val Ala Ile Ser Tyr Arg Val Gly Pro Phe Gly Phe Ile
   145                 150                 155                 160
   Gly Gly Ser Glu Ile Ala Asp Ser Asp Ser Ala Thr Ser Asn Ala Gly
                   165                 170                 175
   Leu Tyr Asp Gln Arg Leu Gly Leu Lys Trp Ile Arg His Asn Ile Gly
               180                 185                 190
   Lys Phe Gly Gly Asp Lys Asn Arg Val Thr Leu Phe Gly Gln Ser Ala
           195                 200                 205
   Gly Ala Met Ser Ile Ala Leu Gln Asn Phe Ala Tyr Asp Gly Asn Asn
       210                 215                 220
   His Gly Leu Trp His Ala Ala Ile Met Asn Ser Gly Gly Ile Ala Pro
   225                 230                 235                 240
   Gly Pro Leu Leu Thr Pro Lys His Pro Thr Val Glu Gln Ser Phe Lys
                   245                 250                 255
   Arg Leu Ala Asn Gly Val Gly Cys Thr Gly Gly Ser Leu Leu Arg Cys
               260                 265                 270
   Leu Arg Lys Ala Asn Ala Ser Glu Val Gln Thr Val Ala Ser Asn Leu
           275                 280                 285
   Thr Ala Gln Ala Gly Gly Thr Phe Pro Ile Pro Gly Ala Leu Ala Trp
       290                 295                 300
   Leu Pro Leu Val Asp Tyr Glu Leu Ile Thr Asn Tyr Pro Ser Val Asn
   305                 310                 315                 320
   Leu Pro Gln Gly Lys Leu Ala Asp Ile Pro Val Ile Gln Gly Asn Ala
                   325                 330                 335
   Leu Asp Glu Gly Thr Ser Phe Ala Gln Lys Gln Leu Asn Ser Ser Ala
               340                 345                 350
   Asp Phe Glu Arg Trp Val Arg Ser Ala Ala Val Ile His Asn Thr Ser
           355                 360                 365
   Tyr Thr Glu Gln Ala Leu Gln Lys Val Phe Glu Leu Tyr Pro Asp Val
       370                 375                 380
   Pro Glu Gln Gly Ser Pro Phe Tyr Asn Ala Glu Thr Ala Thr Ser Ala
   385                 390                 395                 400
   Ala Thr Thr Ser Asp Leu Asn Ser Arg Gln Tyr Pro Pro Leu Thr Ser
                   405                 410                 415
   Asn Gln Tyr Lys Arg Ser Ala Ala Phe Phe Gly Asp Phe Thr Phe Gln
               420                 425                 430
   Ala Gln Arg Arg Thr Tyr Leu Lys Ala Ala Thr Leu Gly Trp Lys Lys
           435                 440                 445
   Asn Lys Ala Lys Val Trp Ser Tyr Glu Phe Gln Gln Asn Asp Lys Phe
       450                 455                 460
   Ala Asn Gly Thr Gly Ser Leu Leu Gly Pro Tyr His Gly Ala Asp Val
   465                 470                 475                 480
   Lys Tyr Tyr Phe Ile Arg Pro Asp Gly Arg Gln Lys Asp Pro Val Leu
                   485                 490                 495
   Ala Asp Arg Met Pro Arg Ala Tyr Ile Ser Phe Val Tyr His His Asp
               500                 505                 510
   Pro Thr Val Leu Gly Gly Phe Glu Trp Pro Pro Tyr Gly Lys Gly Lys
           515                 520                 525
   Lys Leu Leu Gln Met Lys Gly Asp Asn Thr Thr Val Ile Glu Asp Ala
       530                 535                 540
   Tyr Arg Lys Glu Ala Met Asp Ala Leu Thr Asn Arg Lys Ala Ala Lys
   545                 550                 555                 560
   Val Phe Gly Phe
<210> 2
   <211> 1695
   <212> **DNA**
   <213> Ustilago maydis
<400> 2
   cttccacaag tctcttacaa agctgacgcc accgcctctg ctcccacagt caaactccac      60
   caaggtactg tgcgcggctt ggccgacgac aattatggcc tggaacaatt ctttggcatt     120
   ccctatgcca agccacccgt cggctcgctc cgatttgcca agccacagcc tctcggccca     180
   gcctcgtccc acaaaacggt catcgacgct actcgattcg gcgacatctg tatgcagact     240
   gttgcaccct cgcctctcta taacatgagc gaagactgtc tcaatctcaa tgtcgttcgt     300
   cccaaaggaa caacggccaa ggacaagctt ccggtgctgg tttggatcta tggtggggcg     360
   ttcaggcaag gctcaacgcc catttacaac gccagcgaat tggtgcagaa gagcgtcgag     420
   attggcaagc cgattgtatt tgttgcgatc agctatcgag tgggtccatt cggcttcatc     480
   ggtggatccg agattgctga cagtgactcg gctaccagca acgcagggct gtatgatcag     540
   agattgggac tcaagtggat tcgccacaac attggcaagt ttggaggtga caagaacagg     600
   gtcactctgt tcggtcagag tgcgggcgcg atgagcattg ctctgcagaa ctttgcctat     660
   gatggtaaca accacgggct ttggcatgcc gcgatcatga actcgggcgg aattgctccg     720
   ggtccactgt taacgcccaa acaccctacg gtggaacaga gcttcaagag gctggccaat     780
   ggcgttggat gtacaggagg atcactgctc cggtgtctgc gtaaagcaaa cgcgagtgag     840
   gttcagaccg tggccagtaa tctgactgct caagccggcg gaacatttcc cattcctgga     900
   gcgcttgcgt ggttgccact ggtcgactac gagctcatca ccaactatcc cagtgtcaac     960
   cttccgcaag gtaaactggc cgacatcccg gtgatccaag gaaacgctct ggacgaagga    1020
   acatcttttg cccagaaaca gctcaacagc agcgccgatt tcgaacggtg ggtcagaagc    1080
   gctgctgtca tccacaacac ttcgtacacc gagcaagcgc ttcaaaaggt ctttgaactc    1140
   tacccagatg ttcctgaaca gggctcgcca ttctacaatg cggagacggc cacttctgct    1200
   gctaccacgt cggacctcaa ttcgcgccag tatccacctt tgaccagcaa tcagtacaag    1260
   cgttcagcgg cgttctttgg cgacttcact ttccaggcgc agcgacgaac ttatctcaag    1320
   gcggcaacct tgggctggaa gaagaacaag gccaaagtat ggtcctacga gttccagcag    1380
   aacgacaagt ttgctaatgg tactggatcg ctgttgggcc cataccatgg tgctgatgtc    1440
   aagtactatt ttattcgacc tgatggaagg cagaaggacc ccgtcttggc cgatagaatg    1500
   cctagggcgt atatcagttt cgtgtatcac catgatccca cggtgctcgg tggtttcgaa    1560
   tggccgccct acggaaaagg caagaagctg ctgcagatga aaggtgacaa cacgacagtg    1620
   atcgaggatg cctataggaa ggaggcgatg gatgcgctga ccaacaggaa agctgccaag    1680
   gtgtttggct tctaa                                                     1695
<210> 3
   <211> 21
   <212> PRT
   <213> Ustilago maydis
<220>
   <223> Signal sequence
<400> 3
   Met Arg Leu Pro Asn Leu Thr Leu Leu Val Trp Ala Ala Thr Ser Val
   1               5                   10                  15
   Gly Leu Val Ser Ala
               20
<210> 4
   <211> 63
   <212> DNA
   <213> Ustilago maydis
<400> 4
   atgaggctgc ctaatctgac actgctcgta tgggcagcca catccgtcgg cttggtgtcg      60
   gct                                                                    63
<210> 5
   <211> 21
   <212> DNA
   <213> Artificial sequence
<220>
   <223> Primer
<400> 5
   tccgttctta gaagccaaac a 21
<210> 6
   <211> 20
   <212> DNA
   <213> Artificial sequence
<220>
   <223> Primer
<400> 6
   gaaagccaag caacaaggtt 20
<210> 7
   <211> 38
   <212> DNA
   <213> Artificial sequence
<220>
   <223> Primer
<400> 7
   tttttctcga gaaaagagag gctaggctgc ctaatctg 38
<210> 8
   <211> 43
   <212> DNA
   <213> Artificial sequence
<220>
   <223> Primer
<400> 8
   tttttctcga gaaaagagag gctgaagctc ttccacaagt ctc 43
<210> 9
   <211> 46
   <212> DNA
   <213> Artificial sequence
<220>
   <223> Primer
<400> 9
   tttttgcggc cgcttaatga tgatgatgat gatggaagcc aaacac 46
<210> 10
   <211> 11
   <212> PRT
   <213> Ustilago maydis
<220>
   <223> Tryptic peptide obtained by ESI-MS/MS
<400> 10
   Ala Asp Ala Thr Ala Ser Ala Pro Thr Val Lys
   1               5                   10
<210> 11
   <211> 14
   <212> PRT
   <213> Ustilago maydis
<220>
   <223> Tryptic peptide obtained by ESI-MS/MS
<400> 11
   Phe Ala Lys Pro Gln Pro Leu Gly Pro Ala Ser Ser His Lys
   1               5                   10
<210> 12
   <211> 10
   <212> PRT
   <213> Ustilago maydis
<220>
   <223> Tryptic peptide obtained by ESI-MS/MS
<400> 12
   His Pro Thr Val Glu Gln Ser Phe Lys Arg
   1 5 10
<210> 13
   <211> 14
   <212> PRT
   <213> Ustilago maydis
<220>
   <223> Tryptic peptide obtained by ESI-MS/MS
<400> 13
   Leu Ala Asn Gly Val Gly Cys Thr Gly Gly Ser Leu Leu Arg
   1 5 10
<210> 14
   <211> 11
   <212> PRT
   <213> Ustilago maydis
<220>
   <223> Tryptic peptide obtained by ESI-MS/MS
<400> 14
   Val Trp Ser Tyr Glu Phe Gln Gln Asn Asp Lys
   1 5 10
<210> 15
   <211> 9
   <212> PRT
   <213> Ustilago maydis
<220>
   <223> Tryptic peptide obtained by ESI-MS/MS
<400> 15
   Glu Ala Met Asp Ala Leu Thr Asn Arg
   1 5
<210> 16
   <211> 9
   <212> PRT
   <213> Ustilago maydis
<220>
   <223> Tryptic peptide obtained by ESI-MS/MS
<400> 16
   Ala Lys Pro Pro Val Gly Ser Leu Arg
   1 5
<210> 17
   <211> 585
   <212> PRT
   <213> Ustilago maydis
<220>
   <223> Full-length incl. native signal sequence and P492L
<400> 17
   Met Arg Leu Pro Asn Leu Thr Leu Leu Val Trp Ala Ala Thr Ser Val
   1               5                   10                  15
   Gly Leu Val Ser Ala Leu Pro Gln Val Ser Tyr Lys Ala Asp Ala Thr
               20                  25                  30
   Ala Ser Ala Pro Thr Val Lys Leu His Gln Gly Thr Val Arg Gly Leu
           35                  40                  45
   Ala Asp Asp Asn Tyr Gly Leu Glu Gln Phe Phe Gly Ile Pro Tyr Ala
       50                  55                  60
   Lys Pro Pro Val Gly Ser Leu Arg Phe Ala Lys Pro Gln Pro Leu Gly
   65                  70                  75                  80
   Pro Ala Ser Ser His Lys Thr Val Ile Asp Ala Thr Arg Phe Gly Asp
                   85                  90                  95
   Ile Cys Met Gln Thr Val Ala Pro Ser Pro Leu Tyr Asn Met Ser Glu
               100                 105                 110
   Asp Cys Leu Asn Leu Asn Val Val Arg Pro Lys Gly Thr Thr Ala Lys
           115                 120                 125
   Asp Lys Leu Pro Val Leu Val Trp Ile Tyr Gly Gly Ala Phe Arg Gln
       130                 135                 140
   Gly Ser Thr Pro Ile Tyr Asn Ala Ser Glu Leu Val Gln Lys Ser Val
   145                 150                 155                 160
   Glu Ile Gly Lys Pro Ile Val Phe Val Ala Ile Ser Tyr Arg Val Gly
                   165                 170                 175
   Pro Phe Gly Phe Ile Gly Gly Ser Glu Ile Ala Asp Ser Asp Ser Ala
               180                 185                 190
   Thr Ser Asn Ala Gly Leu Tyr Asp Gln Arg Leu Gly Leu Lys Trp Ile
           195                 200                 205
   Arg His Asn Ile Gly Lys Phe Gly Gly Asp Lys Asn Arg Val Thr Leu
       210                 215                 220
   Phe Gly Gln Ser Ala Gly Ala Met Ser Ile Ala Leu Gln Asn Phe Ala
   225                 230                 235                 240
   Tyr Asp Gly Asn Asn His Gly Leu Trp His Ala Ala Ile Met Asn Ser
                   245                 250                 255
   Gly Gly Ile Ala Pro Gly Pro Leu Leu Thr Pro Lys His Pro Thr Val
               260                 265                 270
   Glu Gln Ser Phe Lys Arg Leu Ala Asn Gly Val Gly Cys Thr Gly Gly
           275                 280                 285
   Ser Leu Leu Arg Cys Leu Arg Lys Ala Asn Ala Ser Glu Val Gln Thr
       290                 295                 300
   Val Ala Ser Asn Leu Thr Ala Gln Ala Gly Gly Thr Phe Pro Ile Pro
   305                 310                 315                 320
   Gly Ala Leu Ala Trp Leu Pro Leu Val Asp Tyr Glu Leu Ile Thr Asn
                   325                 330                 335
   Tyr Pro Ser Val Asn Leu Pro Gln Gly Lys Leu Ala Asp Ile Pro Val
               340                 345                 350
   Ile Gln Gly Asn Ala Leu Asp Glu Gly Thr Ser Phe Ala Gln Lys Gln
           355                 360                 365
   Leu Asn Ser Ser Ala Asp Phe Glu Arg Trp Val Arg Ser Ala Ala Val
       370                 375                 380
   Ile His Asn Thr Ser Tyr Thr Glu Gln Ala Leu Gln Lys Val Phe Glu
   385                 390                 395                 400
   Leu Tyr Pro Asp Val Pro Glu Gln Gly Ser Pro Phe Tyr Asn Ala Glu
                   405                 410                 415
   Thr Ala Thr Ser Ala Ala Thr Thr Ser Asp Leu Asn Ser Arg Gln Tyr
               420                 425                 430
   Pro Pro Leu Thr Ser Asn Gln Tyr Lys Arg Ser Ala Ala Phe Phe Gly
           435                 440                 445
   Asp Phe Thr Phe Gln Ala Gln Arg Arg Thr Tyr Leu Lys Ala Ala Thr
       450                 455                 460
   Leu Gly Trp Lys Lys Asn Lys Ala Lys Val Trp Ser Tyr Glu Phe Gln
   465                 470                 475                 480
   Gln Asn Asp Lys Phe Ala Asn Gly Thr Gly Ser Leu Leu Gly Pro Tyr
                   485                 490                 495
   His Gly Ala Asp Val Lys Tyr Tyr Phe Ile Arg Pro Asp Gly Arg Gln
               500                 505                 510
   Lys Asp Pro Val Leu Ala Asp Arg Met Pro Arg Ala Tyr Ile Ser Phe
           515                 520                 525
   Val Tyr His His Asp Pro Thr Val Leu Gly Gly Phe Glu Trp Pro Pro
       530                 535                 540
   Tyr Gly Lys Gly Lys Lys Leu Leu Gln Met Lys Gly Asp Asn Thr Thr
   545                 550                 555                 560
   Val Ile Glu Asp Ala Tyr Arg Lys Glu Ala Met Asp Ala Leu Thr Asn
                   565                 570                 575
   Arg Lys Ala Ala Lys Val Phe Gly Phe
               580                 585
<210> 18
   <211> 1758
   <212> DNA
   <213> Ustilago maydis
<220>
   <223> Encoding full-length incl. native signal sequence and P492L
<400> 18
   atgaggctgc ctaatctgac actgctcgta tgggcagcca catccgtcgg cttggtgtcg      60
   gctcttccac aagtctctta caaagctgac gccaccgcct ctgctcccac agtcaaactc     120
   caccaaggta ctgtgcgcgg cttggccgac gacaattatg gcctggaaca attctttggc     180
   attccctatg ccaagccacc cgtcggctcg ctccgatttg ccaagccaca gcctctcggc     240
   ccagcctcgt cccacaaaac ggtcatcgac gctactcgat tcggcgacat ctgtatgcag     300
   actgttgcac cctcgcctct ctataacatg agcgaagact gtctcaatct caatgtcgtt     360
   cgtcccaaag gaacaacggc caaggacaag cttccggtgc tggtttggat ctatggtggg     420
   gcgttcaggc aaggctcaac gcccatttac aacgccagcg aattggtgca gaagagcgtc     480
   gagattggca agccgattgt atttgttgcg atcagctatc gagtgggtcc attcggcttc     540
   atcggtggat ccgagattgc tgacagtgac tcggctacca gcaacgcagg gctgtatgat     600
   cagagattgg gactcaagtg gattcgccac aacattggca agtttggagg tgacaagaac     660
   agggtcactc tgttcggtca gagtgcgggc gcgatgagca ttgctctgca gaactttgcc     720
   tatgatggta acaaccacgg gctttggcat gccgcgatca tgaactcggg cggaattgct     780
   ccgggtccac tgttaacgcc caaacaccct acggtggaac agagcttcaa gaggctggcc     840
   aatggcgttg gatgtacagg aggatcactg ctccggtgtc tgcgtaaagc aaacgcgagt     900
   gaggttcaga ccgtggccag taatctgact gctcaagccg gcggaacatt tcccattcct     960
   ggagcgcttg cgtggttgcc actggtcgac tacgagctca tcaccaacta tcccagtgtc    1020
   aaccttccgc aaggtaaact ggccgacatc ccggtgatcc aaggaaacgc tctggacgaa    1080
   ggaacatctt ttgcccagaa acagctcaac agcagcgccg atttcgaacg gtgggtcaga    1140
   agcgctgctg tcatccacaa cacttcgtac accgagcaag cgcttcaaaa ggtctttgaa    1200
   ctctacccag atgttcctga acagggctcg ccattctaca atgcggagac ggccacttct    1260
   gctgctacca cgtcggacct caattcgcgc cagtatccac ctttgaccag caatcagtac    1320
   aagcgttcag cggcgttctt tggcgacttc actttccagg cgcagcgacg aacttatctc    1380
   aaggcggcaa ccttgggctg gaagaagaac aaggccaaag tatggtccta cgagttccag    1440
   cagaacgaca agtttgctaa tggtactgga tcgctgttgg gcccatacca tggtgctgat    1500
   gtcaagtact attttattcg acctgatgga aggcagaagg accccgtctt ggccgataga    1560
   atgcctaggg cgtatatcag tttcgtgtat caccatgatc ccacggtgct cggtggtttc    1620
   gaatggccgc cctacggaaa aggcaagaag ctgctgcaga tgaaaggtga caacacgaca    1680
   gtgatcgagg atgcctatag gaaggaggcg atggatgcgc tgaccaacag gaaagctgcc    1740
   aaggtgtttg gcttctaa                                                  1758
<210> 19
   <211> 585
   <212> PRT
   <213> Ustilago maydis
<220>
   <223> NCBI Reference Sequence: XM_751236.1
<400> 19
   Met Arg Leu Pro Asn Leu Thr Leu Leu Val Trp Ala Ala Thr Ser Val
   1               5                   10                  15
   Gly Leu Val Ser Ala Leu Pro Gln Val Ser Tyr Lys Ala Asp Ala Thr
               20                  25                  30
   Ala Ser Ala Pro Thr Val Lys Leu His Gln Gly Thr Val Arg Gly Leu
           35                  40                  45
   Ala Asp Asp Asn Tyr Gly Leu Glu Gln Phe Phe Gly Ile Pro Tyr Ala
       50                  55                  60
   Lys Pro Pro Val Gly Ser Leu Arg Phe Ala Lys Pro Gln Pro Leu Gly
   65                  70                  75                  80
   Pro Ala Ser Ser His Lys Thr Val Ile Asp Ala Thr Arg Phe Gly Asp
                   85                  90                  95
   Ile Cys Met Gln Thr Val Ala Pro Ser Pro Leu Tyr Asn Met Ser Glu
               100                 105                 110
   Asp Cys Leu Asn Leu Asn Val Val Arg Pro Lys Gly Thr Thr Ala Lys
           115                 120                 125
   Asp Lys Leu Pro Val Leu Val Trp Ile Tyr Gly Gly Ala Phe Arg Gln
       130                 135                 140
   Gly Ser Thr Pro Ile Tyr Asn Ala Ser Glu Leu Val Gln Lys Ser Val
   145                 150                 155                 160
   Glu Ile Gly Lys Pro Ile Val Phe Val Ala Ile Ser Tyr Arg Val Gly
                   165                 170                 175
   Pro Phe Gly Phe Ile Gly Gly Ser Glu Ile Ala Asp Ser Asp Ser Ala
               180                 185                 190
   Thr Ser Asn Ala Gly Leu Tyr Asp Gln Arg Leu Gly Leu Lys Trp Ile
           195                 200                 205
   Arg His Asn Ile Gly Lys Phe Gly Gly Asp Lys Asn Arg Val Thr Leu
       210                 215                 220
   Phe Gly Gln Ser Ala Gly Ala Met Ser Ile Ala Leu Gln Asn Phe Ala
   225                 230                 235                 240
   Tyr Asp Gly Asn Asn His Gly Leu Trp His Ala Ala Ile Met Asn Ser
                   245                 250                 255
   Gly Gly Ile Ala Pro Gly Pro Leu Leu Thr Pro Lys His Pro Thr Val
               260                 265                 270
   Glu Gln Ser Phe Lys Arg Leu Ala Asn Gly Val Gly Cys Thr Gly Gly
           275                 280                 285
   Ser Leu Leu Arg Cys Leu Arg Lys Ala Asn Ala Ser Glu Val Gln Thr
       290                 295                 300
   Val Ala Ser Asn Leu Thr Ala Gln Ala Gly Gly Thr Phe Pro Ile Pro
   305                 310                 315                 320
   Gly Ala Leu Ala Trp Leu Pro Leu Val Asp Tyr Glu Leu Ile Thr Asn
                   325                 330                 335
   Tyr Pro Ser Val Asn Leu Pro Gln Gly Lys Leu Ala Asp Ile Pro Val
               340                 345                 350
   Ile Gln Gly Asn Ala Leu Asp Glu Gly Thr Ser Phe Ala Gln Lys Gln
           355                 360                 365
   Leu Asn Ser Ser Ala Asp Phe Glu Arg Trp Val Arg Ser Ala Ala Val
       370                 375                 380
   Ile His Asn Thr Ser Tyr Thr Glu Gln Ala Leu Gln Lys Val Phe Glu
   385                 390                 395                 400
   Leu Tyr Pro Asp Val Pro Glu Gln Gly Ser Pro Phe Tyr Asn Ala Glu
                   405                 410                 415
   Thr Ala Thr Ser Ala Ala Thr Thr Ser Asp Leu Asn Ser Arg Gln Tyr
               420                 425                 430
   Pro Pro Leu Thr Ser Asn Gln Tyr Lys Arg Ser Ala Ala Phe Phe Gly
           435                 440                 445
   Asp Phe Thr Phe Gln Ala Gln Arg Arg Thr Tyr Leu Lys Ala Ala Thr
       450                 455                 460
   Leu Gly Trp Lys Lys Asn Lys Ala Lys Val Trp Ser Tyr Glu Phe Gln
   465                 470                 475                 480
   Gln Asn Asp Lys Phe Ala Asn Gly Thr Gly Ser Pro Leu Gly Pro Tyr
                   485                 490                 495
   His Gly Ala Asp Val Lys Tyr Tyr Phe Ile Arg Pro Asp Gly Arg Gln
               500                 505                 510
   Lys Asp Pro Val Leu Ala Asp Arg Met Pro Arg Ala Tyr Ile Ser Phe
           515                 520                 525
   Val Tyr His His Asp Pro Thr Val Leu Gly Gly Phe Glu Trp Pro Pro
       530                 535                 540
   Tyr Gly Lys Gly Lys Lys Leu Leu Gln Met Lys Gly Asp Asn Thr Thr
   545                 550                 555                 560
   Val Ile Glu Asp Ala Tyr Arg Lys Glu Ala Met Asp Ala Leu Thr Asn
                   565                 570                 575
   Arg Lys Ala Ala Lys Val Phe Gly Phe
               580                 585
<210> 20
   <211> 1578
   <212> DNA
   <213> Rhizoctonia solani
<400> 20
   ctggactgtg cggggttatt caagaaatcg cagacgctta tcaaagggat taatccattt      60
   gttgctgagg tccacccagc aaatgtaacc tttgttccgg tggggaacat agcttaccct     120
   gacccggtac ccgacctacc ggagttttgc cgtttcggtg cagaatacaa cacatcatct     180
   aaatccaagt ttagatttga agtatggcta ccaacgtcaa aaaagtggaa cggtcgtttt     240
   gcattcattg gcaacggtgg ggatgctggc ggtgtgaatt atgctgatat ggccattcca     300
   ttggcgaaat atgggtttgt ggtcgcgagc accaacaccg gtcacgatgg aacccacggt     360
   gacggaacat ttgcgatttc taatcccgag tcacagattg atttcgggca ccgagctgta     420
   catctcagta ccgtgttcgc aaagacaatc gcaaaatcat actatggaaa gccatctacg     480
   tataattact ggattggatg ctctagcgga ggaaaacaag gactgaaatc ggcccaagca     540
   tatcccgcgg actttgatgg ggttatcgca ggtgcaccgg ctcaatggtg gcctcatctt     600
   aatggcttca ctacccatgt gaatatgttg aatgccaaag ctaccactcc gggggcgacg     660
   attcctacct cattttttac ggcgttgaat caggaggtag ttgcccaatg tggaaagctg     720
   gatggagtcg acgatgggat tatcacaaac cctaggttgt gcaagcccga tcttactcgt     780
   gttgcatgcg gatcaaccaa tgcctctcca tatgtcaatt caagtaattg tttatcgaaa     840
   cttcagctcg taacgctcaa agctatttac accaactgga catcgagcaa tggagagttc     900
   ctgttcccta ccttcgagcc cggaagcgaa tttgggtggc caaataccgt caatggggtt     960
   ccatatggac ctggacccga ctacttctta tatcaggtat acaacaagac atccgttcag    1020
   aagttgcaaa tcaatgaggc cgagcttcaa agactggtcg caatagcgga tgtaaccaac    1080
   cctggtcaaa gcagagctat cgaccccaac ttgaatccat tcttcaaacg tggtggaaaa    1140
   ctgttgcagt atcatgggtt cgcagatcct cttatcccgt ctgggagctc cctgtggtac    1200
   tacgaacacg ttcggagtta cttcaagaac gcagacttaa gcgatcgata ccgtttgttc    1260
   atgatcccag gtgttgctca ttgctctgga gggccaggtg cagatgcgtt cggacgaccc    1320
   ggccaacgta gcgtttccct aggcggcgca ggccaatccc tatcatttac tccgcagtac    1380
   gacatgatcc ttgctactat caactgggta gagaagggag tggccccgaa gtcactcatt    1440
   gggacgaagt acaaaaataa caatattaga gaaggcgcaa aattcacccg gctattctgt    1500
   ccttacccgc aagaagcaat ctaccgcggc gggaacgtga atgtagctag cagttattcg    1560
   tgcggtgtcc gcgcttga                                                  1578
<210> 21
   <211> 1692
   <212> DNA
   <213> Rhizoctonia solani
<400> 21
   atgggctgcg atcgacctcg acaagctctt cattcccttc aagagttcac ggtcatgttc      60
   tctaagtggc tacctatctc ggcgatttta ctatatggtg cttccgcaaa agcactggac     120
   tgtgcggggt tattcaagaa atcgcagacg cttatcaaag ggattaatcc atttgttgct     180
   gaggtccacc cagcaaatgt aacctttgtt ccggtgggga acatagctta ccctgacccg     240
   gtacccgacc taccggagtt ttgccgtttc ggtgcagaat acaacacatc atctaaatcc     300
   aagtttagat ttgaagtatg gctaccaacg tcaaaaaagt ggaacggtcg ttttgcattc     360
   attggcaacg gtggggatgc tggcggtgtg aattatgctg atatggccat tccattggcg     420
   aaatatgggt ttgtggtcgc gagcaccaac accggtcacg atggaaccca cggtgacgga     480
   acatttgcga tttctaatcc cgagtcacag attgatttcg ggcaccgagc tgtacatctc     540
   agtaccgtgt tcgcaaagac aatcgcaaaa tcatactatg gaaagccatc tacgtataat     600
   tactggattg gatgctctag cggaggaaaa caaggactga aatcggccca agcatatccc     660
   gcggactttg atggggttat cgcaggtgca ccggctcaat ggtggcctca tcttaatggc     720
   ttcactaccc atgtgaatat gttgaatgcc aaagctacca ctccgggggc gacgattcct     780
   acctcatttt ttacggcgtt gaatcaggag gtagttgccc aatgtggaaa gctggatgga     840
   gtcgacgatg ggattatcac aaaccctagg ttgtgcaagc ccgatcttac tcgtgttgca     900
   tgcggatcaa ccaatgcctc tccatatgtc aattcaagta attgtttatc gaaacttcag     960
   ctcgtaacgc tcaaagctat ttacaccaac tggacatcga gcaatggaga gttcctgttc    1020
   cctaccttcg agcccggaag cgaatttggg tggccaaata ccgtcaatgg ggttccatat    1080
   ggacctggac ccgactactt cttatatcag gtatacaaca agacatccgt tcagaagttg    1140
   caaatcaatg aggccgagct tcaaagactg gtcgcaatag cggatgtaac caaccctggt    1200
   caaagcagag ctatcgaccc caacttgaat ccattcttca aacgtggtgg aaaactgttg    1260
   cagtatcatg ggttcgcaga tcctcttatc ccgtctggga gctccctgtg gtactacgaa    1320
   cacgttcgga gttacttcaa gaacgcagac ttaagcgatc gataccgttt gttcatgatc    1380
   ccaggtgttg ctcattgctc tggagggcca ggtgcagatg cgttcggacg acccggccaa    1440
   cgtagcgttt ccctaggcgg cgcaggccaa tccctatcat ttactccgca gtacgacatg    1500
   atccttgcta ctatcaactg ggtagagaag ggagtggccc cgaagtcact cattgggacg    1560
   aagtacaaaa ataacaatat tagagaaggc gcaaaattca cccggctatt ctgtccttac    1620
   ccgcaagaag caatctaccg cggcgggaac gtgaatgtag ctagcagtta ttcgtgcggt    1680
   gtccgcgctt ga                                                        1692
<210> 22
   <211> 525
   <212> PRT
   <213> Rhizoctonia solani
<400> 22
   Leu Asp Cys Ala Gly Leu Phe Lys Lys Ser Gln Thr Leu Ile Lys Gly
   1               5                   10                  15
   Ile Asn Pro Phe Val Ala Glu Val His Pro Ala Asn Val Thr Phe Val
               20                  25                  30
   Pro Val Gly Asn Ile Ala Tyr Pro Asp Pro Val Pro Asp Leu Pro Glu
           35                  40                  45
   Phe Cys Arg Phe Gly Ala Glu Tyr Asn Thr Ser Ser Lys Ser Lys Phe
       50                  55                  60
   Arg Phe Glu Val Trp Leu Pro Thr Ser Lys Lys Trp Asn Gly Arg Phe
   65                  70                  75                  80
   Ala Phe Ile Gly Asn Gly Gly Asp Ala Gly Gly Val Asn Tyr Ala Asp
                   85                  90                  95
   Met Ala Ile Pro Leu Ala Lys Tyr Gly Phe Val Val Ala Ser Thr Asn
               100                 105                 110
   Thr Gly His Asp Gly Thr His Gly Asp Gly Thr Phe Ala Ile Ser Asn
           115                 120                 125
   Pro Glu Ser Gln Ile Asp Phe Gly His Arg Ala Val His Leu Ser Thr
       130                 135                 140
   Val Phe Ala Lys Thr Ile Ala Lys Ser Tyr Tyr Gly Lys Pro Ser Thr
   145                 150                 155                 160
   Tyr Asn Tyr Trp Ile Gly Cys Ser Ser Gly Gly Lys Gln Gly Leu Lys
                   165                 170                 175
   Ser Ala Gln Ala Tyr Pro Ala Asp Phe Asp Gly Val Ile Ala Gly Ala
               180                 185                 190
   Pro Ala Gln Trp Trp Pro His Leu Asn Gly Phe Thr Thr His Val Asn
           195                 200                 205
   Met Leu Asn Ala Lys Ala Thr Thr Pro Gly Ala Thr Ile Pro Thr Ser
       210                 215                 220
   Phe Phe Thr Ala Leu Asn Gln Glu Val Val Ala Gln Cys Gly Lys Leu
   225                 230                 235                 240
   Asp Gly Val Asp Asp Gly Ile Ile Thr Asn Pro Arg Leu Cys Lys Pro
                   245                 250                 255
   Asp Leu Thr Arg Val Ala Cys Gly Ser Thr Asn Ala Ser Pro Tyr Val
               260                 265                 270
   Asn Ser Ser Asn Cys Leu Ser Lys Leu Gln Leu Val Thr Leu Lys Ala
           275                 280                 285
   Ile Tyr Thr Asn Trp Thr Ser Ser Asn Gly Glu Phe Leu Phe Pro Thr
       290                 295                 300
   Phe Glu Pro Gly Ser Glu Phe Gly Trp Pro Asn Thr Val Asn Gly Val
   305                 310                 315                 320
   Pro Tyr Gly Pro Gly Pro Asp Tyr Phe Leu Tyr Gln Val Tyr Asn Lys
                   325                 330                 335
   Thr Ser Val Gln Lys Leu Gln Ile Asn Glu Ala Glu Leu Gln Arg Leu
               340                 345                 350
   Val Ala Ile Ala Asp Val Thr Asn Pro Gly Gln Ser Arg Ala Ile Asp
           355                 360                 365
   Pro Asn Leu Asn Pro Phe Phe Lys Arg Gly Gly Lys Leu Leu Gln Tyr
       370                 375                 380
   His Gly Phe Ala Asp Pro Leu Ile Pro Ser Gly Ser Ser Leu Trp Tyr
   385                 390                 395                 400
   Tyr Glu His Val Arg Ser Tyr Phe Lys Asn Ala Asp Leu Ser Asp Arg
                   405                 410                 415
   Tyr Arg Leu Phe Met Ile Pro Gly Val Ala His Cys Ser Gly Gly Pro
               420                 425                 430
   Gly Ala Asp Ala Phe Gly Arg Pro Gly Gln Arg Ser Val Ser Leu Gly
           435                 440                 445
   Gly Ala Gly Gln Ser Leu Ser Phe Thr Pro Gln Tyr Asp Met Ile Leu
       450                 455                 460
   Ala Thr Ile Asn Trp Val Glu Lys Gly Val Ala Pro Lys Ser Leu Ile
   465                 470                 475                 480
   Gly Thr Lys Tyr Lys Asn Asn Asn Ile Arg Glu Gly Ala Lys Phe Thr
                   485                 490                 495
   Arg Leu Phe Cys Pro Tyr Pro Gln Glu Ala Ile Tyr Arg Gly Gly Asn
               500                 505                 510
   Val Asn Val Ala Ser Ser Tyr Ser Cys Gly Val Arg Ala
           515                 520                 525
<210> 23
   <211> 563
   <212> PRT
   <213> Rhizoctonia solani
<400> 23
   Met Gly Cys Asp Arg Pro Arg Gln Ala Leu His Ser Leu Gln Glu Phe
   1               5                   10                  15
   Thr Val Met Phe Ser Lys Trp Leu Pro Ile Ser Ala Ile Leu Leu Tyr
               20                  25                  30
   Gly Ala Ser Ala Lys Ala Leu Asp Cys Ala Gly Leu Phe Lys Lys Ser
           35                  40                  45
   Gln Thr Leu Ile Lys Gly Ile Asn Pro Phe Val Ala Glu Val His Pro
       50                  55                  60
   Ala Asn Val Thr Phe Val Pro Val Gly Asn Ile Ala Tyr Pro Asp Pro
   65                  70                  75                  80
   Val Pro Asp Leu Pro Glu Phe Cys Arg Phe Gly Ala Glu Tyr Asn Thr
                   85                  90                  95
   Ser Ser Lys Ser Lys Phe Arg Phe Glu Val Trp Leu Pro Thr Ser Lys
               100                 105                 110
   Lys Trp Asn Gly Arg Phe Ala Phe Ile Gly Asn Gly Gly Asp Ala Gly
           115                 120                 125
   Gly Val Asn Tyr Ala Asp Met Ala Ile Pro Leu Ala Lys Tyr Gly Phe
       130                 135                 140
   Val Val Ala Ser Thr Asn Thr Gly His Asp Gly Thr His Gly Asp Gly
   145                 150                 155                 160
   Thr Phe Ala Ile Ser Asn Pro Glu Ser Gln Ile Asp Phe Gly His Arg
                   165                 170                 175
   Ala Val His Leu Ser Thr Val Phe Ala Lys Thr Ile Ala Lys Ser Tyr
               180                 185                 190
   Tyr Gly Lys Pro Ser Thr Tyr Asn Tyr Trp Ile Gly Cys Ser Ser Gly
           195                 200                 205
   Gly Lys Gln Gly Leu Lys Ser Ala Gln Ala Tyr Pro Ala Asp Phe Asp
       210                 215                 220
   Gly Val Ile Ala Gly Ala Pro Ala Gln Trp Trp Pro His Leu Asn Gly
   225                 230                 235                 240
   Phe Thr Thr His Val Asn Met Leu Asn Ala Lys Ala Thr Thr Pro Gly
                   245                 250                 255
   Ala Thr Ile Pro Thr Ser Phe Phe Thr Ala Leu Asn Gln Glu Val Val
               260                 265                 270
   Ala Gln Cys Gly Lys Leu Asp Gly Val Asp Asp Gly Ile Ile Thr Asn
           275                 280                 285
   Pro Arg Leu Cys Lys Pro Asp Leu Thr Arg Val Ala Cys Gly Ser Thr
       290                 295                 300
   Asn Ala Ser Pro Tyr Val Asn Ser Ser Asn Cys Leu Ser Lys Leu Gln
   305                 310                 315                 320
   Leu Val Thr Leu Lys Ala Ile Tyr Thr Asn Trp Thr Ser Ser Asn Gly
                   325                 330                 335
   Glu Phe Leu Phe Pro Thr Phe Glu Pro Gly Ser Glu Phe Gly Trp Pro
               340                 345                 350
   Asn Thr Val Asn Gly Val Pro Tyr Gly Pro Gly Pro Asp Tyr Phe Leu
           355                 360                 365
   Tyr Gln Val Tyr Asn Lys Thr Ser Val Gln Lys Leu Gln Ile Asn Glu
       370                 375                 380
   Ala Glu Leu Gln Arg Leu Val Ala Ile Ala Asp Val Thr Asn Pro Gly
   385                 390                 395                 400
   Gln Ser Arg Ala Ile Asp Pro Asn Leu Asn Pro Phe Phe Lys Arg Gly
                   405                 410                 415
   Gly Lys Leu Leu Gln Tyr His Gly Phe Ala Asp Pro Leu Ile Pro Ser
               420                 425                 430
   Gly Ser Ser Leu Trp Tyr Tyr Glu His Val Arg Ser Tyr Phe Lys Asn
           435                 440                 445
   Ala Asp Leu Ser Asp Arg Tyr Arg Leu Phe Met Ile Pro Gly Val Ala
       450                 455                 460
   His Cys Ser Gly Gly Pro Gly Ala Asp Ala Phe Gly Arg Pro Gly Gln
   465                 470                 475                 480
   Arg Ser Val Ser Leu Gly Gly Ala Gly Gln Ser Leu Ser Phe Thr Pro
                   485                 490                 495
   Gln Tyr Asp Met Ile Leu Ala Thr Ile Asn Trp Val Glu Lys Gly Val
               500                 505                 510
   Ala Pro Lys Ser Leu Ile Gly Thr Lys Tyr Lys Asn Asn Asn Ile Arg
           515                 520                 525
   Glu Gly Ala Lys Phe Thr Arg Leu Phe Cys Pro Tyr Pro Gln Glu Ala
       530                 535                 540
   Ile Tyr Arg Gly Gly Asn Val Asn Val Ala Ser Ser Tyr Ser Cys Gly
   545                 550                 555                 560
   Val Arg Ala
<210> 24
   <211> 1500
   <212> DNA
   <213> Schizophyllum commune
<400> 24
   caaggcgact tctccgccaa ctgctcctcc ttcgtcgatg cgatcaccct cgagaacgtc 60
   accgtgcagt cgacggagtt cgtcgcggct ggcacgaacg tcagcgtgta cgttcccgag 120
   tcgtgcatgt cggcctcgta ccaggtcgtc agcgctgacc tttgccgcgc cacgatgaac 180
   gtctcgactt cggaccgcag cggtatccgc ctcgaggcgt ggttcccgca gaactatacc 240
   ggtcggttct tgagcacggg caacggcgga attggaggat gcatccagta cagcgacctc 300
   gactacgccg cctccctcgg cttcgccgcc gtcggcgcca acaacggcca cgacggcatg 360
   accggcgagc ccttcctcaa caaccccgac gtcatcaccg acttcgcctg gcgctccctc 420
   cataccggcg tcgtcgtcgg caagcagctc gtcgagacct tctacggcgc cccgcacagc 480
   aagtcgtact acctcggctg ctcgaccggc gggcggcagg ggtggaagat ggtgcaggac 540
   ttcccggagg actttgatgg cgtagtggcg ggcgcgccgg cgatggcgtt caatgcgctg 600
   ttgtattgga gcgggtcgtt ctatacggtc actgggacct cggacgaccc aaccttcgtg 660
   ccggtcgact tttgggcgaa caatatccac caggcggtcc ttgatcagtg cgacgcgctc 720
   gacggggcga ccgacggtgt attggagaat ccggatctct gcgacttcga cgtgacgcct 780
   ctcaactgcg cggacggcaa cacgaccgac tgcttgaccg agacgcaggc ggagaccgtg 840
   cgcacggtct tgagcgctat ctatgacgcg aacggcacgc tcgtgtaccc gcgcctgcag 900
   ccgggcgcgg aggtcttggc ggggcagcag cagttcaacg gccagccttt cgacatcact 960
   caggattggt accgctacgt catctacaac gactcgacct gggatcctgc gacgctgaac 1020
   ctcggcgact acgcagtcgc cctcgcgcag aatcccgcca acatcgagac atttaacggc 1080
   gacatctccg ctttcgaggc cgcaggaggc aaggtcctgc actaccacgg cctcatggac 1140
   ggtctcatct cgtccgacaa ctccaagcgg tactacaagc ttgtgcagga gacgatgggc 1200
   aaggactcgt ccgagctcga cgacttctac cgcttcttcc cgatcagcgg catgtcgcac 1260
   tgcagcgggg gcgacggggc gtataggatt gggaatgttg atggcggagc ggggacgagt 1320
   gcggatgaca atgtgctgat gtcgatggtg cggtgggtgg aggagggcgt ggcgccggaa 1380
   gtcgtgcgcg gggcgaatgc taacgtgacc tactggcgtt cgcattgcaa gtggcccctg 1440
   acgaacaagt acgttggacc tggatcgtat gaggatgagt ctgcatggca gtgcagctga 1500
<210> 25
   <211> 1572
   <212> DNA
   <213> Schizophyllum commune
<400> 25
   atggctccta cagtgcgctc tctcctcagc gcgctcggca gcgtgctcgc actcgcgcgc      60
   ttcacgaatg cccaaggcga cttctccgcc aactgctcct ccttcgtcga tgcgatcacc     120
   ctcgagaacg tcaccgtgca gtcgacggag ttcgtcgcgg ctggcacgaa cgtcagcgtg     180
   tacgttcccg agtcgtgcat gtcggcctcg taccaggtcg tcagcgctga cctttgccgc     240
   gccacgatga acgtctcgac ttcggaccgc agcggtatcc gcctcgaggc gtggttcccg     300
   cagaactata ccggtcggtt cttgagcacg ggcaacggcg gaattggagg atgcatccag     360
   tacagcgacc tcgactacgc cgcctccctc ggcttcgccg ccgtcggcgc caacaacggc     420
   cacgacggca tgaccggcga gcccttcctc aacaaccccg acgtcatcac cgacttcgcc     480
   tggcgctccc tccataccgg cgtcgtcgtc ggcaagcagc tcgtcgagac cttctacggc     540
   gccccgcaca gcaagtcgta ctacctcggc tgctcgaccg gcgggcggca ggggtggaag     600
   atggtgcagg acttcccgga ggactttgat ggcgtagtgg cgggcgcgcc ggcgatggcg     660
   ttcaatgcgc tgttgtattg gagcgggtcg ttctatacgg tcactgggac ctcggacgac     720
   ccaaccttcg tgccggtcga cttttgggcg aacaatatcc accaggcggt ccttgatcag     780
   tgcgacgcgc tcgacggggc gaccgacggt gtattggaga atccggatct ctgcgacttc     840
   gacgtgacgc ctctcaactg cgcggacggc aacacgaccg actgcttgac cgagacgcag     900
   gcggagaccg tgcgcacggt cttgagcgct atctatgacg cgaacggcac gctcgtgtac     960
   ccgcgcctgc agccgggcgc ggaggtcttg gcggggcagc agcagttcaa cggccagcct    1020
   ttcgacatca ctcaggattg gtaccgctac gtcatctaca acgactcgac ctgggatcct    1080
   gcgacgctga acctcggcga ctacgcagtc gccctcgcgc agaatcccgc caacatcgag    1140
   acatttaacg gcgacatctc cgctttcgag gccgcaggag gcaaggtcct gcactaccac    1200
   ggcctcatgg acggtctcat ctcgtccgac aactccaagc ggtactacaa gcttgtgcag    1260
   gagacgatgg gcaaggactc gtccgagctc gacgacttct accgcttctt cccgatcagc    1320
   ggcatgtcgc actgcagcgg gggcgacggg gcgtatagga ttgggaatgt tgatggcgga    1380
   gcggggacga gtgcggatga caatgtgctg atgtcgatgg tgcggtgggt ggaggagggc    1440
   gtggcgccgg aagtcgtgcg cggggcgaat gctaacgtga cctactggcg ttcgcattgc    1500
   aagtggcccc tgacgaacaa gtacgttgga cctggatcgt atgaggatga gtctgcatgg    1560
   cagtgcagct ga                                                        1572
<210> 26
   <211> 499
   <212> PRT
   <213> Schizophyllum commune
<400> 26
   Gln Gly Asp Phe Ser Ala Asn Cys Ser Ser Phe Val Asp Ala Ile Thr
   1               5                   10                  15
   Leu Glu Asn Val Thr Val Gln Ser Thr Glu Phe Val Ala Ala Gly Thr
               20                  25                  30
   Asn Val Ser Val Tyr Val Pro Glu Ser Cys Met Ser Ala Ser Tyr Gln
           35                  40                  45
   Val Val Ser Ala Asp Leu Cys Arg Ala Thr Met Asn Val Ser Thr Ser
       50                  55                  60
   Asp Arg Ser Gly Ile Arg Leu Glu Ala Trp Phe Pro Gln Asn Tyr Thr
   65                  70                  75                  80
   Gly Arg Phe Leu Ser Thr Gly Asn Gly Gly Ile Gly Gly Cys Ile Gln
                   85                  90                  95
   Tyr Ser Asp Leu Asp Tyr Ala Ala Ser Leu Gly Phe Ala Ala Val Gly
               100                 105                 110
   Ala Asn Asn Gly His Asp Gly Met Thr Gly Glu Pro Phe Leu Asn Asn
           115                 120                 125
   Pro Asp Val Ile Thr Asp Phe Ala Trp Arg Ser Leu His Thr Gly Val
       130                 135                 140
   Val Val Gly Lys Gln Leu Val Glu Thr Phe Tyr Gly Ala Pro His Ser
   145                 150                 155                 160
   Lys Ser Tyr Tyr Leu Gly Cys Ser Thr Gly Gly Arg Gln Gly Trp Lys
                   165                 170                 175
   Met Val Gln Asp Phe Pro Glu Asp Phe Asp Gly Val Val Ala Gly Ala
               180                 185                 190
   Pro Ala Met Ala Phe Asn Ala Leu Leu Tyr Trp Ser Gly Ser Phe Tyr
           195                 200                 205
   Thr Val Thr Gly Thr Ser Asp Asp Pro Thr Phe Val Pro Val Asp Phe
       210                 215                 220
   Trp Ala Asn Asn Ile His Gln Ala Val Leu Asp Gln Cys Asp Ala Leu
   225                 230                 235                 240
   Asp Gly Ala Thr Asp Gly Val Leu Glu Asn Pro Asp Leu Cys Asp Phe
                   245                 250                 255
   Asp Val Thr Pro Leu Asn Cys Ala Asp Gly Asn Thr Thr Asp Cys Leu
               260                 265                 270
   Thr Glu Thr Gln Ala Glu Thr Val Arg Thr Val Leu Ser Ala Ile Tyr
           275                 280                 285
   Asp Ala Asn Gly Thr Leu Val Tyr Pro Arg Leu Gln Pro Gly Ala Glu
       290                 295                 300
   Val Leu Ala Gly Gln Gln Gln Phe Asn Gly Gln Pro Phe Asp Ile Thr
   305                 310                 315                 320
   Gln Asp Trp Tyr Arg Tyr Val Ile Tyr Asn Asp Ser Thr Trp Asp Pro
                   325                 330                 335
   Ala Thr Leu Asn Leu Gly Asp Tyr Ala Val Ala Leu Ala Gln Asn Pro
               340                 345                 350
   Ala Asn Ile Glu Thr Phe Asn Gly Asp Ile Ser Ala Phe Glu Ala Ala
           355                 360                 365
   Gly Gly Lys Val Leu His Tyr His Gly Leu Met Asp Gly Leu Ile Ser
       370                 375                 380
   Ser Asp Asn Ser Lys Arg Tyr Tyr Lys Leu Val Gln Glu Thr Met Gly
   385                 390                 395                 400
   Lys Asp Ser Ser Glu Leu Asp Asp Phe Tyr Arg Phe Phe Pro Ile Ser
                   405                 410                 415
   Gly Met Ser His Cys Ser Gly Gly Asp Gly Ala Tyr Arg Ile Gly Asn
               420                 425                 430
   Val Asp Gly Gly Ala Gly Thr Ser Ala Asp Asp Asn Val Leu Met Ser
           435                 440                 445
   Met Val Arg Trp Val Glu Glu Gly Val Ala Pro Glu Val Val Arg Gly
       450                 455                 460
   Ala Asn Ala Asn Val Thr Tyr Trp Arg Ser His Cys Lys Trp Pro Leu
   465                 470                 475                 480
   Thr Asn Lys Tyr Val Gly Pro Gly Ser Tyr Glu Asp Glu Ser Ala Trp
                   485                 490                 495
   Gln Cys Ser
<210> 27
   <211> 523
   <212> PRT
   <213> Schizophyllum commune
<400> 27
   Met Ala Pro Thr Val Arg Ser Leu Leu Ser Ala Leu Gly Ser Val Leu
   1               5                   10                  15
   Ala Leu Ala Arg Phe Thr Asn Ala Gln Gly Asp Phe Ser Ala Asn Cys
               20                  25                  30
   Ser Ser Phe Val Asp Ala Ile Thr Leu Glu Asn Val Thr Val Gln Ser
           35                  40                  45
   Thr Glu Phe Val Ala Ala Gly Thr Asn Val Ser Val Tyr Val Pro Glu
       50                  55                  60
   Ser Cys Met Ser Ala Ser Tyr Gln Val Val Ser Ala Asp Leu Cys Arg
   65                  70                  75                  80
   Ala Thr Met Asn Val Ser Thr Ser Asp Arg Ser Gly Ile Arg Leu Glu
                   85                  90                  95
   Ala Trp Phe Pro Gln Asn Tyr Thr Gly Arg Phe Leu Ser Thr Gly Asn
               100                 105                 110
   Gly Gly Ile Gly Gly Cys Ile Gln Tyr Ser Asp Leu Asp Tyr Ala Ala
           115                 120                 125
   Ser Leu Gly Phe Ala Ala Val Gly Ala Asn Asn Gly His Asp Gly Met
       130                 135                 140
   Thr Gly Glu Pro Phe Leu Asn Asn Pro Asp Val Ile Thr Asp Phe Ala
   145                 150                 155                 160
   Trp Arg Ser Leu His Thr Gly Val Val Val Gly Lys Gln Leu Val Glu
                   165                 170                 175
   Thr Phe Tyr Gly Ala Pro His Ser Lys Ser Tyr Tyr Leu Gly Cys Ser
               180                 185                 190
   Thr Gly Gly Arg Gln Gly Trp Lys Met Val Gln Asp Phe Pro Glu Asp
           195                 200                 205
   Phe Asp Gly Val Val Ala Gly Ala Pro Ala Met Ala Phe Asn Ala Leu
       210                 215                 220
   Leu Tyr Trp Ser Gly Ser Phe Tyr Thr Val Thr Gly Thr Ser Asp Asp
   225                 230                 235                 240
   Pro Thr Phe Val Pro Val Asp Phe Trp Ala Asn Asn Ile His Gln Ala
                   245                 250                 255
   Val Leu Asp Gln Cys Asp Ala Leu Asp Gly Ala Thr Asp Gly Val Leu
               260                 265                 270
   Glu Asn Pro Asp Leu Cys Asp Phe Asp Val Thr Pro Leu Asn Cys Ala
           275                 280                 285
   Asp Gly Asn Thr Thr Asp Cys Leu Thr Glu Thr Gln Ala Glu Thr Val
       290                 295                 300
   Arg Thr Val Leu Ser Ala Ile Tyr Asp Ala Asn Gly Thr Leu Val Tyr
   305                 310                 315                 320
   Pro Arg Leu Gln Pro Gly Ala Glu Val Leu Ala Gly Gln Gln Gln Phe
                   325                 330                 335
   Asn Gly Gln Pro Phe Asp Ile Thr Gln Asp Trp Tyr Arg Tyr Val Ile
               340                 345                 350
   Tyr Asn Asp Ser Thr Trp Asp Pro Ala Thr Leu Asn Leu Gly Asp Tyr
           355                 360                 365
   Ala Val Ala Leu Ala Gln Asn Pro Ala Asn Ile Glu Thr Phe Asn Gly
       370                 375                 380
   Asp Ile Ser Ala Phe Glu Ala Ala Gly Gly Lys Val Leu His Tyr His
   385                 390                 395                 400
   Gly Leu Met Asp Gly Leu Ile Ser Ser Asp Asn Ser Lys Arg Tyr Tyr
                   405                 410                 415
   Lys Leu Val Gln Glu Thr Met Gly Lys Asp Ser Ser Glu Leu Asp Asp
               420                 425                 430
   Phe Tyr Arg Phe Phe Pro Ile Ser Gly Met Ser His Cys Ser Gly Gly
           435                 440                 445
   Asp Gly Ala Tyr Arg Ile Gly Asn Val Asp Gly Gly Ala Gly Thr Ser
       450                 455                 460
   Ala Asp Asp Asn Val Leu Met Ser Met Val Arg Trp Val Glu Glu Gly
   465                 470                 475                 480
   Val Ala Pro Glu Val Val Arg Gly Ala Asn Ala Asn Val Thr Tyr Trp
                   485                 490                 495
   Arg Ser His Cys Lys Trp Pro Leu Thr Asn Lys Tyr Val Gly Pro Gly
               500                 505                 510
   Ser Tyr Glu Asp Glu Ser Ala Trp Gln Cys Ser
           515                 520
<210> 28
   <211> 1500
   <212> DNA
   <213> Schizophyllum commune
<400> 28
   caagacgact tcgcctcaga ctgctcggcg tttatcgaca agataatcct cgacaatgtc      60
   actgtcacct cgacggagtt tgtggcagcg ggtacgaact tgactgtgta cgtccccaac     120
   tcgtgcgggt cgccgtcgta tcaggtcgtc agcaccgacc tctgccgcgc gactatgaac     180
   gtcacgacct ctgagcgcag cggcatacgc ctcgaggcct ggttcccgca gaactacacc     240
   ggccgcttct tgagcactgg taacggtggt atcggaggat gcattcagta tagcgacatc     300
   gactatgccg cctccctcgg tttcgctgcc gtcggcgcca acaacggcca cgacggcatg     360
   accggcgagc ccttcctcaa caacccggac gttgtcaccg acttcgcctg gcgctcgctg     420
   cacaccggcg tcgtcgtcgg caagcaactc accgagacct tctacggcgc gccccacaac     480
   aagtcgtact acctcggctg ctctactggc ggtcggcagg gcttcaaggc ggtgcaggac     540
   tttccggacg acttcgacgg cgtcgtcgcg ggcgcgccgg ccttcgcttt caatgggctg     600
   ctgtactgga gcggctcgtt cctgcccgtc accggaacct cggaggaccc gaccttcgtc     660
   cccacggatt tctgggcgca gaatatccac gcggaggtcc tccagcaatg tgacgcgctc     720
   gacggcgcga cggatggggt cctggagaac ccggacctct gcgacttcga cgtgacgccg     780
   ctcatctgca cggacggcaa cacgacgggc tgcctgaccg agacgcaggc ggagaccgtg     840
   cgcacggtgc tcagccccat ctacgacaag aacggcaccc tcgtgtaccc gcgtctgcag     900
   cctggtgcag aggtcctcgc cgggtttgcc cagttcggcg gccagccctt cctttattct     960
   acggactggt accgctacgt cgtctacaac gactcgaatt gggaccccat gacgctctcc    1020
   attgacgact acgccgccgc ccgcgcccag aaccccgcca atgtcgagac gttcgagggc    1080
   gacatctccg ccttcgcgtc ctccggtggc aaggtcctgc actaccacgg cctgatggac    1140
   ggcctgatct cctccgacaa ctccaagcgg tactacgccc tcgttcagca aaccctcaac    1200
   caagagcccg ccgagctcga cgagttctac cgcttcttcc ccatcagcgg gatgtcgcac    1260
   tgcagcggcg gcgacggcgc gtaccgcatc gggaacgtcg agggcggcgc gggcacgagt    1320
   gcggacgaca acgtgctgat gtccatggtg cggtgggtgg aggagggcgt ggcgccggag    1380
   gttgtgcgcg gggcggacgc gaatgcgact tattggcgcg cgcattgcaa gtggccgaag    1440
   acgaacaagt acgtcggacc gggggattat gaggatgaga gtgcatggga gtgttcttga    1500
<210> 29
   <211> 1569
   <212> DNA
   <213> Schizophyllum commune
<400> 29
   atggcgcccc tccgctcagt ttctagcgtg ctggtgggtg cgctcgcgct cctgcgcgcc      60
   gcgcacgcgc aagacgactt cgcctcagac tgctcggcgt ttatcgacaa gataatcctc     120
   gacaatgtca ctgtcacctc gacggagttt gtggcagcgg gtacgaactt gactgtgtac     180
   gtccccaact cgtgcgggtc gccgtcgtat caggtcgtca gcaccgacct ctgccgcgcg     240
   actatgaacg tcacgacctc tgagcgcagc ggcatacgcc tcgaggcctg gttcccgcag     300
   aactacaccg gccgcttctt gagcactggt aacggtggta tcggaggatg cattcagtat     360
   agcgacatcg actatgccgc ctccctcggt ttcgctgccg tcggcgccaa caacggccac     420
   gacggcatga ccggcgagcc cttcctcaac aacccggacg ttgtcaccga cttcgcctgg     480
   cgctcgctgc acaccggcgt cgtcgtcggc aagcaactca ccgagacctt ctacggcgcg     540
   ccccacaaca agtcgtacta cctcggctgc tctactggcg gtcggcaggg cttcaaggcg     600
   gtgcaggact ttccggacga cttcgacggc gtcgtcgcgg gcgcgccggc cttcgctttc     660
   aatgggctgc tgtactggag cggctcgttc ctgcccgtca ccggaacctc ggaggacccg     720
   accttcgtcc ccacggattt ctgggcgcag aatatccacg cggaggtcct ccagcaatgt     780
   gacgcgctcg acggcgcgac ggatggggtc ctggagaacc cggacctctg cgacttcgac     840
   gtgacgccgc tcatctgcac ggacggcaac acgacgggct gcctgaccga gacgcaggcg     900
   gagaccgtgc gcacggtgct cagccccatc tacgacaaga acggcaccct cgtgtacccg     960
   cgtctgcagc ctggtgcaga ggtcctcgcc gggtttgccc agttcggcgg ccagcccttc    1020
   ctttattcta cggactggta ccgctacgtc gtctacaacg actcgaattg ggaccccatg    1080
   acgctctcca ttgacgacta cgccgccgcc cgcgcccaga accccgccaa tgtcgagacg    1140
   ttcgagggcg acatctccgc cttcgcgtcc tccggtggca aggtcctgca ctaccacggc    1200
   ctgatggacg gcctgatctc ctccgacaac tccaagcggt actacgccct cgttcagcaa    1260
   accctcaacc aagagcccgc cgagctcgac gagttctacc gcttcttccc catcagcggg    1320
   atgtcgcact gcagcggcgg cgacggcgcg taccgcatcg ggaacgtcga gggcggcgcg    1380
   ggcacgagtg cggacgacaa cgtgctgatg tccatggtgc ggtgggtgga ggagggcgtg    1440
   gcgccggagg ttgtgcgcgg ggcggacgcg aatgcgactt attggcgcgc gcattgcaag    1500
   tggccgaaga cgaacaagta cgtcggaccg ggggattatg aggatgagag tgcatgggag    1560
   tgttcttga                                                            1569
<210> 30
   <211> 499
   <212> PRT
   <213> Schizophyllum commune
<400> 30
   Gln Asp Asp Phe Ala Ser Asp Cys Ser Ala Phe Ile Asp Lys Ile Ile
   1               5                   10                  15
   Leu Asp Asn Val Thr Val Thr Ser Thr Glu Phe Val Ala Ala Gly Thr
               20                  25                  30
   Asn Leu Thr Val Tyr Val Pro Asn Ser Cys Gly Ser Pro Ser Tyr Gln
           35                  40                  45
   Val Val Ser Thr Asp Leu Cys Arg Ala Thr Met Asn Val Thr Thr Ser
       50                  55                  60
   Glu Arg Ser Gly Ile Arg Leu Glu Ala Trp Phe Pro Gln Asn Tyr Thr
   65                  70                  75                  80
   Gly Arg Phe Leu Ser Thr Gly Asn Gly Gly Ile Gly Gly Cys Ile Gln
                   85                  90                  95
   Tyr Ser Asp Ile Asp Tyr Ala Ala Ser Leu Gly Phe Ala Ala Val Gly
               100                 105                 110
   Ala Asn Asn Gly His Asp Gly Met Thr Gly Glu Pro Phe Leu Asn Asn
           115                 120                 125
   Pro Asp Val Val Thr Asp Phe Ala Trp Arg Ser Leu His Thr Gly Val
       130                 135                 140
   Val Val Gly Lys Gln Leu Thr Glu Thr Phe Tyr Gly Ala Pro His Asn
   145                 150                 155                 160
   Lys Ser Tyr Tyr Leu Gly Cys Ser Thr Gly Gly Arg Gln Gly Phe Lys
                   165                 170                 175
   Ala Val Gln Asp Phe Pro Asp Asp Phe Asp Gly Val Val Ala Gly Ala
               180                 185                 190
   Pro Ala Phe Ala Phe Asn Gly Leu Leu Tyr Trp Ser Gly Ser Phe Leu
           195                 200                 205
   Pro Val Thr Gly Thr Ser Glu Asp Pro Thr Phe Val Pro Thr Asp Phe
       210                 215                 220
   Trp Ala Gln Asn Ile His Ala Glu Val Leu Gln Gln Cys Asp Ala Leu
   225                 230                 235                 240
   Asp Gly Ala Thr Asp Gly Val Leu Glu Asn Pro Asp Leu Cys Asp Phe
                   245                 250                 255
   Asp Val Thr Pro Leu Ile Cys Thr Asp Gly Asn Thr Thr Gly Cys Leu
               260                 265                 270
   Thr Glu Thr Gln Ala Glu Thr Val Arg Thr Val Leu Ser Pro Ile Tyr
           275                 280                 285
   Asp Lys Asn Gly Thr Leu Val Tyr Pro Arg Leu Gln Pro Gly Ala Glu
       290                 295                 300
   Val Leu Ala Gly Phe Ala Gln Phe Gly Gly Gln Pro Phe Leu Tyr Ser
   305                 310                 315                 320
   Thr Asp Trp Tyr Arg Tyr Val Val Tyr Asn Asp Ser Asn Trp Asp Pro
                   325                 330                 335
   Met Thr Leu Ser Ile Asp Asp Tyr Ala Ala Ala Arg Ala Gln Asn Pro
               340                 345                 350
   Ala Asn Val Glu Thr Phe Glu Gly Asp Ile Ser Ala Phe Ala Ser Ser
           355                 360                 365
   Gly Gly Lys Val Leu His Tyr His Gly Leu Met Asp Gly Leu Ile Ser
       370                 375                 380
   Ser Asp Asn Ser Lys Arg Tyr Tyr Ala Leu Val Gln Gln Thr Leu Asn
   385                 390                 395                 400
   Gln Glu Pro Ala Glu Leu Asp Glu Phe Tyr Arg Phe Phe Pro Ile Ser
                   405                 410                 415
   Gly Met Ser His Cys Ser Gly Gly Asp Gly Ala Tyr Arg Ile Gly Asn
               420                 425                 430
   Val Glu Gly Gly Ala Gly Thr Ser Ala Asp Asp Asn Val Leu Met Ser
           435                 440                 445
   Met Val Arg Trp Val Glu Glu Gly Val Ala Pro Glu Val Val Arg Gly
       450                 455                 460
   Ala Asp Ala Asn Ala Thr Tyr Trp Arg Ala His Cys Lys Trp Pro Lys
   465                 470                 475                 480
   Thr Asn Lys Tyr Val Gly Pro Gly Asp Tyr Glu Asp Glu Ser Ala Trp
                   485                 490                 495
   Glu Cys Ser
<210> 31
   <211> 522
   <212> PRT
   <213> Schizophyllum commune
<400> 31
   Met Ala Pro Leu Arg Ser Val Ser Ser Val Leu Val Gly Ala Leu Ala
   1               5                   10                  15
   Leu Leu Arg Ala Ala His Ala Gln Asp Asp Phe Ala Ser Asp Cys Ser
               20                  25                  30
   Ala Phe Ile Asp Lys Ile Ile Leu Asp Asn Val Thr Val Thr Ser Thr
           35                  40                  45
   Glu Phe Val Ala Ala Gly Thr Asn Leu Thr Val Tyr Val Pro Asn Ser
       50                  55                  60
   Cys Gly Ser Pro Ser Tyr Gln Val Val Ser Thr Asp Leu Cys Arg Ala
   65                  70                  75                  80
   Thr Met Asn Val Thr Thr Ser Glu Arg Ser Gly Ile Arg Leu Glu Ala
                   85                  90                  95
   Trp Phe Pro Gln Asn Tyr Thr Gly Arg Phe Leu Ser Thr Gly Asn Gly
               100                 105                 110
   Gly Ile Gly Gly Cys Ile Gln Tyr Ser Asp Ile Asp Tyr Ala Ala Ser
           115                 120                 125
   Leu Gly Phe Ala Ala Val Gly Ala Asn Asn Gly His Asp Gly Met Thr
       130                 135                 140
   Gly Glu Pro Phe Leu Asn Asn Pro Asp Val Val Thr Asp Phe Ala Trp
   145                 150                 155                 160
   Arg Ser Leu His Thr Gly Val Val Val Gly Lys Gln Leu Thr Glu Thr
                   165                 170                 175
   Phe Tyr Gly Ala Pro His Asn Lys Ser Tyr Tyr Leu Gly Cys Ser Thr
               180                 185                 190
   Gly Gly Arg Gln Gly Phe Lys Ala Val Gln Asp Phe Pro Asp Asp Phe
           195                 200                 205
   Asp Gly Val Val Ala Gly Ala Pro Ala Phe Ala Phe Asn Gly Leu Leu
       210                 215                 220
   Tyr Trp Ser Gly Ser Phe Leu Pro Val Thr Gly Thr Ser Glu Asp Pro
   225                 230                 235                 240
   Thr Phe Val Pro Thr Asp Phe Trp Ala Gln Asn Ile His Ala Glu Val
                   245                 250                 255
   Leu Gln Gln Cys Asp Ala Leu Asp Gly Ala Thr Asp Gly Val Leu Glu
               260                 265                 270
   Asn Pro Asp Leu Cys Asp Phe Asp Val Thr Pro Leu Ile Cys Thr Asp
           275                 280                 285
   Gly Asn Thr Thr Gly Cys Leu Thr Glu Thr Gln Ala Glu Thr Val Arg
       290                 295                 300
   Thr Val Leu Ser Pro Ile Tyr Asp Lys Asn Gly Thr Leu Val Tyr Pro
   305                 310                 315                 320
   Arg Leu Gln Pro Gly Ala Glu Val Leu Ala Gly Phe Ala Gln Phe Gly
                   325                 330                 335
   Gly Gln Pro Phe Leu Tyr Ser Thr Asp Trp Tyr Arg Tyr Val Val Tyr
               340                 345                 350
   Asn Asp Ser Asn Trp Asp Pro Met Thr Leu Ser Ile Asp Asp Tyr Ala
           355                 360                 365
   Ala Ala Arg Ala Gln Asn Pro Ala Asn Val Glu Thr Phe Glu Gly Asp
       370                 375                 380
   Ile Ser Ala Phe Ala Ser Ser Gly Gly Lys Val Leu His Tyr His Gly
   385                 390                 395                 400
   Leu Met Asp Gly Leu Ile Ser Ser Asp Asn Ser Lys Arg Tyr Tyr Ala
                   405                 410                 415
   Leu Val Gln Gln Thr Leu Asn Gln Glu Pro Ala Glu Leu Asp Glu Phe
               420                 425                 430
   Tyr Arg Phe Phe Pro Ile Ser Gly Met Ser His Cys Ser Gly Gly Asp
           435                 440                 445
   Gly Ala Tyr Arg Ile Gly Asn Val Glu Gly Gly Ala Gly Thr Ser Ala
       450                 455                 460
   Asp Asp Asn Val Leu Met Ser Met Val Arg Trp Val Glu Glu Gly Val
   465                 470                 475                 480
   Ala Pro Glu Val Val Arg Gly Ala Asp Ala Asn Ala Thr Tyr Trp Arg
                   485                 490                 495
   Ala His Cys Lys Trp Pro Lys Thr Asn Lys Tyr Val Gly Pro Gly Asp
               500                 505                 510
   Tyr Glu Asp Glu Ser Ala Trp Glu Cys Ser
           515                 520